# EUROPÄISCHE PATENTSCHRIFT

(11) **EP 2 478 896 B1**
(45) Veröffentlichungstag und Bekanntmachung des Hinweises auf die Patenterteilung: **06.09.2017**
(21) Anmeldenummer: 12001792.6
(22) Anmeldetag: 06.02.2006
(51) Int. Cl.: A61K 9/20

(54) **Bruchfeste Darreichungsformen mit retardierter Freisetzung**
Fracture-resistant application forms with delayed release
Formes pharmaceutiques résistantes à la rupture avec libération prolongée

(30) Priorität: 04.02.2005 DE 102005005446
(43) Veröffentlichungstag der Anmeldung: 25.07.2012
(62) Teilanmeldung aus: 06706682.9
(73) Patentinhaber: Grünenthal GmbH, 52078 Aachen (DE)
(72) Erfinder: Ashworth, Judy, Dr., 52064 Aachen (DE); Arkenau-Maric, Elisabeth, Dr., 50931 Köln (DE); Bartholomäus, Johannes, Dr., 52080 Aachen (DE); Kugelmann, Heinrich, D-52068 Aachen (DE)
(74) Vertreter: Bülle, Jan

(56) Entgegenhaltungen:
- WO-A-97/33566
- WO-A-2005/016313
- WO-A-2005/016314
- WO-A-2005/063214
- WO-A-2005/102286
- US-A1- 2003 068 392
- US-B1- 6 488 963
- SCHROEDER R ET AL: "GRANULIERUNG HYDROPHOBER WIRKSTOFFE IM PLANETWALZENEXTRUDER GRANULATION OF HYDROPHOBIC ACTIVES WITH THE PLANETARY ROLLER EXTRUDER", PHARMAZEUTISCHE INDUSTRIE, AULENDORF, DE, Bd. 65, Nr. 4, 2003, Seiten 367-372, XP009012058, ISSN: 0031-711X

## Beschreibung

Die vorliegende Erfindung betrifft eine Darreichungsform zur Verabreichung einer physiologisch wirksamen Substanz (A), wobei die Darreichungsform mechanisch stabilisiert ist, so dass sie mit herkömmlichen Methoden, wie Hämmern, Mahlen, Mörsern, etc., nicht, oder zumindest nur sehr schwierig zerkleinert werden kann. Die Substanz (A) wird aus der erfindungsgemäßen Darreichungsform unter physiologischen Bedingungen mit einem zumindest teilweise retardierten Profil freigesetzt.

Zahlreiche physiologisch wirksame Substanzen, wie Nahrungsergänzungsmittel, Arzneistoffe, etc. werden als Retard-Formulierungen bereitgestellt, d.h. im Gegensatz zu herkömmlichen Formulierungen (z.B. sog. *"immediate release"* Formulierungen) werden die Substanzen aus diesen Formulierungen verzögert über eine vergleichsweise lange Zeitspanne, die oft mehrere Stunden beträgt, an den Organismus abgegeben. Die Freisetzung der Substanz aus der Darreichungsform einerseits und eine Metabolisierung bzw. Ausscheidung durch den Organismus andererseits gewährleisten einen relativ gleichmäßigen Blutplasmaspiegel der verabreichten Substanz. Als eine Folge davon kann häufig für den Patienten die Anzahl der pro Tag einzunehmenden Dosiseinheiten reduziert werden, oft ist eine Einnahme nur noch ein- oder zweimal am Tag erforderlich.

Retard-Formulierungen können in bestimmten Fällen auch das Ausmaß an Nebenwirkungen der Substanz vermindern. So treten z.B. bei einigen Arzneistoffen verstärkt Nebenwirkungen auf, wenn im Blutplasma zumindest vorübergehend eine bestimmte Grenzkonzentration des Arzneistoffs überschritten wird. Derartige Arzneistoffe sind daher für *"immediate release"* Formulierungen weitgehend ungeeignet, insbesondere wenn eine nur zwei- oder dreimalige tägliche Verabreichung erwünscht ist. Solche Arzneistoffe werden daher üblicherweise als Retard-Formulierungen verabreicht, wodurch eine kontinuierliche Freisetzung des Wirkstoffs gewährleistet und das kurzfristige Auftreten hoher Konzentrationen verhindert werden.

Bei Retard-Formulierungen ist üblicherweise die physiologisch wirksame Substanz entweder in einer die Freisetzung steuernden Matrix eingebettet, und/oder die Darreichungsform ist mit einem Film überzogen, welcher die Freisetzung steuert.

Insbesondere ältere Patienten haben jedoch häufig Schwierigkeiten beim Einnehmen fester Darreichungsformen, wie Tabletten, Gelatinekapseln, etc. Sie verschlucken sich dabei und entwickeln mitunter ausgeprägte Aversionen gegen derartige Darreichungsformen.

Um diesem Problem zu begegnen, wurden verschiedene Apparaturen entwickelt, mit deren Hilfe feste Darreichungsformen zerkleinert oder pulverisiert werden können ("*tablet crusher*")*.* Die Anwendung solcher Apparaturen erfolgt beispielsweise durch das Pflegepersonal in Altenheimen. Den pflegebedürftigen Personen werden dann die Darreichungsformen nicht als Tablette etc., sondern als Pulver verabreicht, beispielsweise um die Schwierigkeiten beim Herunterschlucken von Tabletten zu umgehen.

Problematisch ist die Zerkleinerung der Darreichungsformen mit solchen Apparaturen jedoch, wenn es sich bei den Darreichungsformen um Retard-Formulierungen handelt. Die Zerkleinerung führt nämlich im Regelfall dazu, dass die innere Struktur der Darreichungsform, welche für die retardierte Freisetzung verantwortlich ist, zerstört wird, wodurch die retardierende Wirkung aufgehoben wird. Infolge der Zerkleinerung werden die Diffusionswege der enthaltenen physiologisch wirksamen Substanzen verkürzt und/oder die Diffusionsbarrieren entfernt So weist eine Retard-Formulierung, bei welcher die verzögerte Freisetzung mit Hilfe eines Filmüberzugs erreicht werden soll, nach der Zerkleinerung nur noch auf einem geringen prozentualen Anteil ihrer Feststoffoberfläche den Filmüberzug auf. Als Folge davon wird nach Verabreichung häufig die gesamte in der Darreichungsform ursprünglich enthaltene physiologisch wirksame Substanz in relativ kurzer Zeit freigesetzt, wodurch sprunghaft für eine relativ kurze Zeitspanne eine vergleichsweise sehr hohe Plasmakonzentration der Substanz erreicht wird. Aus den ursprünglichen Retard-Formulierungen werden auf diese Weise "*immediate release*" Formulierungen.

Je nach physiologischer Wirksamkeit der Substanz kann dies jedoch erhebliche Nebenwirkungen hervorrufen, in Extremfällen sogar bis zum Tod des Patienten führen. Beispiele für Substanzen mit einem solchen Gefährdungspotential sind Antiparkinsonmittel, Antiepileptika, Antidiabetika, Antihypertensiva, Antiarhythmika, etc.

Im Regelfall sind diese Gefahren den Personen, welche die Darreichungsformen für sich selbst oder für andere zerkleinern, nicht bewusst. Es wurden Todesfälle von Patienten bekannt, welche wahrscheinlich auf eine Pulverisierung von Retard-Formulierungen durch Krankenschwestern bzw. Pfleger zurückzuführen sind. Hinsichtlich weiterer Einzelheiten kann beispielsweise verwiesen werden auf J.E. Mitchell, Oral Dosage Forms That Should Not Be Crushed: 2000 Update. Hospital Pharmacy, 2000; H. Miller et al., To Crush or Not to Crush, Nursing 2000; R. Grittith et al., Tablet Crushing and the law: the implications for nursing; Prof. Nurse 2003; J.G. Schier et al, Fatality from administration of labetalol and crushed extended-release nifedipine, Ann. Pharmacotherapy 2003; A. James, The legal and clinical implications of crushing tablet medication, Nurse Times 2005, 100(50), 28-9; und P. Cornish, "Avoid the Crush": hazards of medication administration in patients with dysphagia or a feeding tube, CMAJ. 2005,172(7), 871-2.

Auch bei Kleinkindern können Retard-Formulierungen Probleme bereiten. So können Kinder häufig feste Darreichungsformen nicht von Süßigkeiten unterscheiden. Finden die Kinder solche Darreichungsformen, beispielsweise weil ihre Eltern diese aus Unachtsamkeit in der Wohnung haben herumliegen lassen, so besteht die Gefahr, dass die Kinder die Darreichungsformen für Bonbons halten, in den Mund nehmen und zerkauen. Handelt es sich dabei um Retard-Formulierungen, welche einen Arzneistoff in einer Dosierung enthalten, die für einen Erwachsenen bestimmt ist, so besteht in einem solchen Fall für das Kind bereits durch die größere enthaltene Arzneistoffmenge die Gefahr einer Überdosierung. Durch das Zerkauen der Darreichungsform und die damit einhergehende Aufhebung der Retard-Wirkung wird diese Gefahr jedoch noch verstärkt, da die bereits ohnehin zu hohe Dosis zusätzlich auch noch innerhalb eines stark verkürzten Zeitintervalls freigesetzt wird, was bereits für einen Erwachsenen erhebliche Gefahren mit sich bringen würde, für ein Kind jedoch umso drastischere Folgen haben kann.

Das Kauen von Retard-Formulierungen kann auch bei Erwachsenen zu einer Überdosierung der enthaltenen Substanz führen. So zerkauen Erwachsene die Darreichungsformen mitunter ganz bewusst, da sie sich - oft in Unkenntnis der Art und des Zwecks einer Retard-Formulierung - davon einen schnelleren Wirkungserfolg versprechen.

Eine bekannte Möglichkeit zur Reduzierung der Gefahren, welche von einer Zerkleinerung der Retard-Formulierungen ausgehen, besteht darin, der Darreichungsform Antagonisten, d.h. Gegenmittel, oder Verbindungen, die zu physiologischen Abwehrreaktionen führen, zuzusetzen, wobei die physiologische Wirkung dieser Zusatzstoffe möglichst nur dann entfaltet wird, wenn die Darreichungsform vor der Verabreichung.zerkleinert wurde. Diese Methode hat jedoch den Nachteil, dass die physiologisch wirksame Substanz dennoch in nicht-retardierter Form verabreicht wird und dass der Organismus zusätzlich mit einer weiteren physiologisch wirksamen Substanz, z.B. einem Gegenmittel belastet oder eine Abwehrreaktion, wie z.B. Erbrechen, ausgelöst wird.

Schroeder, R. et al.: "granulierung hydrophober Wirkstoffe im Platenwalzenextruder", Pharmazeutische Industrie, Aulendorf, DE, Bd. 65, Nr. 4, 2003, S. 367.372 offenbart Tabletten umfassend eine wirksame Substanz (z.B. Mefenaminsäure oder Ibuprofen) und Polymere (Polyvidon und Cellulose). Die Tabletten weisen eine Bruchfestigkeit von ungefähr 1 MPa und eine zumindest teilweise retardierte Freisetzung auf.

WO 2005/102286 offenbart eine Methode zur Herstellung einer missbrauchssicheren, harten Form der Verabreichung die zumindest eine aktive Substanz, die missbraucht werden kann, und ein Bindemittel enthält und eine Bruchfestigkeit von 500 N hat. Nach dieser Methode werden Ultraschall und eine Kraft auf eine Mischung ausgeübt, die die aktive Substanz und das Bindemittel enthält.

WO 2005/016313 offenbart eine Darreichungsform die thermogeformt ist ohne extrudiert worden zu sein und die gegen Missbrauch gesichert ist, enthaltend neben einem oder mehreren Wirkstoffen mit Missbrauchspotential sowie ggf. physiologisch verträglichen Hilfsstoffen mindestens ein synthetisches oder natürliches Polymer mit einer Bruchfestigkeit von mindestens 500.

WO 2005/016314 offenbart eine Form der Verabreichung die thermogeformt ist ohne extrudiert worden zu sein und die gegen Missbrauch gesichert ist, enthaltend neben einem oder mehreren Wirkstoffen mit Missbrauchspotential sowie ggf. physiologisch verträglichen Hilfsstoffen mindestens ein synthetisches oder natürliches Polymer mit einer Bruchfestigkeit von mindestens 500.

US 6 488 963 offenbart Darreichungsformen enthaltend eine Mischung eines therapeutischen Wirkstoffs und eines Polyethylenoxids mit hohem Molekulargewicht, die heißschmelzextrudiert werden kann.

Es besteht daher ein Bedarf an pharmazeutischen Darreichungsformen mit retardierter Freisetzung, welche die Gefahr von Überdosierungen vermindern, so dass auf Gegenmittel etc. verzichtet werden kann.

Der Erfindung liegt die Aufgabe zugrunde, eine Darreichungsform bereitzustellen, welche Vorteile gegenüber den Darreichungsformen des Standes der Technik aufweist. Die Darreichungsform sollte eine physiologisch wirksame Substanz retardiert freisetzen, jedoch die Gefahr von Überdosierungen, insbesondere infolge einer unsachgemäßen Handhabung der Darreichungsform, wie Kauen, Mahlen, Mörsern, etc. vermindern.

Es wurde überraschend gefunden, dass diese Aufgabe gelöst wird durch eine Darreichungsform umfassend
- eine physiologisch wirksame Substanz (A) (= Komponente(A));
- ggf. einen oder mehrere physiologisch verträgliche Hilfsstoffe (B) (= Komponente (B));
- ein synthetisches Polymer (C) (= Komponente (C)); und
- ggf. ein natürliches, halbsynthetisches oder synthetisches Wachs (D) (= Komponente (D));
wobei die Substanz (A) in einer Retardmatrix vorliegt; wobei die Retardmatrix das Polymers (C) und/oder das gegebenenfalls vorhandene Wachs (D) als Retardmatrixmaterial umfasst;
wobei das Polymer C ein Polyalkylenoxid ist ausgewählt aus der Gruppe bestehend aus Polymethylenoxid, Polyethylenoxid, Polypropylenoxid, deren Copolymerisaten, deren Blockcopolymerisaten und deren Mischungen, wobei Polymer (C) ein viskositätsmittleres Molekulargewicht von mindestens 0,5 10⁶ g/mol aufweist; und
wobei die Darreichungsform
- eine Bruchfestigkeit von mindestens 400 N aufweist, und
- unter physiologischen Bedingungen nach 5 Stunden höchstens 99% der Substanz (A) freisetzt;
mit der Maßgabe, dass die Darreichungsform
(1) keine Tablette mit einer Bruchfestigkeit von mindestens 500 N ist, welche ein Gesamtgewicht von 300,0 mg, einen Durchmesser von 10 mm und einen Wölbungsradius von 8 mm aufweist; durch Verpressen eines auf 80°C erwärmten Gemisches bestehend aus 100 mg Tramadolhydrochlorid und 200 mg Polyethylenoxid mit einem Molekulargewicht von 7 000 000 pro Tablette mittels eines Tablettierwerkzeugs mit Oberstempel, Unterstempel und Matrize unter Aufrechterhaltung des Pressdrucks für mindestens 15 Sekunden erhältlich ist; bei einer *in-vitro-*Freisetzungsbestimmung gemäß *Ph. Eur.* in einer Blattrührerapparatur mit dem Freisetzungsmedium bei einer Umdrehungsgeschwindigkeit von 75 U. pro Minute, nach 30 min. 15%, nach 240 min. 52%, nach 480 min. 80% und nach 720 min. 99% der Substanz (A) freisetzt; und wobei zu Beginn der Untersuchung die Tablette in 600 ml künstlichen Magensaft mit einem pH-Wert von 1,2 gegeben wird, welcher durch Zugabe von Lauge nach 30 min. auf 2,3, nach weiteren 90 min. auf 6,5 und nach nochmals 60 weiteren min. auf 7,2 erhöht wird;
(2) keine Tablette mit einer Bruchfestigkeit von mindestens 500 N ist, welche ein Gesamtgewicht von 300,0 mg, einen Durchmesser von 10 mm und einen Wölbungsradius von 8 mm aufweist; durch Verpressen eines auf 80°C erwärmten Gemisches bestehend aus 100 mg Tramadolhydrochlorid und 200 mg Polyethylenoxid mit einem Molekulargewicht von 7 000 000 pro Tablette mittels eines Tablettierwerkzeugs mit Oberstempel, Unterstempel und Matrize unter Aufrechterhaltung des Pressdrucks für mindestens 15 Sekunden erhältlich ist, wobei das Gemisch in die Matrize des Tablettierwerkzeugs eingefüllt wird; bei einer *in-vitro*-Freisetzungsbestimmung gemäß *Ph. Eur.* in einer Blattrührerapparatur mit dem Freisetzungsmedium bei einer Umdrehungsgeschwindigkeit von 75 U. pro Minute, nach 30 min. 15%, nach 240 min. 52%, nach 480 min. 80% und nach 720 min. 99% der Substanz (A) freisetzt; und wobei zu Beginn der Untersuchung die Tablette in 600 ml künstlichen Magensaft mit einem pH-Wert von 1,2 gegeben wird, welcher durch Zugabe von Lauge nach 30 min. auf 2,3, nach weiteren 90 min. auf 6,5 und nach nochmals 60 weiteren min. auf 7,2 erhöht wird;
(3) keine Tablette mit einer Bruchfestigkeit von mindestens 500 N ist, welche ein Gesamtgewicht von 150,0 mg und einen Durchmesser von 7 mm aufweist; durch Verpressen eines auf 80°C erwärmten Gemisches bestehend aus 50 mg Tramadolhydrochlorid und 100 mg Polyethylenoxid mit einem Molekulargewicht von 7 000 000 pro Tablette mittels eines Tablettierwerkzeugs mit Oberstempel, Unterstempel und Matrize unter Aufrechterhaltung des Pressdrucks für mindestens 15 Sekunden erhältlich ist; bei einer *in-vitro*-Freisetzungsbestimmung gemäß *Ph. Eur.* in einer Blattrührerapparatur mit dem Freisetzungsmedium bei einer Umdrehungsgeschwindigkeit von 75 U. pro Minute, nach 30 min. 15%, nach 240 min. 62%, nach 480 min. 88% und nach 720 min. 99% der Substanz (A) freisetzt; und wobei zu Beginn der Untersuchung die Tablette in 600 ml künstlichen Magensaft mit einem pH-Wert von 1,2 gegeben wird, welcher durch Zugabe von Lauge nach 30 min. auf 2,3, nach weiteren 90 min. auf 6,5 und nach nochmals 60 weiteren min. auf 7,2 erhöht wird;
(4) keine Tablette mit einer Bruchfestigkeit von mindestens 500 N ist, welche ein Gesamtgewicht von 300,0 mg, einen Durchmesser von 10 mm und einen Wölbungsradius von 8 mm aufweist; durch Verpressen eines auf 80°C erwärmten Gemisches bestehend aus 100 mg Tramadolhydrochlorid, 20 mg Xanthan und 180 mg Polyethylenoxid mit einem Molekulargewicht von 7 000 000 pro Tablette mittels eines Tablettierwerkzeugs mit Oberstempel, Unterstempel und Matrize unter Aufrechterhaltung des Pressdrucks für mindestens 15 Sekunden erhältlich ist; bei einer *in-vitro*-Freisetzunasbestimmung gemäß *Ph. Eur.* in einer Blattrührerapparatur mit dem Freisetzungsmedium bei einer Umdrehungsgeschwindigkeit von 75 U. pro Minute, nach 30 min. 14%, nach 240 min. 54%, nach 480 min. 81% und nach 720 min. 99% der Substanz (A) freisetzt; und wobei zu Beginn der Untersuchung die Tablette in 600 ml künstlichen Magensaft mit einem pH-Wert von 1,2 gegeben wird, welcher durch Zugabe von Lauge nach 30 min. auf 2,3, nach weiteren 90 min. auf 6,5 und nach nochmals 60 weiteren min. auf 7,2 erhöht wird;
(5) keine Oblongtablette mit einer Bruchfestigkeit von mindestens 500 N ist, welche ein Gesamtgewicht von 150,0 mg, eine Länge von 10 mm und eine Breite von 5 mm aufweist; durch Verpressen eines auf 90°C erwärmten Gemisches bestehend aus 50 mg Tramadolhydrochlorid, 10 mg Xanthan und 90 mg Polyethylenoxid mit einem Molekulargewicht von 7 000 000 pro Tablette mittels eines Tablettierwerkzeugs mit Oberstempel, Unterstempel und Matrize unter Aufrechterhaltung des Pressdrucks für mindestens 15 Sekunden erhältlich ist; bei einer *in-vitro*-Freisetzungsbestimmung gemäß *Ph. Eur.* in einer Blattrührerapparatur mit dem Freisetzungsmedium bei einer Umdrehungsgeschwindigkeit von 75 U. pro Minute, nach 30 min. 22%, nach 120 min. 50%, nach 240 min. 80%, nach 360 min. 90% und nach 480 min. 99% der Substanz (A) freisetzt; und wobei zu Beginn der Untersuchung die Tablette in 600 ml künstlichen Magensaft mit einem pH-Wert von 1,2 gegeben wird, welcher durch Zugabe von Lauge nach 30 min. auf 2,3, nach weiteren 90 min. auf 6,5 und nach nochmals 60 weiteren min. auf 7,2 erhöht wird;
(6) keine Tablette mit einer Bruchfestigkeit von mindestens 500 N ist, welche ein Gesamtgewicht von 150,0 mg, einen Durchmesser von 10 mm und einen Wölbungsradius von 8 mm aufweist; durch Verpressen eines auf 80°C erwärmten Gemisches bestehend aus 20 mg Oxycodon Hydrochlorid, 20,0 mg Xanthan und 110 mg Polyethylenoxid mit einem Molekulargewicht von 7 000 000 pro Tablette mittels eines Tablettierwerkzeugs mit Oberstempel, Unterstempel und Matrize unter Aufrechterhaltung des Pressdrucks für mindestens 15 Sekunden erhältlich ist; und bei einer *in-vitro*-Freisetzungsbestimmung gemäß *Ph. Eur.* in einer Blattrührerapparatur mit dem in der USP beschriebenen Phosphat-gepuffertem Freisetzungsmedium mit pH 6,8 bei 37 °C und einer Umdrehungsgeschwindigkeit von 75 U. pro Minute, nach 30 min. 17%, nach 240 min. 61%, nach 480 min. 90% und nach 720 min. 101,1% der Substanz (A) freisetzt;
(7) keine Tablette mit einer Bruchfestigkeit von mindestens 500 N ist, welche ein Gesamtgewicht von 341,2 mg, einen Durchmesser von 10 mm und einen Wölbungsradius von 8 mm aufweist; durch Lösen von 0,2 mg Butylhydroxytoluol in 96%-igem Ethanol, Trocknung der erhaltenen 7,7%-igen ethanolischen Lösung bei 40°C für 12 h in Gegenwart von 221 mg Polyethylenoxid mit einem Molekulargewicht von 7 000 000, und durch anschließendes Verpressen zusammen mit einem auf 80°C erwärmten Gemisch bestehend aus 100 mg Tramadolhydrochlorid und 20 mg Hydroxypropylmethylcellulose pro Tablette mittels eines Tablettierwerkzeugs mit Oberstempel, Unterstempel und Matrize unter Aufrechterhaltung des Pressdrucks für mindestens 15 Sekunden erhältlich ist; bei einer *in-vitro*-Freisetzungsbestimmung gemäß *Ph. Eur.* in einer Blattrührerapparatur mit dem Freisetzungsmedium bei einer Umdrehungsgeschwindigkeit von 75 U. pro Minute, nach 30 min. 17%, nach 240 min. 59%, nach 480 min. 86% und nach 720 min. 98% der Substanz (A) freisetzt; und wobei zu Beginn der Untersuchung die Tablette in 600 ml künstlichen Magensaft mit einem pH-Wert von 1,2 gegeben wird, welcher durch Zugabe von Lauge nach 30 min. auf 2,3, nach weiteren 90 min. auf 6,5 und nach nochmals 60 weiteren min. auf 7,2 erhöht wird;
(8) keine Tablette mit einer Bruchfestigkeit von mindestens 500 N ist, welche ein Gesamtgewicht von 341,0 mg, einen Durchmesser von 10 mm und einen Wölbungsradius von 8 mm aufweist; durch Verpressen eines auf 80°C erwärmten Gemisches bestehend aus 100 mg Tramadolhydrochlorid, 221 mg Polyethylenoxid mit einem Molekulargewicht von 7 000 000 und 20 mg Hydroxypropylmethylcellulose pro Tablette mittels eines Tablettierwerkzeugs mit Oberstempel, Unterstempel und Matrize unter Aufrechterhaltung des Pressdrucks für mindestens 15 Sekunden erhältlich ist; bei einer *in-vitro*-Freisetzungsbestimmung gemäß *Ph. Eur.* in einer Blattrührerapparatur mit dem Freisetzungsmedium bei einer Umdrehungsgeschwindigkeit von 75 U. pro Minute, nach 30 min. 16%, nach 240 min. 57%, nach 480 min. 84% und nach 720 min. 96% der Substanz (A) freisetzt; und wobei zu Beginn der Untersuchung die Tablette in 600 ml künstlichen Magensaft mit einem pH-Wert von 1,2 gegeben wird, welcher durch Zugabe von Lauge nach 30 min. auf 2,3, nach weiteren 90 min. auf 6,5 und nach nochmals 60 weiteren min. auf 7,2 erhöht wird;
(9) keine biplane Tablette mit einer Bruchfestigkeit von mindestens 500 N ist, welche ein Gesamtgewicht von 335,0 mg aufweist; durch Lösen von 0,2 mg Butylhydroxytoluol in 96%-igem Ethanol, Mischen der erhaltenen 7,7%-igen ethanolischen Lösung mit 150 mg Polyethylenoxid mit einem Molekulargewicht von 7 000 000 in einem Schnellmischer für 30 min., anschließendes Zugeben von 17,8 mg Polyethylenoxid mit einem Molekulargewicht von 7 000 000 und Rühren für 30 min., und anschließendes Trocknen bei 40°C für 12h; Zugabe von 100 mg Tramadolhydrochlorid, 33,5 mg Hydroxypropylmethylcellulose und 33,5 mg Polyethylenglycol; und Extrusion der Mischung in einem Doppelschneckenextruder unter Stickstoffatmosphäre bei einer Schneckendrehzahl von 100 U. pro min., einem Durchsatz von 4 kg/h, einer Produkttemperatur von 125°C und einer Manteltemperatur von 120°C erhältlich ist;
(10) keine Tablette mit einer Bruchfestigkeit von mindestens 500 N ist, welche ein Gesamtgewicht von 586,0 mg und einen Durchmesser von 12 mm aufweist; durch Verpressen eines Gemisches bestehend aus 205 mg Tramadolhydrochlorid und 381 mg Polyethylenoxid mit einem Molekulargewicht von 7 000 000 pro Tablette mittels gleichzeitiger Einwirkung einer Kraft von 250 N und einem Ultraschall mit einer Frequenz von 20 Hz und einer Amplitudenstärke von 50%, für 0,5 Sekunden, erhältlich ist; bei einer *in-vitro*-Freisetzungsbestimmung gemäß *Ph. Eur.* in einer Blattrührerapparatur mit Sinker bei einer Umdrehungsgeschwindigkeit von 75 U. pro Minute, nach 30 min. 13%, nach 240 min. 51%, nach 480 min. 76% und nach 720 min. 100% der Substanz (A) freisetzt; und wobei das Freisetzungsmedium 600 ml Darmsaft mit einer Temperatur von 37°C und einem pH-Wert von 6,8 ist; und
(11) keine Tablette mit einer Bruchfestigkeit von mindestens 500 N ist, welche ein Gesamtgewicht von 300,0 mg und einen Durchmesser von 10 mm und einen Wölbungsradius von 8 mm aufweist; durch Verpressen eines Gemisches bestehend aus 100 mg Tramadolhydrochlorid und 200 mg Polyethylenoxid mit einem Molekulargewicht von 7 000 000 pro Tablette mittels Einwirkung
   - einer Kraft von 970 N und einem Ultraschall mit einer Frequenz von 20 Hz und einer Amplitudenstärke von 75%, gleichzeitig für 0,15 Sekunden, anschließend
   - einer Kraft von 970 N und einem Ultraschall mit einer Frequenz von 20 Hz und einer Amplitudenstärke von 32,5%, gleichzeitig für 0,55 Sekunden, und anschließend
   - einer Kraft von 970 N für 2,3 Sekunden,
erhältlich ist; bei einer *in-vitro*-Freisetzungsbestimmung gemäß *Ph. Eur.* in einer Blattrührerapparatur mit Sinker bei einer Umdrehungsgeschwindigkeit von 75 U. pro Minute, nach 30 min. 17,1%, nach 240 min. 60,6%, nach 480 min. 84% und nach 720 min. 94,2% der Substanz (A) freisetzt; und wobei das Freisetzungsmedium 600 ml Darmsaft mit einer Temperatur von 37°C und einem pH-Wert von 6,8 ist.

Die Darreichungsform weist eine Bruchfestigkeit von mindestens 400 N, bevorzugt mindestens 420 N, bevorzugter mindestens 440 N, noch bevorzugter mindestens 460 N, am bevorzugtesten mindestens 480 N und insbesondere mindestens 500 N auf und setzt unter physiologischen Bedingungen die physiologisch wirksame Substanz (A) zumindest teilweise retardiert frei. Demzufolge umfasst die erfindungsgemäße Darreichungsform eine physiologisch wirksame Substanz (A) mit zumindest teilweise retardierter Freisetzung.

Die erfindungsgemäße Darreichungsform weist über einen weiten Temperaturbereich eine mechanische Festigkeit auf, neben der Bruchfestigkeit ggf. auch ausreichende Härte und Schlagzähigkeit, so dass sie durch Kauen, Mörsern, Hämmern, etc. und auch mit Hilfe kommerziell erhältlicher Apparaturen zur Pulverisierung herkömmlicher Darreichungsformen praktisch nicht zerkleinert oder pulverisiert werden kann. Dabei wird dies nicht notwendigerweise durch die Härte der Darreichungsform erreicht. So kann insbesondere auch ihre Schlagzähigkeit dazu führen, dass sich die erfindungsgemäße Darreichungsform zwar infolge einer mechanischen Einwirkung von außen verformen lässt, beispielswiese mit Hilfe eines Hammers, dabei jedoch nicht in mehrere Bruchstücke zerfällt. Eine Zerkleinerung gelingt selbst dann nicht, wenn die Darreichungsform zur Erhöhung ihrer Sprödigkeit zunächst abgekühlt wird, beispielsweise auf Temperaturen unterhalb von -25°C, -40°C oder etwa in flüssigem Stickstoff.

Als Folge davon bleibt die retardierte Freisetzung erhalten und eine Überdosierung infolge unsachgemäßer Handhabung der Darreichungsform wird effektiv verhindert.

Die vorteilhaften Eigenschaften der erfindungsgemäßen Darreichungsformen, insbesondere auch ihre mechanischen Eigenschaften, lassen sich nicht automatisch durch Verabreitung der Komponenten (A), (C), ggf. (B) und ggf. (D) mit Hilfe beliebiger, herkömmlicher Verfahren zur Herstellung von Darreichungsformen erhalten. Vielmehr ist es üblicherweise erforderlich, bei der Herstellung geeignete Apparaturen auszuwählen und dabei geeignete Parameter einzustellen, insbesondere Druck/ Kraft, Tempertur und Zeit. Nur wenn die Komponenten bei der Herstellung der Darreichungsform einem ausreichenden Druck bei einer ausreichenden Temperatur für eine ausreichende Dauer ausgesetzt werden, lassen sich Darreichungsformen mit den gewünschten Eigenschaften erhalten. Selbst wenn also herkömmliche Apparaturen verwendet werden, müssen die Herstellungsprotokolle üblicherweise angepasst werden, um die erforderlichen Kriterien zu erfüllen.

Unter retardierter Freisetzung wird erfindungsgemäß bevorzugt ein Freisetzungsprofil verstanden, bei dem die physiologisch wirksame Substanz mit dem Ziel einer verlängerten therapeutischen Wirkung über einen längeren Zeitraum bei verringerter Einnahmefrequenz freigegeben wird. Insbesondere wird dies bei peroraler Verabreichung erreicht. Der Ausdruck "mit zumindest teilweise retardierter Freisetzung" umfasst erfindungsgemäß jegliche Darreichungsformen, welche eine modifizierte Freigabe der darin enthaltenen physiologisch wirksamen Substanzen gewährleisten. Bei den Darreichungsformen handelt es sich bevorzugt um überzogene oder nicht überzogene Darreichungsformen, die mit speziellen Hilfsstoffen, nach besonderen Verfahren oder durch Kombination beider Möglichkeiten hergestellt werden, um die Freisetzungsgeschwindigkeit oder den Ort der Freisetzung gezielt zu verändern.

Hinsichtlich des zeitlichen Ablaufs der Freigabe sind bei den erfindungsgemäßen Darreichungsformen folgende Arten umfasst: verzögerte Freigabe (*extended release,* delayed release), gestaffelte Freigabe (repeat action release), hinhaltende Freigabe (prolonged release) und gleichmäßig hinhaltende Freigabe (sustained release).

Zum Zwecke der Beschreibung definiert "verzögerte Freigabe" vorzugsweise eine verzögerte Freisetzung der physiologisch wirksamen Substanz für eine definierte, endliche Zeitspanne (lag time), nach deren Ende die Freisetzung ungehindert erfolgt. "Gestaffelte Freigabe" definiert vorzugsweise die initiale Freisetzung einer ersten Teilmenge der physiologisch wirksamen Substanz gefolgt von zumindest einer weiteren Teilmenge, welche daran anschließend freigesetzt wird. "Hinhaltende Freigabe" definiert vorzugsweise eine Freisetzung mit verringerter Freisetzungsrate, um eine therapeutische Wirksamkeit aufrecht zu erhalten, toxische Wirkungen zu vermindern oder aus anderen therapeutischen Gründen. "Gleichmäßig hinhaltende Freigabe" definiert vorzugsweise eine stetige Freisetzung über einen vergleichsweise langen Zeitraum, um die Häufigkeit der Verabreichung zu verringern. Hinsichtlich weiterer Einzelheiten kann beispielsweise verwiesen werden auf K.H. Bauer, Lehrbuch der Pharmazeutischen Technologie, 6. Auflage, WVG Stuttgart, 1999; und das Europäisches Arzneibuch.

Die erfindungsgemäße Darreichungsform setzt unter physiologischen Bedingungen nach 5 Stunden höchstens 99%, bevorzugt höchstens 90%, bevorzugter höchstens 75%, noch bevorzugter höchstens 50%, am bevorzugtesten höchstens 40% und insbesondere höchstens 30% der Substanz (A) frei. Dabei ist es besonders bevorzugt, dass die Darreichungsform in diesem Fall weder Tramadol Hydrochlorid, noch Oxycodon Hydrochlorid, bevorzugter kein Opioid [N02A] enthält (zur Bedeutung von "N02A" siehe unten). Die Freisetzung wird dabei bevorzugt gemäß den standardisierten Verfahren im Europäischen Arzneibuch ermittelt, vorzugsweise unter den in Beispiel 1 angegebenen Bedingungen.

Vorzugsweise hat die erfindungsgemäße Darreichungsform unter physiologischen Bedingungen nach 30 Minuten 0,1 bis 75 Gew.%, nach 240 Minuten 0,5 bis 95 Gew.-%, nach 480 Minuten 1,0 bis 100 New.-% und nach 720 Minuten 2,5 bis 100 Gew.-% der physiologisch wirksamen Substanz (A) freigesetzt.

Weitere bevorzugte Freisetzungsprofile 1 bis 5 sind in nachfolgender Tabelle zusammengefasst [Angaben in Gew.-% freigesetzte Komponente (A)]:

| Zeit [h] | Nr. 1 | Nr. 2 | Nr. 3 | Nr. 4 | Nr. 5 |
|---|---|---|---|---|---|
| 1 | 0-30 | 0-50 | 0-50 | 15-25 | 20-50 |
| 2 | 0-40 | 0-75 | 0-75 | 25-35 | 40-75 |
| 4 | 3-55 | 3-95 | 10-95 | 30-45 | 60-95 |
| 8 | 10-65 | 10-100 | 35-100 | 40-60 | 80-100 |
| 12 | 20-75 | 20-100 | 55-100 | 55-70 | 90-100 |
| 16 | 30-88 | 30-100 | 70-100 | 60-75 | |
| 24 | 50-100 | 50-100 | >90 | | |
| 36 | >80 | >80 | | | |

Bevorzugt ist das Freisetzungsverhalten der erfindungsgemäßen Darreichungsform vom pH-Wert des Freisetzungsmediums weitgehend unabhängig, d.h. das Freisetzungsprofil in künstlichem Darmsaft entspricht bevorzugt im wesentlichen dem Freisetzungsprofil in künstlichem Magensaft. Vorzugsweise beträgt die Abweichung der beiden Freisetzungsprofile voneinander zu jedem Zeitpunkt der Messung höchstens 20%, bevorzugter höchstens 15%, noch bevorzugter höchstens 10%, noch bevorzugter höchstens 7,5%, am bevorzugtesten höchstens 5,0% und insbesondere höchstens 2,5%.

Vorzugsweise weist die erfindungsgemäße Darreichungsform ein einheitliches Freisetzungsverhalten auf. Dabei ist es bevorzugt, dass das Freisetzungsverhalten der physiologisch wirksamen Substanz (A) interindividuell (d.h. im Vergleich von durch dasselbe Verfahren hergestellten Darreichungsformen) und/oder innerhalb einer einzelnen Darreichungsform (d.h. im Vergleich von Teilsegmente derselben Darreichungsform) einheitlich ist. Vorzugsweise weicht bei einem solchen Vergleich von zwei Proben mit einer Masse von vorzugsweise jeweils 500 mg die insgesamt freigesetzte Wirkstoffmenge zu jedem Zeitpunkt der Messung um höchstens 20%, bevorzugter um höchstens 15%, noch bevorzugter um höchstens 10%, noch bevorzugter um höchstens 7,5%, am bevorzugtesten um höchstens 5,0% und insbesondere um höchstens 2,5% voneinander ab.

Vorzugsweise ist das Freisetzungsprofil der erfindungsgemäßen Darreichungsform lagerstabil, bevorzugt bei Lagerung bei erhöhter Temperatur, z.B. bei 37°C, für 3 Monate in versiegelten Behältern. In diesem Zusammenhang bedeutet "lagerstabil" , dass die beiden Freisetzungsprofile bei Vergleich des initialen Freisetzungsprofils mit dem Freisetzungsprofil nach der Lagerung zu jedem Zeitpunkt der Messung um höchstens 20%, bevorzugter um höchstens 15%, noch bevorzugter um höchstens 10%, noch bevorzugter um höchstens 7,5%, am bevorzugtesten um höchstens 5,0% und insbesondere um höchstens 2,5% voneinander abweichen.

Durch den Einsatz bestimmter Polymere in geeigneter Menge und unter geeigneten Bedingungen wird erfindungsgemäß erreicht, dass die Darreichungsform eine Bruchfestigkeit von mindestens 400 N, bevorzugt mindestens 420 N, bevorzugter mindestens 440 N, noch bevorzugter mindestens 460 N, am bevorzugtesten 480 N und insbesondere mindestens 500 N aufweist (gemessen, wie in der Beschreibung angegeben; die bevorzugte erfindungsgemäße Methode zur Bestimmung der Bruchfestigkeit ist eine Abwandlung der im Europäischen Arzneibuch 5,0 auf Seite 235, 2.9.8 beschriebenen Methode *"Resistance to Crushing of Tablets*"). Dadurch gelingt es, eine Zerkleinerung, z.B. ein Pulverisieren der Darreichungsform mit üblichen Mitteln wirksam zu verhindern.

Unter einer Zerkleinerung wird erfindungsgemäß die Pulverisierung der Darreichungsform unter Krafteinwirkung mit üblichen Mitteln, wie z. B. Mörser und Pistill, Hammer, Schlegel oder anderen gebräuchlichen Mitteln zum Pulverisieren, insbesondere auch eigens dafür entwickelten Vorrichtungen (*tablet crusher*) verstanden, wobei ein gegebenenfalls anfallender Feinanteil (Teilchengröße gleich oder kleiner 0,3 mm) von 5 New.-% nicht überschritten werden darf.

Die erfindungsgemäße Darreichungsform ist daher zur Verhinderung der Überdosierung physiologisch wirksamer Substanzen geeignet, insbesondere von Nahrungsergänzungsmitteln und Arzneistoffen, welche in Retard-Formulierungen bereitgestellt werden. Auf Gegenmittel, Reizstoffe, etc. kann dabei verzichtet werden. Neben der Verhinderung von Überdosierungen und den damit einhergehenden Risiken für den Patienten gewährleisten die erfindungsgemäßen Darreichungsformen außerdem, dass die übrigen Vorteile der Retard-Formulierung, wie beispielsweise eine gleichmäßige Freigabe über einen längeren Zeitraum, erhalten bleiben und nicht ohne weiteres aufgehoben werden können.

Zur Erzielung der notwendigen Bruchfestigkeit der erfindungsgemäßen Darreichungsform wird mindestens ein synthetisches Polymer (C) eingesetzt, welches maßgeblich zur erhöhten Bruchfestigkeit der Darreichungsform beiträgt. Die Bruchfestigkeit der Darreichungsform beträgt mindestens 400 N, bevorzugt mindestens 420 N, bevorzugter mindestens 440 N, noch bevorzugter mindestens 460 N, am bevorzugtesten mindestens 480 N und insbesondere mindestens 500 N, wobei die Bruchfestigkeit nach der in der Beschreibung angegebenen Methode bestimmt wird. In einer bevorzugten Ausführungsform beträgt die Bruchfestigkeit der Darreichungsform mindestens 500 N, bevorzugter mindestens 600 N, bevorzugter mindestens 700 N, noch bevorzugter mindestens 800 N, noch bevorzugter mindestens 900 N, am bevorzugtesten mindestens 1000 N und insbesondere mindestens 1100 N.

Neben ihrer Bruchfestigkeit zeichnet sich die erfindungsgemäße Darreichungsform vorzugsweise auch durch weitere mechanische Eigenschaften aus, beispielsweise durch ihre Härte, Schlagzähigkeit, Stoßelastizität und/oder ihren Elastizitätsmodul, ggf. auch bei tiefen Temperaturen (z.B. unterhalb von -24°C, unterhalb von -40°C oder in flüssigem Stickstoff).

In einer bevorzugten Ausführungsform weist die erfindungsgemäße Darreichungsform eine Dichte von mindestens 0,80 oder mindestens 0,85 g/cm³, bevorzugter mindestens 0,90 oder mindestens 0,95 g/cm³, noch bevorzugter mindestens 1,00, mindestens 1,05 oder mindestens 1,10 g/cm³, am bevorzugtesten im Bereich von 0,80 bis 1,35 g/cm³ und insbesondere im Bereich von 0,95 bis 1,25 g/cm³ auf.

Die erfindungsgemäße Darreichungsform zeichnet sich durch eine vergleichsweise homogene Dichteverteilung aus. Vorzugsweise weichen die Dichten zweier Teilsegmente der Darreichungsform mit einem Volumen von jeweils 1,0 mm³ um höchstens ±10%, bevorzugter um höchstens ±7,5%, noch bevorzugter um höchstens ±5,0%, am bevorzugtesten um höchstens ±2,5% und insbesondere um höchstens ±1,0% voneinander ab.

Die erfindungsgemäße Darreichungsform zeichnet sich durch eine vergleichsweise homogene Verteilung der physiologisch wirksamen Substanz (A) aus. Vorzugsweise weicht der Gehalt an Komponente (A) in zwei Teilsegmenten der Darreichungsform mit einem Volumen von jeweils 1,0 mm³ um höchstens ±10%, bevorzugter um höchstens ±7,5%, noch bevorzugter um höchstens ±5,0%, am bevorzugtesten um höchstens ±2,5% und insbesondere um höchstens ±1,0% voneinander ab.

Das Gesamtgewicht der erfindungsgemäßen Darreichungsform liegt vorzugsweise im Bereich von 0,01 g bis 1,5 g, bevorzugter von 0,05 g bis 1,2 g, noch bevorzuter von 0,1 g bis 1,0 g, am bevorzugtesten von 0,2 g bis 0,9 g und insbesondere von 0,25 g bis 0,8 g.

Das Polymer (C) ist ein Polyalkylenoxid ausgewählt aus der Gruppe bestehend aus Polymethylenoxid, Polyethylenoxid, Polypropylenoxid, deren Copolymerisaten, deren Blockcopolymerisaten und deren Mischungen, wobei Polymer (C) ein viskositätsmittleres Molekulargewicht von mindestens 0,5 10⁶ g/mol aufweist.

Bevorzugt enthält die erfindungsgemäße Darreichungsform mindestens ein Polymer (C) ausgewählt aus der Gruppe bestehend aus Polyalkylenoxid, vorzugsweise Polymethylenoxid, Polyethylenoxid, Polypropylenoxid; Polyethylen, Polypropylen, Polyvinylchlorid, Polycarbonat, Polystyrol, Polyacrylat, Poly(hydroxyfettsäuren), wie z. B. Poly(3-hydroxybutyrat-co-3-hydroxyvalerat) (Biopol®), Poly(hydroxyvaleriansäure), Polycaprolacton, Polyvinylalkohol, Polyesteramid, Polyethylensuccinat, Polylacton, Polyglykolid, Polyurethan, Polyvinylpyrrolidon, Polyamid, Polylactid, Polyacetal (z.B. Polysaccharide ggf. mit modifizierten Seitenketten), Polylactidglykolid, Polylacton, Polyglykolid, Polyorthoester, Polyanhydrid, Blockpolymer aus Polyethylenglykol und Polybutylenterephthalat (Polyactive®), Polyanhydrid (Polifeprosan), deren Copolymerisaten, und Mischungen aus mindestens zwei der genannten Polymere.

Bevorzugt sind hochmolekulare, thermoplastische Polyalkylenoxide, insbesondere Polyethylenoxid, Polypropylenoxid oder deren (Block-)Copolymere. Besonders bevorzugt sind hochmolekulare Polyalkylenoxide, insbesondere Polyethylenoxide, mit einem, vorzugsweise gewichtsmittleren Molekulargewicht (Mw) oder viskositätsmittleren Molekulargewicht (Mη) von mindestens 0,5 10⁶ g/mol, bevorzugt mindestens 1,0 10⁶ g/mol, bevorzugter mindestens 2,5 10⁶ g/mol, noch bevorzugter mindestens 5,0 10⁶ g/mol, am bevorzugtesten mindestens 7,5 10⁵ g/mol oder 7,5 10⁶ g/mol und insbesondere mindestens 10 10⁶ g/mol, bevorzugt 1,0 10⁶ bis 15 10⁶ g/mol. Dem Fachmann sind geeignete Methoden bekannt, wie Mw bzw. Mη bestimmt werden können. Vorzugsweise erfolgt die Bestimmung von Mη durch rheologische

Messungen und die Bestimmung von M_{w} durch Gelpermeationschromatographie (GPC) an geeigneten Phasen.

Die Polymere (C) weisen bei 25 °C bevorzugt eine Viskosität von 4.500 bis 17.600 mPa s (cP), gemessen in einer 5 Gew.-% wässrigen Lösung mit Hilfe eines Brookfield Viskosimeters, Model RVF (Spindel Nr. 2 / Rotationsgeschwindigkeit 2 Upm), von 400 bis 4.000 mPa s (cP), gemessen an einer 2 Gew.-% wässrigen Lösung mit Hilfe des genannten Viskosimeters (Spindel Nr. 1 bzw. 3 / Rotationsgeschwindigkeit 10 Upm) bzw. von 1.650 bis 10.000 mPa s (cP), gemessen an einer 1 Gew.-% wässrigen Lösung mit Hilfe des genannten Viskosimeters (Spindel Nr. 2 / Rotationsgeschwindigkeit 2 Upm) auf.

Das Polymer (C) wird bevorzugt als Pulver eingesetzt Es kann in Wasser löslich sein.

Vorzugsweise wird das Polymer (C) in einer Menge von mindestens 20 Gew.-%, bevorzugter mindestens 30 Gew.-%, noch bevorzugter mindestens 40 Gew.-%, am bevorzugtesten mindestens 50 Gew.-% und insbesondere mindestens 60 Gew.-%, bezogen auf das Gesamtgewicht der Darreichungsform, eingesetzt. In einer bevorzugten Ausführungsform liegt die Menge im Bereich von 20 bis 49 Gew.-%, bezogen auf das Gesamtgewicht der Darreichungsform.

Die erfindungsgemäße Darreichungsform eignet sich für die Verabreichung von mehreren physiologisch wirksamen Substanzen (A) in einer Darreichungsform. Vorzugsweise erhält die Darreichungsform nur eine bestimmte physiologisch wirksame Substanz (A), bevorzugt ein Nahrungsergänzungsmittel oder einen Arzneistoff (= pharmazeutischen Wirkstoff).

Der Gewichtsanteil der physiologisch wirksamen Substanz (A) bezogen auf das Gesamtgewicht der erfindungsgemäßen Darreichungsform liegt bevorzugt im Bereich von 0,01 bis 95 Gew.-%, bevorzugter 0,5 bis 80 Gew.-%, noch bevorzugter 1,0 bis 70 Gew.-%, am bevorzugtesten 5,0 bis 60 Gew.-% und insbesondere 10 bis 50 Gew.-%. In einer bevorzugten Ausführungsform ist der Gewichtsanteil größer als 20 Gew.-%.

In einer bevorzugten Ausführungsform der erfindungsgemäßen Darreichungsform enthält sie keine psychotrop wirkende Substanz. Dem Fachmann ist bekannt, welche Substanzen eine psychotrope Wirkung haben. Gemeinhin haben Substanzen, welche psychische Prozesse beeinflussen, eine psychotrope Wirkung, d.h. eine spezifische Wirkung auf psychische Funktionen. Substanzen mit psychotroper Wirkung können somit die Stimmung beeinflussen, entweder aufhellend oder dämpfend. Zum Zwecke der Beschreibung zählen insbesondere Opioide, Stimulanzien, Tranquilizer (Barbiturate und Benzodiazepine) und weitere Betäubungsmittel zu den Substanzen mit psychotroper Wirkung. Bevorzugt handelt es sich bei Substanzen mit psychotroper Wirkung um Substanzen, die insbesondere bei nicht bestimmungsgemäßer Art der Verabreichung (insbesondere in der Absicht eines Missbrauchs) ein gegenüber der bestimmungsgemäßen oralen Applikation beschleunigtes Anfluten des Wirkstoffes mit dem von einem Missbraucher gewünschten Ergebnis bewirken, nämlich den Kick. Dieser Kick kann z.B. erreicht werden, wenn die gepulverte Darreichungsform nasal appliziert, d. h. geschnupft wird. Bevorzugt sind Substanzen mit psychotroper Wirkung solche Substanzen, welche (bei entsprechender Dosierung, Darreichungsform und Darreichungsart) die menschliche Verstandestätigkeit und/oder Sinneswahmehmung in einer Weise beeinflussen, dass sie grundsätzlich zu einem Missbrauch geeignet sind.

Die folgenden Opiate, Opioide, Tranquillanzien oder anderen Betäubungsmittel sind Substanzen mit psychotroper Wirkung und sind daher erfindungemäß bevorzugt nicht in der Darreichungsform enthalten: Alfentanil, Allobarbital, Allylprodin, Alphaprodin, Alprazolam, Amfepramon, Amfetamin, Amfetaminil, Amobarbital, Anileridin, Apocodein, Barbital, Bemidon, Benzylmorphin, Bezitramid, Bromazepam, Brotizolam, Buprenorphin, Butobarbital, Butorphanol, Camazepam, Carfentanil, Cathin / D-Norpseudoephedrin, Chlordiazepoxid, Clobazam, Clofedanol, Clonazepam, Clonitazen, Clorazepat, Clotiazepam, Cloxazolam, Cocain, Codein, Cyclobarbital, Cyclorphan, Cyprenorphin, Delorazepam, Desomorphin, Dextromoramid, Dextropropoxyphen, Dezocin, Diampromid, Diamorphon, Diazepam, Dihydrocodein, Dihydromorphin, Dihydromorphon, Dimenoxadol, Dimephetamol, Dimethylthiambuten, Dioxaphetylbutyrat, Dipipanon, Dronabinol, Eptazocin, Estazolam, Ethoheptazin, Ethylmethylthiambuten, Ethylloflazepat, Ethylmorphin,. Etonitazen, Etorphin, Fencamfamin, Fenetyllin, Fenpipramid, Fenproporex, Fentanyl, Fludiazepam, Flunitrazepam, Flurazepam, Halazepam, Haloxazolam, Heroin, Hydrocodon, Hydromorphon, Hydroxypethidin, Isomethadon, Hydroxymethylmorphinan, Ketazolam, Ketobemidon, Levacetylmethadol (LAAM), Levomethadon, Levorphanol, Levophenacylmorphan, Levoxemacin, Lofentanil, Loprazolam, Lorazepam, Lormetazepam, Mazindol, Medazepam, Mefenorex, Meperidin, Meprobamat, Metapon, Meptazinol, Metazocin, Methylmorphin, Metamfetamin, Methadon, Methaqualon, 3-Methylfentanyl, 4-Methylfentanyl, Methylphenidat, Methylphenobarbital, Methyprylon, Metopon, Midazolam, Modafinil, Morphin, Myrophin, Nabilon, Nalbuphen, Nalorphin, Narcein, Nicomorphin, Nimetazepam, Nitrazepam, Nordazepam, Norlevorphanol, Normethadon, Normorphin, Norpipanon, Opium, Oxazepam, Oxazolam, Oxycodon, Oxymorphon, Papaver somniferum, Papaveretum, Pernolin, Pentazocin, Pentobarbital, Pethidin, Phenadoxon, Phenomorphan, Phenazocin, Phenoperidin, Piminodin, Pholcodein, Phenmetrazin, Phenobarbital, Phentermin, Pinazepam, Pipradrol, Piritramid, Prazepam, Profadol, Proheptazin, Promedol, Properidin, Propoxyphen, Remifentanil, Secbutabarbital, Secobarbital, Sufentanil, Temazepam, Tetrazepam, Tilidin (cis und trans)), Tramadol, Triazolam, Vinylbital, N-(1-Methyl-2-piperidinoethyl)-N-(2-pyridyl)propionamld, (1R,2R)-3-(3-Dimethylamino-1-ethyl-2-methyl-propyl)-phenol, (1R, 2R, 4S)-2-(Dimethylamino)methyl-4-(p-fluorbenzyloxy)-1-(m-methoxyphenyl)cyclohexanol, (1R, 2R)-3-(2-Dimethylaminomethyl-cyclohexyl)-phenol, (1S, 2S)-3(3-Dimethylamino-1-ethyl-2-methyl-propyl)-phenol, (2R, 3R)-1-Dimethylamlno-3(3-Methoxy-phenyl)-2-methyl-pentan-3-ol, (1 RS, 3RS, 6RS)-6-Dimethylaminomethyl-1-(3-methoxy-phenyl)-cyclohexan-1,3-diol, vorzugsweise als Racemat, 3-(2-Dimethylamlnomethyl-1-hydroxy-cyclohexyl)-phenyl 2-(4-isobutyl-phenyl)-propionat, 3-(2-Dimethylaminomethyl-1-hydroxy-cyclohexyl)phenyl 2-(6-methoxy-naphthalen-2-yl)-propionat, 3-(2-Dimethylaminomethyl-cyclohex-1-enyl)-phenyl 2-(4-isobutyl-phenyl)-propionat, 3-(2-Dimethylaminomethyl-cyclohex-1-enyl)-phenyl 2-(6-methoxy-naphthalen-2-yl)-propionat, (RR-SS)-2-Acetoxy-4-trifluoromethyl-benzoesäure 3-(2-dimethylaminomethyl-1-hydroxy-cyclohexyl)-phenyl ester, (RR-SS)-2-Hydroxy-4-trifluoromethylbenzoesäure 3-(2-dimethylaminomethyl-1-hydroxy-cyclohexyl)-phenyl ester, (RR-SS)-4-Chloro-2-hydroxy-benzoesäure 3-(2-dimethylaminomethyl-1-hydroxycyclohexyl)-phenyl ester, (RR-SS)-2-Hydroxy-4-methyl-benzoesäure 3-(2-dimethylaminomethyl-1-hydroxy-cyclohexyl)-phenyl ester, (RR-SS)-2-Hydroxy-4-methoxy-benzoesäure 3-(2-dimethylaminomethyl-1-hydroxy-cyclohexyl)-phenyl-ester, (RR-SS)-2-Hydroxy-5-nitro-benzoesäure 3-(2-dimethylaminomethyl-1-hydroxycyclohexyl)-phenyl ester, (RR-SS)-2',4'-Difluoro-3-hydroxy-biphenyl-4-carbonsäure 3-(2-dimethylaminomethyl-1-hydroxy-cyclohexyl)-phenyl ester sowie entsprechende stereoisomere Verbindungen, jeweils deren entsprechende Derivate, physiologisch verträglichen Enantiomere, Stereoisomere, Diastereomere und Racemate und deren physiologisch verträglichen Derivate, beispielsweise Ether, Ester oder Amide, und jeweils deren physiologisch verträgliche Verbindungen, insbesondere deren Salze und Solvate, beispielsweise Hydrochloride.

Insbesondere bevorzugt enthält die erfindungsgemäße Darreichungsform keine Substanz ausgewählt aus der Gruppe bestehend aus Opioiden [A07DA, N01AH, N02A, R05DA, R05FA,], Barbituraten [N01AF, N01AG, N03AA], Benzodiazepin-Derivaten [N03AE], Mitteln zur Behandlung der Opiatabhängigkeit [N07BC], Anxiolytika [N05B], Hypnotika und Sedativa [N05C], Psychostimulanzien, Mitteln zur Behandlung von Aufmerksamkeitsdefizit/Hyperaktivitätsstörung (ADHD) und Nootropika [N06B], Antiemetika [A04A], Abmagerungsmitteln ausgenommen Diätetika [A08A], zentral wirkenden Muskelrelaxantien [M03B], und Antidoten [V03AB]. Die in eckigen Klammern angegebenen Bezeichnungen entsprechen dabei dem ATC-Index, wie er von der WHO zur Klassifizierung der Arzneistoffe verwendet wird (bevorzugter Stand: Januar 2005 oder 2006). Hinsichtlich weiterer Einzelheiten zum ATC-Index kann beispielsweise verwiesen werden auf U. Fricke, J. Günther, Anatomisch-therapeutisch-chemische Klassifikation mit Tagesdosen für den deutschen Arzneimittelmarkt: Methodik der ATC-Klassifikation und DDD-Festlegung. ATC-Index mit DDD-Angaben, Wissenschaftliches Institut der AOK; und Swiss Pharmaceutical Society, Index Nominum: International Drug Directory, CRC Press; 18th edition (January 31, 2004).

In einer bevorzugten Ausführungsform enthält die erfindungsgemäße Darreichungsform keine Verbindung ausgewählt aus der Gruppe bestehend aus
(1) Analgetika, wie z.B. Aspirin, Acetaminophen, Deflunisal, etc.;
(2) Anästhetika, wie z.B. Lidocain, Procain, Benzocain, Xylocain, etc.;
(3) Antiarthritika und entzündungshemmende Mittel, wie z.B. Phenylbutazon, Indomethacin, Sulindac, Dexamethason, Ibuprofen, Allopurinol, Oxyphenbutazon Probenecid, Cortison, Hydrocortison, Betamethason, Dexamethason, Fluocortolon, Prednisolon, Triamcinolon, Indomethacin, Sulindac sowie seine Salze und entsprechenden Sulfide, etc.;
(4) Antiasthmatika, wie z.B. Theophyllin, Ephedrin, Beclomethason Dipropionat, Epinephrin, etc.;
(5) Harntraktdesinfektiva, wie z.B. Sulfarmethoxazol, Trimethoprim, Nitrofurantoin, Norfloxicin, etc.;
(6) Antikoagulantien, wie z.B. Heparin, Bishydroxycoumarin, Warfarin, etc.;
(7) Antikonvulsiva, wie z.B. Diphenylhydantoin, Diazepam, etc.;
(8) Antidepressiva, wie z.B. Amitriptylin, Chlordiazepoxid, Perphenazin, Protriptylin, Imipramin, Doxepin, etc.;
(9) Substanzen, welche zur Behandlung von Diabetes und zur Regulierung des Blutzuckers geeignet sind, wie z.B. Insulin, Tolbutamid, Tolazamid, Somatotropin, Acetohexamid, Chlorpropamid, etc.;
(10) Antineoplastika, wie z.B. Adriamycin, Fluouracil, Methotrexat, Asparaginase, etc.;
(11) Antipsychotika, wie z.B. Prochlorperazin, Lithiumcarbonat, Lithiumcitrat, Thioridazin, Molindon, Fluphenazin, Trifluoperazin, Perphenazin, Amitriptylin, Triflupromazin, etc.;
(12) Antihypertensiva, wie z.B. Spironolacton, Methyldopa, Hydralazin, Clonidin, Chlorothiazid, Deserpidin, Timolol, Propanolol, Metaprotol, Prazosin Hydrochlorid, Reserpin, etc.;
(13) Muskelrelaxantien, wie z.B. Mephalan, Danbrolene, Cyclobenzaprine, Methocarbarnol, Diazepam, Succinoylchlorid, etc.;
(14) Antiprotozoika, wie z.B. Chloramphenicol, Chloroquine, Trimethoprim und Sulfamethoxazol;
(15) Spermicide, wie z.B. Nonoxynol;
(16) antibakteriellen Substanzen, wie z.B. beta-Lactamantibiotika, Tetracycline, Chloramphenicol, Neomycin, Cefoxitin, Thienamycin, Gramicidin, Bacitracin, Sulfonamide, Aminoglykosidantibiotika, Tobramycin, Nitrofurazon, Nalidixinsäure und Analoga und die antimikrobielle Kombination von Fludalanin/Pentizidon;
(17) Antihistamine und Dekongestiva, wie z.B. Perilamin, Chlorpheniramin (z.B. Chlorpheniramin Maleat), Tetrahydrozolin und Antazolin;
(18) antiparasitären Substanzen, wie z.B. Ivermectin;
(19) antiviralen Substanzen, wie z.B. Acyclovir und Interferon;
(20) antifungalen, amöbiziden, trichomonaziden Mittel oder Antiprotozoika, wie z.B. Polyoxyethylennonylphenol, Alkylarylsulfonat, Oxychinolinsulfat, Miconazolnitrat, Sulfanilamid, Candicidin, Sulfisoxazol, Nysatidin, Clotrimazol, Metronidazol, etc.; und
(21) Losoxanthron, Theophyllin oder β-Hydroxyethyl-theophyllin (Etophyllin), Diphenhydramin bzw. sein Hydrochlorid, Diltiazem bzw. sein Hydrochlorid, und Diphenylethyl(adenosin).

In einer bevorzugten Ausführungsform enthält die erfindungsgemäße Darreichungsform keine den Nasen- und/oder Rachenraum reizenden Stoffe, d.h. Stoffe, die bei Applikation über den Nasen- und/oder Rachenraum eine Reaktion des Körpers hervorrufen, welche entweder für den Patienten so unangenehm ist, dass er die Applikation nicht weiter fortsetzen will oder kann, so z.B. ein Brennen, oder auf physiologische Art und Weise einer Aufnahme des entsprechenden Wirkstoffes entgegenwirken, z.B. über eine vermehrte nasale Sekretbildung oder Niesen. Weitere Beispiele für den Nasen- und/oder Rachenraum reizende Stoffe sind solche Stoffe, die ein Brennen, einen Juckreiz, einen Niesreiz, eine vermehrte Sekretbildung oder eine Kombination mindestens zweier dieser Reize verursachen. Entsprechende Stoffe und deren üblicherweise einzusetzenden Mengen sind dem Fachmann bekannt. So basieren einige der den Nasen- und/oder Rachenraum reizenden Stoffe auf einem oder mehreren Inhaltsstoffen oder einem oder mehreren Pflanzenteilen einer Scharfstoffdroge. Entsprechende Scharfstoffdrogen sind dem Fachmann an sich bekannt und werden beispielsweise in "Pharmazeutische Biologie - Drogen und ihre Inhaltsstoffe" von Prof. Dr. Hildebert Wagner, 2., bearbeitete Auflage, Gustav Fischer Verlag, Stuttgart-New York, 1982, Seiten 82 ff., beschrieben. Die entsprechende Beschreibung wird hiermit als Referenz eingeführt und gilt als Teil der Offenbarung.

Ferner enthält die erfindungsgemäße Darreichungsform bevorzugt keine Antagonisten für die physiologisch wirksame Substanz (A), vorzugsweise keine Antagonisten gegen psychotrope Substanzen, insbesondere keine Antagonisten gegen Opioide. Für eine gegebene physiologisch wirksame Substanz (A) geeignete Antagonisten sind dem Fachmann bekannt und können als solche oder in Form entsprechender Derivate, insbesondere Ester oder Ether, oder jeweils in Form entsprechender physiologisch verträglicher Verbindungen, insbesondere in Form ihrer Salze oder Solvate vorliegen. Bevorzugt enthält die erfindungsgemäße Darreichungsform keine Antagonisten ausgewählt aus der Gruppe umfassend Naloxon, Naltrexon, Nalmefen, Nalid, Nalmexon, Nalorphin oder Naluphin, jeweils ggf. in Form einer entsprechenden physiologisch verträglichen Verbindung, insbesondere in Form einer Base, eines Salzes oder Solvates; und kein Neuroleptikum, z.B. eine Verbindung ausgewählt aus der Gruppe umfassend Haloperidol, Promethacin, Fluphenazin (Fluophenozin), Perphenazin, Levomepromazin, Thioridazin, Perazin, Chlorpromazin, Chlorprothixin (Chlorprotheaxin), Zuclopentixol (*Zucklopantexol*), Flupentixol (*Flupentexol*), Prothipendyl (Prithipendyl), Zotepin, Benperidol (Penperidol), Pipamperon (*Piparmeron*), Melperon (Melperol) und Bromperidol.

Darüber hinaus enthält die erfindungsgemäße Darreichungsform bevorzugt kein Emetikum. Emetika sind dem Fachmann bekannt und können als solche oder in Form entsprechender Derivate, insbesondere Ester oder Ether, oder jeweils in Form entsprechender physiologisch verträglicher Verbindungen, insbesondere in Form ihrer Salze oder Solvate vorliegen. Bevorzugt enthält die erfindungsgemäße Darreichungsform kein Emetikum auf Basis eines oder mehrerer Inhaltsstoffe von Radix Ipecacuanhae (Brechwurzel), z.B. auf Basis des Inhaltsstoffes Emetin, wie sie z.B. in "Pharmazeutische Biologie - Drogen und ihre Inhaltsstoffe" von Prof. Dr. Hildebert Wagner, 2., bearbeitete Auflage, Gustav Fischer Verlag, Stuttgart, New York 1982 beschrieben werden. Die entsprechende Literaturbeschreibung wird hiermit als Referenz eingeführt und gilt als Teil der Offenbarung. Bevorzugt enthält die erfindungsgemäße Darreichungsform auch kein Apomorphin als Emetikum.

Schließlich enthält die erfindungsgemäße Darreichungsform bevorzugt auch keinen Bitterstoff. Bitterstoffe sowie die für den Einsatz wirksamen Mengen sind der US-2003/0064099 A1 zu entnehmen, deren entsprechende Offenbarung als Offenbarung der vorliegenden Anmeldung gelten soll und hiermit als Referenz eingeführt wird. Beispiele für Bitterstoffe sind Aromaöle, wie Pfefferminzöl, Eukalyptusöl, Bittermandelöl, Menthol, Fruchtaromastoffe, Aromastoffe von Zitronen, Orangen, Limonen, Grapefruit oder Mischungen davon, und/oder Denatonium-Benzoat.

Somit enthält die erfindungsgemäße Darreichungsform bevorzugt weder Substanzen mit psychotroper Wirkung, noch den Nasen- und/oder Rachenraum reizende Stoffe, noch Antagonisten für die physiologisch wirksame Substanz (A), noch Emetika, noch Bitterstoffe.

In einer bevorzugten Ausführungsform enthält die erfindungsgemäße Darreichungsform als physiologisch wirksame Substanz (A) ein Nahrungsergänzungsmittel. Nahrungsergänzungsmittel enthalten bevorzugt einen oder mehrere Nährstoffe in konzentrierter, dosierter, lebensmittel-untypischer Form. Sie sollen die tägliche Nahrung in den Fällen ergänzen, in denen eine Versorgung durch die Nahrung unzureichend ist bzw. eine Ergänzung gewünscht wird. Bevorzugt ist das Nahrungsergänzungsmittel ausgewählt aus der Gruppe bestehend aus Vitaminen, Mineralstoffen, Spurenelementen, Enzymen, Fettsäuren, Aminosäuren, und Antioxidantien. Besonders bevorzugte Nahrungsergänzungsmittel sind Vitamine, Provitamine und deren Derivate, insbesondere Retinol, Calcitriol, Tocopherol, Phyllochinon, Thiamin, Riboflavin, Folsäure, Niacin (insbesondere Nicotinamid), Pantothensäure, Pyridoxal, Cobalamin, L-Ascorbinsäure, Biocytin, Biotin, und Karotinoide.

In einer bevorzugten Ausführungsform enthält die erfindungsgemäße Darreichungsform als physiologisch wirksame Substanz (A) einen Arzneistoff (= pharmazeutischen Wirkstoff), der eine Anwendung der Darreichungsform als Arzneimittel begründet und Ursache für deren Wirksamkeit ist. Als Arzneistoffe kommen in der erfindungsgemäßen Darreichungsform grundsätzlich alle bekannten Arzneistoffe infrage, wobei die Arzneistoffe als solche, in Form ihrer Derivate, insbesondere Ester oder Ether, oder jeweils in Form entsprechender physiologisch verträglicher Verbindungen, insbesondere in Form ihrer entsprechenden Salze oder Solvate, als Racemate oder in angereicherter Form eines oder mehrerer Stereoisomere (Enantiomere oder Diastereomere) in der erfindungsgemäßen Darreichungsform vorliegen können.

Besonders bevorzugt enthält die erfindungsgemäße Darreichungsform eine Substanz (A) oder mehrere Substanzen (A) ausgewählt aus der Gruppe bestehend aus
- Mitteln zur Behandlung und Vorbeugung von Erkrankungen des alimentären Systems und Stoffwechsels [A]; insbesondere Stomatologika [A01], Mitteln zur Behandlung und Vorbeugung von säurebedingten Erkrankungen [A02], Mitteln zur Behandlung und Vorbeugung von funktionellen gastrointestinalen Störungen [A03], Serotonin-5HT₃-Antagonisten [A04AA], Antihistaminika [A04AB], Mitteln zur Gallen- und Lebertherapie [A05], Laxanzien [A06], Intestinalen Antiinfektiva [A07A], Intestinalen Adsorbentien [A07B], Elektrolyten mit Kohlenhydraten [A07C], Intestinalen Antiphlogistika [A07E], Mikrobiellen Antidiarrhoika [A07F], Digestiva einschließlich Enzymen [A09], Antidiabetika [A10], Vitaminen [A11], Mineralstoffen [A12], Anabolika zur systemischen Anwendung [A14] und Appetit stimulierenden Mitteln [A15];
- Mitteln zur Behandlung und Vorbeugung von Erkrankungen des Bluts und der blutbildenden Organe [B]; insbesondere antithrombotischen Mitteln [B01], Antihämorrhagika [B02], Antianämika [B03] und anderen Hämatologika [B06];
- Mitteln zur Behandlung und Vorbeugung von Erkrankungen des kardiovaskulären Systems [C]; insbesondere Mitteln zur Herztherapie [C01], Antihypertonika [C02], Diuretika [C03], peripheren Vasodilatatoren [C04], Vasoprotektoren [C05], Antihypotonika [C06A], β-Adrenozeptor-Antagonisten [C07], Calciumkanalblockem [C08], Mitteln mit Wirkung auf das Renin-Angiotensin-System [C09] und Lipid senkenden Mitteln [C10];
- Dermatika [D]; insbesondere Antimykotika zur systemischen Anwendung [D01B], Antipsoriatika zur systemischen Anwendung [D05B], Aknemittel zur systemischen Anwendung [D10B];
- Mitteln zur Behandlung und Vorbeugung von Erkrankungen des Urogenitalsystems und Sexualhormonen [G]; insbesondere gynäkologischen Antiinfektiva und Antiseptika [G01], Wehen fördernden Mitteln [G02A], Wehen hemmenden Sympathomimetika [G02CA], Prolactinhemmern [G02CB], hormonalen Kontrazeptiva zur systemischen Anwendung [G03] und Urologika [G04];
- systemischen Hormonpräparaten ausschließlich Sexualhormonen und Insulinen [H]; insbesondere Hypophysen- und Hypothalamushormonen und Analoga [H01], Corticosteroiden zur systemischen Anwendung [H02], Schilddrüsenpräparaten [H03], Pankreashormonen [H04], und Mitteln zur Regulation der Calciumhomöostase [H05];
- Antiinfektiva zur systemischen Anwendung [J]; insbesondere Antibiotika zur systemischen Anwendung [J01], Antimykotika zur sytemischen Anwendung [J02], Mitteln gegen Mykobakterien [J04], antiviralen Mitteln zur systemischen Anwendung [J05], Immunsera und Immunglobulinen [J06], und Impfstoffen [J07];
- antineoplastischen und immunmodulierenden Mitteln [L]; insbesondere antineoplastischen Mitteln [L01], Mitteln zur endokrinen Therapie [L02], Immunstimulantien [L03] und Immunsuppressiva [L04];
- Mitteln zur Behandlung und Vorbeugung von Erkrankungen der Muskeln und des Skelettsystems [M]; insbesondere Antiphlogistika und Antirheumatika [M01], peripher wirkenden Muskelrelaxanzien [M03A], direkt wirkenden Muskelrelaxanzlen [M03C], Gichtmitteln [M04] und Mitteln zur Behandlung von Knochenerkrankungen [M05];
- Mitteln zur Behandlung und Vorbeugung von Erkrankungen des Nervensystems [N]; insbesondere Salicyclsäure und ihren Derivaten [N02BA], Pyrazolonen [N02BB], Aniliden [N02BE], Mutterkom-Alkaloiden [N02CA], Corticosteroid-Derivaten [N02CB], selektiven Serotonin-5HT₁-Agonisten [N02CC], Hydantoin-Derivaten [N03AB], Oxazolidin-Derivaten [N03AC], Succinimid-Derivaten [N03AD], Carboxamid-Derivaten [N03AF], Fettsäure-Derivaten [N03AG], Antiparkinsonmftteln [N04]), Antipsychotika [N05A], Antidepressiva [N06A], Antidementiva [N06D], Parasympathomimetika [N07A] und Antivertiginosa [N07C];
- antiparasitären Mitteln, Insektiziden und Repellenzien [P]; insbesondere Mitteln gegen Protozoen-Erkrankungen [P01], Antihelmintika [P02] und Mitteln gegen Ektoparasiten einschließlich Antiscabiosa, Insektiziden und Repellenzien [P03];
- Mitteln zur Behandlung und Vorbeugung von Erkrankungen des Respirationstrakts [R]; insbesondere Rhinologika [R01], Hals- und Rachentherapeutika [R02], Mitteln bei obstruktiven Atemwegserkrankungen [R03], Expektoranzien ausschließlich Kombinationen mit Antitussiva [R05C] und Antihistaminika zur systemischen Anwendung [R06];
- Mitteln zur Behandlung und Vorbeugung von Erkrankungen der Sinnesorgane [S]; insbesondere Otologika [S02];
- Allgemeinen Diätetika [V06] und Radiotherapeutlka [V10],
wobei auch hier die in eckigen Klammern angegebenen Bezeichnungen dem ATC-Index entsprechen, wie er von der WHO zur Klassifizierung der Arzneistoffe verwendet wird (bevorzugter Stand: Januar 2005 oder 2006).

Bevorzugt enthält die erfindungsgemäße Darreichungsform eine Substanz (A) oder mehrere Substanzen (A) ausgewählt aus der Gruppe bestehend aus 4-Aminomethylbenzoesäure, Abacavir, Abamectin, Abciximab, Abibendan, Abrin, Acamprosat, Acarbose, Acebutolol, Aceclidin, Aceclofenac, Acediasulfon, Acemetacin, Acenocoumarol, Acetazolamid, Acetessigsäure, Acetyl Digoxin, Acetylandromedol, Acetylcystein, β-Acetyldigoxin, Acetylhistamin, Acetylsalicylsäure, Acetylthiocholin, Aciclovir, Acipimox, Acitretin, Aclarubicin, Aconitin, Acriflavinium Chlorid, Acrivastin, Actinoquinol, Acylaminopenicillin, Adalimumab, Adapalen, Adefovir, Adefovir Dipivoxil, Adenosin, Adenosinphosphat, Adenosintriphosphat, Adipiodon, Adrenalin, Aescin, Agalsidase Alfa, Agalsidase Beta, Agaricinsäure, Ajmalin, Alanin, Albendazol, Alcuronium, Aldesleukin, Aldosteron, Alemtuzumab, Alendronsäure, Alfacalcidol, Alfuzosin, Algeldrat F, Alitretinoin, Alizaprid, Allantoin F, Allopurinol, Allylisorhodanat, Almasilat F, Almotriptan, α-Acetyldigoxin, Alprenolol, Alprostadil, Alteplase, Aluminiumglycinat F, Aluminiumhydroxyd F, Aluminiumphosphat F, Aluminiumtriformiat, Amantadin, Ambazon, Ambroxol, Ambutonium Bromid, Ameisensäure, Amicacin, Amidefrin, Amidotrizoesäure, Amifostin, Amikacin, Amilorid, Amino-Essigsäure, Aminoglutethimid, Aminophyllin, Aminoquinurid, Amiodaron, Amisulprid, Amitriptylin, Amitryptilin, Amlodipin, Amorolfin, Amoxicillin, Amphotericin B, Ampicillin, Amprenavir, Amylmetacresol, Amylnitrit, Anagrelid, Anakinra, Anastrozol, Ancrod, Anistreplase, Antazolin, Antithrombin III, Apomorphin, Apraclonidin, Aprepitant, Aprindin, Aprotinin, Arcitumomab, Arginin, Aripiprazole, Arsen Trioxid, Artemether, Articain, Ascorbinsäure, Asparagin, L-Asparaginase, Asparaginsäure, Atazanavir, Atenolol, Atomoxetin, Atorvastatin, Atosiban, Atovaquon, Atracurium, Atracurium Besilat, Atropin, Auranofin, Azapropazon, Azathioprin, Azelainsäure, Azelastin, Azidothymidin, Azithromycin, Azlocillin, Aztreonam, N2-Alanyllevoglutamid, p-Aminosalicylsäure,
Bacampicillin, Bacitracin, Baclofen, Balsalazid, Bambuterol, Bamethan, Bamipin, Barbexaclon, Bariumsulfat F, Barnidipin, Basiliximab, Batroxobin, Becaplermin, Beclometason, Bendamustin (Bedamustin), Befunolol, Bemiparin, Benactyzin, Benazepril, Bencyclan, Bendazac, Bendroflumethiazid, Benperidol (Penperidol), Benproperin, Benserazid, Benzaserid, Benzathin, Benzatropin, Benzbromaron, Benzocain, Benzoylperoxid, Benzyclan, Benzydamin, Benzylpenicillin, Benzylphenylglykolat, Betacaroten, Betahistidin, Betahistin, Betamethason, Betanechol, Betaxolol, Bethanechol Chlorid, Betiatid, Bevacizumab, Bexaroten, Bezafibrat, Bibenzonium Bromid, Bicalutamid, Bicisat, Bifonazol, Bimatoprost, Biperiden, Bisoprolol, Bivalirudin, Bleomycin, Blutgerinnungsfaktor VII, VIII, IX, X, XIII, Bomapin, Bomaprin, Bortezomib, Bosentan, Botulinum Toxin Typ B, Brimonidin, Brinzolamid, Brivudin, Bromhexin, Bromocriptin, Bromperidol, Brompheniramin, Brotizolam, Budesonid, Budipin, Bufexamac, Buflomedil, Bumetanid, Bunazosin, Buphenin, Bupivacain, Bupranolol, Bupropion, Buserelin, Buspiron, Busulfan, Butalamin, Butanilicain, Butenafin, Butetamat, Butinolin, Butizid, Butylscopolaminium,
5-Chlorcarvacrol, C1-Esterase-Inhibitor, Cabergolin, Cadexomer Jod, Cafedrin, Calcipotriol, Calcitonin, Calcitriol, Camylofin, Candesartan Cilexetil, Canrenoinsäure, Capecitabin, Capreomycin, Capsaicin, Captopril, Carazolol, Carbaldrat F, Carbamazepin, Carbasalat Calcium, Carbenoxolon, Carbidopa, Carbimazol, Carbinoxamin, Carboplatin, Carglumic Acid, Carglumsäure, Carmustin, Caroverin, Carteolol, Carvedilol, Caspofungin, Cefaclor, Cefadroxil, Cefalexin, Cefaloridin, Cefamandol, Cefazolin, Cefdinir, Cefepim, Cefetametpivotil, Cefixim, Cefodizim, Cefoperazon, Cefotaxim, Cefotiam, Cefoxitin, Cefpirom, Cefpodoxim, Cefpodoximproxetil, Cefprozil, Ceftazidim, Ceftibuten, Ceftizoxim, Ceftriaxon, Cefuroxim, Celecoxib, Celiprolol, Certoparin, Cetirizin, Cetrimid, Cetrimonium Bromid, Cetrorelix, Cetuximab, Cetylpiridinium, Chenodeoxycholsäure, Chinidin, Chinin, Chinin-Eisen-Citrat F, Chinin-Tannat F, Chlorambucil, Chloramphenicol, Chiorbutinol, Chlofiexidin, Chlormidazol, Chlorobutanol, Chloroquin, Chloroxylenol, Chlorphenamin, Chlorphenesin, Chlorphenoxamin, Chlorpromazin, Chlorprothixen (Chlorprotheaxin), Chlortalidon, Chlortetracyclin, Chlorzoxazon, Cholin, Chondroitinsulfat, Choriogonadotropin Alfa, Choriongonadotropin, Chrysarobin, Chymotrypsin, Ciclesonid, Cicletanin, Ciclopirox, Ciclosporin, Cidofovir, Cilastatin, Cilazapril, Cimetidin, Cinacalcet, Cinchocain, Cinnarizin, Cinolazepam, Ciprofloxacin, Cisaprid, Cisatracurium Besilat, Cisplatin, Citalopram, Citicolin, Cladribin,
Clarithromycin, Clavulansäure, Clemastin, Clenbuterol, Clindamycin, Clioquinol, Clobetasol, Clobetason, Clobutinol, Clocortolon, Clodronsäure, Clofibrat, Clomifen, Clomipramin, Clonazepam, Clonidin, Clopamid, Clopidogrel, Clostebolacetat, Clostridium Botulinum, Clotrimazol, Cloxiquin, Clozapin, Cocarboxylase, Colchicin, Colecalciferol, Colesevelam, Colestipol, Colestyramin, Colfosceril Palmitat, Colistin, Collyrium Zinci F, Corticorelin, Corticotrophin, Cortison, Cresol, Croconazol, Cromoglicinsäure, Crotamiton, Cryofluoran, Cumarin, Cyanamid, Cyanocobalamin, Cyclizin, Cyclobutyrol, Cyclopentolat, Cyclophosphamid, Cycloserin, Cyproheptadin, Cyproteron, Cystein, Cytarabin, Cytarabine,
2,4-Dichlorbenzylalkohol, 2-Diethylaminoethanol, Dacarbazin, Daclizumab, Dactinomycin, Dalfopristin, Dalteparin, Danaparoid, Danazol, Dantrolen, Dapiprazol, Dapson, Darbepoetin Alfa, Darifenacin, Daunorubicin, Deamol, Deanol (Deanolace), Decarbazin, Dectaflur F, Deferipron, Deferoxamin, Delapril, Demeclocyclin, Denaverin, Depreotid, Dequalinium, Desfluran, Desipramin, Desirudin, Deslanosid, Desloratadin, Desmeninol, Desmopressin, Desogestrel, Desoximetason, Desoxyribonudease, Detajmium, Dexamethason, Dexchlorpheniramin, Dexibuprofen, Dexketoprofen, Dexrazoxan, Dextran, Dextromethorphan, Diacerein, Diacetylmorphin, Dibenzepin, Dibotermin Alfa, Diclofenac, Diclofenamid, Didanosin, Dienestrol, Dienogest, Dlethylstilbestrol, Difloxacin, Diflucortolon, Diflunisal, Digitoxin, Digoxin, Dihydralazin, Dihydroergocomin, Dihydroergocristin, Dihydroergocryptin, Dihydroergotamin, Dihydroergotoxin, Dihydrotachysterol, Diisopropylamin, Dikaliumclorazepat, Diltiazem, Dimenhydrinat, Dimepranol, Dimercaprol, Dimethylsulfoxid, Dimetinden, Dinatriumselenit, Dinoprost, Dinoproston, Diosmin, Diphenhydramin, Diphenoxylat, Diphenylpyralin, Dipivefrin, Diprophyllin, Dipyridamol, Disopyramid, Distickstoffmonoxid, Distigmin, Disulfiram, Dithranol, Dixyrazin, D- . Norpseudoephedrin, Dobesilat Calcium, Dobutamin, Docetaxel, Dofetilid, Dolasetron, Domperidon, Donepezil, Dopamin, Dopexamin, Dornase Alfa, Dorzolamid, Dosulepin, Doxapram, Doxazosin, Doxepin, Doxorubicin, Doxycyclin, Doxylamin, Drofenin, Droperidol, Drospirenon, Drotrecogin Alfa, Duloxetine, Dutasterid, Dydrogesteron, N,N'-Dihydroxymethyl-Hamstoff,
Ebastin, Econazol, Ecothiopat Jodid, Efalizumab, Efavirenz, Eflornithin, Eisen IIIammonium citrat F, Eisenoxid superparamagnetisch, Elcatonin, Eletriptan, Emedastin, Emepronium, Emepronium Carragenat, Emetin, Emtricitabine, Enalapril, Enalaprilat, Enfluran, Enfuvirtid, Enoxacin, Enoxaparin, Entacapon, Ephedrin, Ephedrin Racephedrin, Epinastin, Epinephrin, Epirubicin, Eplerenon, Epoetin Alfa, Epoetin Beta, Epoetin Delta, Epoprostenol, Eprazinon, Eprosartan, Eptacog Alfa, Eptifibatid, Eptotermin Alfa, Erdostein, Ergocalciferol, Ergometrin, Ergotamid, Ertapenem, Erythromycin, Escitalopram, Esmolol, Esomeprazol, Estradiol, Estramustin, Estriol, Estron, Etacrynsäure, Etamivan, Etanercept, Ethacridin, Ethambutol, Ethaverin, Ethinylestradiol, Ethisteron, Ethosuximid, Etidronsäure, Etilefrin, Etodolac, Etofenamat, Etofibrat, Etofyllin, Etomidat, Etonogestrel, Etoposid, Etoricoxib, Everolimus, Exametazim, Exemestan, Ezetimib,
3-Fluortyrosin, Famciclovir, Famotidin, Felbamat, Felbinac, Felodipin, Fenbufen, Fendilin, Fenofibrat, Fenoterol, Fenticonazol, Fexofenadin, Fibrinogen, Fibrinolysin, Filgrastim, Finasterid, Flavoxat, Flecainid, Flucloxacillin, Fluconazol, Fludarabin, Fludeoxyglucose (¹⁸F], Fludrocortison, Flufenaminsäure, Flumazenil, Flumetason, Flunarizin, Flunisolid, Fluocinolon Acetonid, Fluocinonid, Fluocortolon, Fluphenazin (Fluophenozin), Fluorescein Dilaurat, Fluorescein-Natrium, Fluorometholon, Fluorouracil, Fluorphosphorsäure, Fluorsilan, Fluoxetil, Fluoxetin, Flupentixol, Fluphenazin, Flupirtin, Flupredniden, Flurbiprofen, Flutamid, Fluticason, Flutrimazol, Fluvastatin, Fluvoxamin, Folinsäure, Follitropin Alfa, Follitropin Beta, Folsäure, Fomepizol, Fomivirsen, Fondaparinux, Formestan, Formoterol, Fosamprenavir, Foscamet, Fosfestrol, Fosfomycin, Fosinopril, Fosphenytoin, Fotemustin, Framycetin, Framycetin, Frovatriptan, Fulvestrant, Furosemid, Fusafungin, Fusidinsäure, Fytinsäure,
Gabapentin, Gadobensäure, Gadobutrol, Gadodiamid, Gadopentetsäure, Gadoteridol, Gadotersäure, Gadotersäure-Meglumin, Gadoxetsäure, Galantamin, Gallopamil, Ganciclovir, Ganirelix, Gatifloxacin, Gemcitabin, Gemfibrozil, Gentamicin, Gepefrin, Gestoden, Glatiramer, Glibenclamid, Glibornurid, Gliclazid, Glimepirid, Glipizid, Gliquidon, Glisoxepid, Glucagon, Glutamin, Glutaminsäure, Glycopyrronium, Glycopyrronium Bromid, Glycyrrhetinsäure, Gonadorelin, Goserelin, Gramicidin, Granisetron, Grepafloxacin, Griseofulvin, G-Strophanthin, Guajacol, Guanethidin, Guanfacin,
¹³C-Hamstoff, 4-Hydroxybuttersäure, Halcinonid, Halofantrin, Halometason, Haloperidol, Halothan, Häm, Hämatoporphyrin, Heparin, Hepatitis B Vaccine, Heptaminol, Hexobarbital, Hexobendin, Hexoprenalin, Histamin, Histidin, Homatropin, Homofenazin, Humanalbumin, Hyaluronidase, Hydralazin, Hydrastinin, Hydrochinon, Hydrochlorothiazid, Hydrocortison, Hydrotalcit F, Hydroxocobalamin, Hydroxycarbamid, Hydroxychloroquin, Hydroxycin, Hydroxylamin, Hydroxyprogesteron, Hydroxyzin, Hymecromon,
Ibandronsäure, Ibopamin, Ibritumomab Tiuxetan, Ibuprofen, Ibutilid, Idarubicin, Ifosfamid, Iloprost, Imatinib, Imatinib Mesilate, Imidapril, Imiglucerase, Imipenem, Imipramin, Imiquimod, Immunocyanin, Indanazolin, Indapamid, Indinavir, Indium Chlorid [¹¹¹In], Indobufen, Indometacin, Indoramin, Infliximab, Inosin, Insulin, Insulin Aspart, Insulin Detemir, Insulin Glargin, Insulin Glulisine, Insulin Lispro, Interferon Alfa, Interferon Alfa-2b, Interferon Alfacon-1, Interferon Beta, Interferon Beta-1a, Interferon Beta-1b, Interferon Gamma, Iobitridol, Iod, Iodamid, lodixanol, loflupan [¹²³I], lohexol, lomeprol, lopamidol, lopentol, lopromid, losarcol, lotrolan, lotroxinsäure, loversol, loxaglinsäure, loxitalaminsäure, Ipatropium, Irbesartan, Irinotecan, Irinotecan, Isepamicin, Isoaminil, Isoconazol, Isofluran, Isoleucin, Isoniazid, Isonicotinsäure, Isoprenalin, Isosorbld, Isospagluminsäure, Isotretinoin, Isoxsuprin, Isradipin, Itraconazol,
Josamycin,
Kaliumpermanganat, Kallidinogenase, Kanamycin, Kawain, Kebuzon, Ketamin, Ketoconazol, Ketoprofen, Ketorofac, Ketotifen, Kollagenase, Kreosot,
Labetalol, Lacidipin, Lactitol, Lamivudin, Lamotrigin, Lanreotid, Lansoprazol, Laronidase, Latanoprost, Leflunomide, Lenograstim, Lepirudin, Lercanidipin, Letrozol, Leucin, Leuprorelin, Levallorphan, Levamisol, Levetiracetam, Levobunolol, Levobupivacain, Levocabastin, Levocetirizin, Levodopa, Levofloxacin, Levofolinat Calcium, Levomepromazin, Levomethadyl, Levonorgestrel, Levopropylhexedrin, Levosimendan, Levothyroxin, Lidocain, Lincomycin, Lindan, Linezolid, Liothyronin, Lisinopril, Lisurid, Lobelin, Lodoxamid, Lofepramin, Lomefloxacin, Lomustin, Lonazolac, Loperamid, Lopinavir, Loratadin, Lorazepam Oxid, Lornoxicam, Losartan, Loteprednol, Lovastatin, Lumefantrin, Lutropin Alfa, Lymecyclin, Lynestrenol, Lypressin, Lysin,
Magaldrat F, Magnesium Pidolat, Magnesium-L-Aspartat, Mangafodipir, Manidipin, Maprotilin, Mebendazol, Mebeverin, Meclofenoxat, Mecloxamin, Meclozin, Medrogeston, Medroxyprogesteron, Mefenaminsäure, Mefloquin, Megestrol, Melagatran, Melitracen, Melperon (Melperoi), Melphalan, Memantin, Menadion, Mepacrin, Mepartricin, Mephenytoin, Mepindolol, Mepivacain, Mepyramin, Mequinol, Mercaptamin, Mercaptopurin, Meropenem, Mesalazin, Mesna, Mesterolon, Mesuximid, Metaclazepam, Metamizol, Metamphetamin, Metenolon, Metenolonacetat, Metformin, Methanthelinium, Methazolamid, Methenamin, Methionin, Methohexital, Methotrexat, 5-Methoxypsoralen, 8-Methoxypsoralen, Methyl 5-aminolewlinat, Methylbenactyzium Bromid, Methyldopa, Methylergometrin, Methylprednisolon, Methylrosanilinium, Methyltestosteron, Methylthionium Chlorid, Methysergid, Metildigoxin, Metipranolol, Metoclopramid, Metoprolol, Methixen (Metrixen), Metronidazol, Mexiletin, Mezlocillin, Mianserin, Miconazol, Midodrin, Mifepriston, Miglitol, Miglustat, Milnacipran, Milrinon, Miltefosin, Minocyclin, Minoxidil, Mirtazapin, Misoprostol, Mitobronitol, Mitomycin, Mitotane, Mitoxantron, Mivacurium Chlorid, Mivacuronium, Mizolastin, Moclobemid, Moexipril, Molgramostim, Molsidomin, Mometason, Monochloressigsäure, Montelukast, Moroctocog Alfa, Moxaverin, Moxifloxacin, Moxonidin, Mupirocin, Mycophenolat Mofetil,
Nadifloxacin, Nadrolondecanonat, Nadroparin Calcium, Naftidrofuryl, Naftifin, Nalbuphin, Nalid, Nalmefen, Nalmexon, Naloxon, Naltrexon, Naluphin, Naphazolin, 2-Naphthol, Naproxen, Naratriptan, Naratriptan, Nateglinid, Natrium Aurothiomalat, Natrium Phenylbutyrate, Natriumfluorid, Natriumhyaluronat, Natriumiodid [¹³¹I], Natriummolybdat[⁹⁹Mo], Natriumphenylbutyrat, N-Butyl-P-Aminobenzoat, N-Butylscopolaminiumbromid, Nebivolol, Nedocromil, Nefazodon, Nefopam, Nelfinavir, Neomycin, Neostigmin, Neostigmin Metilsulfat, Netilmicin, Nevirapin, N-Heptyl-2-Phenylglycinat, Nicardipin, Nicergolin, Nicethamid, Niclosamin, Nicoboxil, Nicorandil, Nicotin, Nicotinaldehyd, Nicotinamid, Nicotinresinat, Nicotinsäure, Nicotinsäureester, Nicotinylalkohol, Nifedipin, Nifluminsäure, Nifuratel, Nilvadipin, Nimesulid, Nimodipin, Nimorazol, Nimustin (Nimustatin), Nisoldipin, Nitisinon, Nitrendipin, Nitric Oxide, Nitrofurantoin, Nitroglycerin, Nizatidin, N-Methylephedrin, Nonacog Alfa, Nonivamid, Noradrenalin, Norelgestromin, Norepinephrin, Norethisteron, Norfenefrin, Norfloxacin, Norgestimat, Norgestrel, Nortriptylin, Noscapin, Nystatin,
Obidoxim Chlorid, Octafluoropropane, Octocog Alfa, Octodrin, Octreotid, Odansetron, Ofloxacin, Olaflur F, Olanzapin, Olmesartan Medoxomil, Olopatadin, Olsalazin, Omeprazol, Omoconazol, Ondansetron, Opipramol, Oral Cholera Vaccine, Ordprenalin, Orlistat, Omipressin, Orphenadrin, Oseltamivir, Osteogenes Protein-1:
Bmp-7, Oxaprozin, Oxatomid, Oxcarbazepin, Oxedrintartrat, Oxetacain, Oxiconazol, Oxilofrin, Oxitropium, 2-Oxo-3-Methylbuttersäure, 2-Oxo-3-Methylvalerlansäure, 2-Oxo-3-Phenylpropionsäure, 2-Oxo-4-Methylvaleriansäure, Oxprenolol, Oxybuprocain, Oxybuprocain, Oxybutynin, Oxybutynin, Oxyfedrin, Oxymetazolin, Oxytetracyclin, Oxytocin,
Paclitaxel, Palinavir, Pallvizumab, Palonosetron, Pamidronsäure, Pancuronium (Pancurmium), Pantoprazol, Papaverin, Paracetamol, Paraldehyd, Parecoxib, Paricalcitol, Parnaparin, Paromomycin, Paroxetin, Pefloxacin, Pegfilgrastim, Peginterferon Alfa, Pegvisomant, Pemetrexed, Penbutolol, Penciclovir, Penfluridol, Penicillamin, Pentaerithrityl Tetranitrat, Pentamidin, Pentetrazol, Pentetreotid, Pentosan Polysulfat Natrium, Pentoxifyllin, Pentoxyverin, Perazin, Perchlorsäure, Perflenapent, Perflisopent, Perflutren, Pergolid, Perindopril, Perphenazin, Phenacetin, Phenamazid, Phenazon, Phenazopyridin, Pheniramin, Phenol, Phenolphthalein, Phenoxybenzamin, Phenoxymethylpenicillin, Phenprocoumon, Phentolamin, Phenylalanin, Phenylbutazon, Phenylephrin, Phenylpropanolamin, Phenyltoloxamin, Phenytoin, Phloroglucin, Pholedrin, Phthalylsulfathiazol, Physostigmin, Phytomenadion, Phytosterin, Picrinsäure, Pilocarpin, Pimecrolimus, Pimozid, Pinaverium Bromid, Pindolol, Pioglitazon, Pipamperon, Pipazetat, Pipecuronium Bromid, Pipemidsäure, Pipenzolat, Piperacillin, Piprinhydrinat, Piracetam, Pirarubicin, Pirbuterol, Pirenzepin, Piritramid, Piroxicam, Pivmecillinam, Pizotifen, Podophyllotoxin, Polidocanol, Polycarbophil, Polyestradiol Phosphat, Polymyxin B, Polymyxin-B, Polystyroisulfonsäure, Porfimer, Prajmalin, Prajmalium Bitartrat, Pramipexol, Pranoprofen, Prasteron, Pravastatin, Prazepam, Prazosin, Prednicarbat, Prednisolon, Prednison, Pregabalin, Proglumetacin (Preglumetacin), Pridinol, Prilocain, Primaquin, Primidon, Procain, Procainamid, Procarbazil, Procarbazin, Procyclidin, Progesteron, Proglumetacin, Proglumid, Proguanil, Prolin, Promethazin, Propacetamol, Propafenon, Propanolol, Propicillin, Propiverin, Propofol, Propranolol, Propylthiouracil, Propyphenazon, Protamin, Protaminsulfat, Protein C, Prothipendyl (Prithipendyl), Prothrombin, Protionamid, Protirelin, Proxymetacain, Proxyphyllin, Pseudoephedrin, Pulmonal, Pyrantel, Pyrazinamid, Pyridostigmin, Pyridostigmin Bromid, Pyridoxin, 3-Pyridylmethanol, Pyrimethamin, Pyrithion-Zink, Pyritinol, Pyrogallol, Pyrvinium, Pyrvinium Embonat,
Quecksilberamidochlorid, Quetiapin, Quinagolid, Quinapril, Quinupristin, Rabeprazol, Racecadotril, Racephedrin, Raloxifen, Raltitrexed, Ramipril, Ranitidin, Rasagilin, Rasburicase, Raubasin, Reboxetin, Repaglinid, Reproterol, Reserpin, Resorcin, Reteplase, Retinol, Reviparin, Ribavirin, Riboflavin, Rifabutin, Rifampicin, Rifamycin, Rifaximin, Rilmenidin, Riluzol, Rimexolon, Risedronsäure, Risperidon, Ritonavir, Rituximab, Rivastigmin, Rizatriptan, Rocuronium Bromid, Rofecoxib, Ropinirol, Ropivacain, Ropivacain, Rosiglitazon, Rotes Quecksilbersulfid F, Roxatidin, Roxithromycin,
Salbutamol, Salicylsäure, Salmeterol, Salpetersäure, Salpetrige Säure, Salverin, Samarium [¹⁵³Sm] Lexidronam, Saquinavir, Schwefelhexafluorid, Scopolamin, Selegilin, Selensulfid, Serin, Sermorelin, Sertaconazol, Sertindol, Sertralin, Sevelamer, Sevofluran, Sibutramin, Silberchlorid F, Sildenafil, Silibinin, Simvastatin, Sirolimus, Solifenacin, Solutio Formaldehydi, Somatostatin, Somatropin, Sotalol, Spagluminsäure, Spartein, Spectinomycin, Spiramycin, Spirapril, Spironolacton, Stavudin, Streptodomase, Streptokinase, Streptomycin, Strontium Ranelate, Strontiumchlorid, Strychnin, Sucralfat F, Sulbactam, Sulesomab, Sulfacetamid, Sulfadiazin, Sulfadimethoxin, Sulfaguanidin, Sulfamerazin, Sulfamethoxazol, Sulfamethoxydiazin, Sulfametrol, Sulfanilamid, Sulfasalazin, Sulfathiazol, Sulfisomidin, Sulindac, Sulodexid, Sulphur Hexafluoride, Sulpirid, Sulproston, Sultamicillin, Sultiam, Sumatriptan, Suxamethonium,
Tacalcitol, Tacrolimus, Tadalafil, Tamoxifen, Tamsulosin, Tasonermin, Taurolidin, Tazaroten, Tazobactam, Tegafur, Teicoplanin, Telithromycin, Telmisartan, Temoporfin, Temozolomid, Tenecteplase, Teniposid, Tenofovir, Tenofovir Disoproxil, Tenoxicam, Terazosin, Terbinafin, Terbutalin, Terfenadin, Teriparatid, Terizidon, Terlipressin, Testosteron, Testosteronpropionat, Testosteronundecanonat, Tetracain, Tetracosactid, Tetracyclin, Tetrafluoroborat-1+, Tetrofosmin, Tetryzolin, Thallium Chlorid [²⁰¹TI], Theobromin, Theodrenalin, Theodrenalin, Theophyllin, Thiamazol, Thiamin, Thiethylperazin, Thiocolchicosid, Thiopental, Thioridazin, Thiotepa, Threonin, Thrombin, Thrombokinase, Thymol, Thyrotropin Alfa, Tiagabin, Tianeptin, Tiaprid, Tibolon, Ticlopidin, Tiludronsäure, Timolol, Tinzaparin, Tioconazol, Tioguanin, Tiotropium Bromid, Tirilazad, Tirofiban, Tisopurin, Tizamidin, Tizanidin, Tobramycin, Tocainid, Tolazolin, Tolbutamid, Tolcapon, Tolfenaminsäure, Tolmetin, Tolperison, Tolterodin, Topiramat, Topotecan, Torasemid, Toremifen, Tramazolin, Trandolapril, Tranexamsäure, Tranylcypromin, Trapidil, Trastuzumab, Travoprost, Trazodon, Tretinoin, Triamcinolon, Triamcinolonacetonid, Triamteren, Trichloressigsäure, Triethylperazin, Trifluoperazin, Triflupromazin, Trihexyphenidyl, Trimebutin, Trimecain, Trimegeston, Trimetazidin, Trimethoprim, Trimipramin, Tripelenamin, Triprolidin, Triptorelin, Tritoqualin, Trofosfamid, Tromantadin, Trometamol, Tropicamid, Tropisetron, Trospium, Tryptophan, Tubocurarin Chlorid, Tulobuterol, Tyloxapol, Tyrosin, Tyrothricin,
Unoproston, Urapid, Urapidil, Urokinase, Ursodeoxycholsäure,
Valaciclovir, Valdecoxib, Valganciclovir, Valin, Valproinsäure, Valsartan, Vancomycin, Vardenafil, Vecuronium (Vecurmium), Vecuronium Bromid, Venlafaxin, Verapamil, - Verteporfin, Vigabatrin, Viloxacin, Vinblastin, Vincamin, Vincristin, Vindesin, Vinorelbin, Vinpocetin, Viquidil, Voriconazol, Votumumab,
Wasserstoffperoxid,
Xantinol Nicotinat, Ximelagatran, Xipamid, Xylometazolin,
Yohimbin, Yttrium⁹⁰Y Chlorid,
Zalcitabin, Zalepion, Zanamivir, Zidovudin, Zinc Acetate Dihydrate, Zinkchlorid, Zinkcitrat, Zinksulfat, Ziprasidon, Zofenopril, Zoledronsäure, Zolmitriptan, Zolpidem, Zolpidemtartrat, Zonisamid, Zopiclon, Zotepin und Zuclopenthixol (Zucklopantexol).

Die vorstehend genannten Verbindungen sind vorwiegend durch ihre intemationeln Freinamen (INN) benannt und dem Fachmann bekannt. Hinsichtlich weiterer Einzelheiten kann beispielsweise verwiesen werden auf International Nonproprietary Names (INN) for Pharmaceutical Substances, World Health Organisation (WHO).

In einer bevorzugten Ausführungsform enthält die erfindungsgemäße Darreichungsform eine Substanz (A) oder mehrere Substanzen (A) ausgewählt aus der Gruppe bestehend aus 1,1-(3-Dimethylamino-3-phenylpentamethylen)-6-fluor-1,3,4,9-tetrahydropyrano[3,4-b]indol, insbesondere das Hemicitrat; 1,1-[3-Dimethylamino-3-(2-thienyl)pentamethylen]-1,3,4,9-tetrahydropyrano[3,4-b]indol, insbesondere das Citrat; und 1,1-[3-Dimethylamino-3-(2-thienyl)pentamethylen]-1,3,4,9-tetrahydropyrano[3,4-b]-6-fluoroindol, insbesondere das Hemicitrat. Die vorstehend genannten Substanzen sind im Stand der Technik bekannt (vgl. WO 2004/043967, WO 2005/066183).

Des weiteren kann zusätzlich zur Erzielung der notwendigen Bruchfestigkeit der erfindungsgemäßen Darreichungsform mindestens ein natürliches, halbsynthetisches oder synthetisches Wachs (D) eingesetzt werden (= Komponente (D)). Bevorzugt sind Wachse mit einem Erweichungspunkt von mindestens 50°C, bevorzugter mindestens 55°C, noch bevorzugter mindestens 60°C, am bevorzugtesten mindestens 65°C und insbesondere mindestens 70°C. Besonders bevorzugt sind Carnaubawachs und Bienenwachs. Ganz besonders bevorzugt ist Carnaubawachs. Carnaubawachs ist ein natürliches Wachs, das aus den Blättern der Camaubapalme gewonnen wird und einen Erweichungspunkt von wenigstens 80°C aufweist. Beim zusätzlichen Einsatz der Wachskomponente wird diese zusammen mit wenigstens einem Polymeren (C) in solchen Mengen eingesetzt, dass die Darreichungsform eine Bruchfestigkeit von mindestens 400 N, bevorzugt mindestens 500 N, aufweist.

Als Hilfsstoffe (B) können die üblichen für die Formulierung von festen Darreichungsformen bekannten Hilfsstoffe verwendet werden. Vorzugsweise sind dies Weichmacher, wie Triacetin und Polyethylenglykol, vorzugsweise ein niedermolekulares Polyethylenglykol, Hilfsstoffe, die Wirkstofffreisetzung beeinflussende, vorzugsweise hydrophobe oder hydrophile, vorzugsweise hydrophile Polymere, ganz besonders bevorzugt Hydroxypropylmethylcellulose, und/oder Antioxidantien. Vorzugsweise werden als hydrophile Matrixmaterialien Polymere, besonders bevorzugt Celluloseether, Celluloseester und/oder Acrylharze verwendet. Ganz besonders bevorzugt werden als Matrixmaterialien Ethylcellulose, Hydroxypropylmethylcellulose, Hydroxypropylcellulose, Hydroxymethylcellulose, Poly(meth)acrylsäure und/oder deren Derivate, wie deren Salze, Amide oder Ester eingesetzt.

Als Antioxidantien eignen sich Ascorbinsäure, Butylhydroxyanisol (BHA), Butylhydroxytoluol (BHT), Salze der Ascorbinsäure, Monothioglycerln, phosphorige Säure, Vitamin C, Vitamin E und dessen Derivate, Natriumbisulfit, besonders bevorzugt Butylhydroxytoluol oder Butylhydroxyanisol und α-Tocopherol.

Das Antioxidanz wird vorzugsweise in Mengen von 0,01 bis 10 Gew.%, vorzugsweise 0,03 bis 5 Gew.%, bezogen auf das Gesamtgewicht der Darreichungsform, eingesetzt.

Die erfindungsgemäßen Darreichungsformen zeichnen sich dadurch aus, dass sie aufgrund ihrer Bruchfestigkeit mit Hilfe von üblichen Zerkleinerungsmitteln, wie Mörser und Pistill, nicht zu pulverisieren sind. Eine Überdosierung ist dadurch praktisch ausgeschlossen. Um jedoch die Bruchfestigkeit der Darreichungsform weiter zu erhöhen, können die erfindungsgemäßen Darreichungsformen als Hilfsstoffe (B) weitere Bruchfestigkeits-erhöhende Mittel enthalten.

Die erfindungsgemäße Darreichungsform ist vorzugsweise fest und eignet sich zur oralen, vaginalen oder rektalen, vorzugsweise zur oralen Einnahme. Vorzugsweise ist sie nicht filmförmig. In einer weiteren bevorzugten Ausführungsform liegt die erfindungsgemäße Darreichungsform in Form einer Tablette, einer Kapsel oder in Form eines oralen osmotischen therapeutischen Systems (OROS) vor.

In einer bevorzugten Ausführungsform liegt die erfindungsgemäße Darreichungsform als Tablette vor.

Die erfindungsgemäße Darreichungsform kann in multipartikulärer Form, bevorzugt in Form von Mikrotabletten, Mikrokapseln, Mikropellets, Granulaten, Sphäroiden, Perlen oder Pellets, ggf. in Kapseln abgefüllt oder zu Tabletten verpresst, vorzugsweise zur oralen Verabreichung, vorliegen. Dabei weisen bevorzugt die einzelnen Partikel als solche eine Bruchfestigkeit von wenigstens 400 N auf, ggf. auch eine daraus hergestellte Tablette.

Vorzugsweise weisen die multipartikulären Formen eine Größe bzw. Größenverteilung Im Bereich von 0,1 bis 3 mm, besonders bevorzugt Im Bereich von 0,5 bis 2 mm auf. Je nach gewünschter Darreichungsform werden ggf. auch die üblichen Hilfsstoffe (B) zur Formulierung der Darreichungsform mitverwendet.

Die erfindungsgemäße Darreichungsform kann durch unterschiedliche Verfahren hergestellt werden, die nachfolgend näher erläutert werden; die Erfindung betrifft auch Darreichungsformen, welche nach einem dieser Verfahren erhältlich sind:
Das Verfahren zur Herstellung der erfindungsgemäßen Darreichungsform umfasst bevorzugt folgende Schritte:
   (a) Mischen der Komponente (A), ggf. (B), (C), ggf. (D);
   (b) ggf. Vorformen der aus Schritt (a) erhaltenen Mischung, vorzugsweise unter Wärme- und/oder Krafteinwirkung auf die aus (a) erhaltene Mischung, wobei die zugeführte Wärmemenge bevorzugt nicht dazu ausreicht, die Komponente (C) bis zu ihrem Erweichungspunkt zu erwärmen;
   (c) Härten der Mischung unter Wärme- und Krafteinwirkung, wobei die Wärmezufuhr während und/oder vor der Krafteinwirkung erfolgen kann und die zugeführte Wärmemenge dazu ausreicht, die Komponente (C) wenigstens bis zu ihrem Erweichungspunkt zu erwärmen;
   (d) ggf. Vereinzeln der gehärteten Mischung;
   (e) ggf. Formen der Darreichungsform; und
   (f) ggf. Beschichten mit einem Filmüberzug.

Die Wärme kann direkt oder mit Hilfe von Ultraschall zugeführt werden. Die Krafteinwirkung und/oder das Formen der Darreichungsform kann beispielsweise durch Direkttablettierung oder mit Hilfe von geeigneten Extrudern erfolgen, insbesondere mit Doppeischneckenextrudem (Doppelwalzenextrudem) oder Planetwalzenextrudem.

Die folgenden Verfahrensvarianten sind besonders bevorzugt:

### Verfahrensvariante 1:

In dieser Ausführungsform wird die erfindungsgemäße Darreichungsform vorzugsweise ohne Extruder-Einsatz hergestellt, indem vorzugsweise die Komponenten (A), ggf. (B), (C) und die ggf. vorhandene Komponente (D) gemischt werden und die resultierende Mischung ggf. nach einer Granulierung zu der Darreichungsform unter vorangehender oder gleichzeitiger Wärmeeinwirkung durch Krafteinwirkung geformt wird.

Diese Erwärmung und Krafteinwirkung zur Herstellung der Darreichungsform erfolgt ohne Extruder-Einsatz.

Die Mischung der Komponenten (A), ggf. (B), (C) und ggf. (D) erfolgt in einem dem Fachmann bekannten Mischgerät. Das Mischgerät kann beispielsweise ein Wälzmischer, Schüttelmischer, Schermischer oder Zwangsmischer sein.

Die resultierende Mischung wird vorzugsweise direkt zu der erfindungsgemäßen Darreichungsform unter vorangehender oder gleichzeitiger Wärmeeinwirkung durch Krafteinwirkung geformt. Beispielsweise kann die Mischung durch Direkttablettierung zu Tabletten geformt werden. Bei einer Direkttablettierung unter gleichzeitiger Wärmeeinwirkung wird mit Hilfe des Tablettierwerkzeugs, d. h. des Unterstempels, Oberstempels und der Matrize die zu verpressende Mischung zumindest bis zum Erweichungspunkt der Polymeren-Komponente (C) erhitzt und dabei verpresst. Bei einer Direkttablettierung unter vorangehender Wärmeeinwirkung wird das zu verpressende Gut unmittelbar vor der Tablettierung mindest bis zur Erweichungstemperatur der Komponete (C) erhitzt und anschließend mit Hilfe des Tablettierwerkzeugs verpresst.

So können beispielsweise in einem Tablettierwerkzeug mit Oberstempel, Unterstempel und Matrize für Tabletten mit 10 mm Durchmesser und einem Wölbungsradius von 8 mm bei einer Temperatur von 80 °C 300 mg Pulvermischung verpresst werden, wobei ein Pressdruck bei einer Krafteinwirkung von beispielsweise 2 kN oder 4 kN für beispielsweise 15 s aufrechterhalten wird.

Die resultierende Mischung aus den Komponenten (A), ggf. (B), (C) und ggf. der Komponente (D) kann auch zuerst granuliert und anschließend unter vorangehender oder gleichzeitiger Wärmeeinwirkung unter Krafteinwirkung zu der erfindungsgemäßen Darreichungsform geformt werden.

Bei jeder Krafteinwirkung erfolgt diese solange, bis die Darreichungsform eine Bruchhärte von mindestens 400 N, 420 N, 440 N, 460 N, 480 N, vorzugsweise mindestens 500 N, erreicht hat.

Die Granulierung kann durch Feuchtgranulation oder Schmelzgranulation in bekannten Granulatoren durchgeführt werden.

Jeder der erwähnten Verfahrensschritte, insbesondere die Erwärmungen und gleichzeitige oder nachfolgende Krafteinwirkung zur Herstellung der erfindungsgemäßen Darreichungsform erfolgt ohne Extruder-Einsatz.

### Verfahrensvariante 2:

Bei dieser Verfahrensvariante wird die erfindungsgemäße Darreichungsform durch Thermoverformung mit Hilfe eines Extruders hergestellt, ohne dass dabei eine Verfärbung des Extrudates zu beobachten ist.

Um das Ausmaß der Verfärbung durch diese Thermoformgebung zu untersuchen, wird zunächst die Färbung der Mischung der Ausgangkomponenten, aus denen die Darreichungsform besteht, ohne Zugabe einer farbgebenden Komponente, wie z. B. einem Farbpigment oder einer eigengefärbten Komponente (z. B. α-Tocopherol) festgestellt. Diese Zusammensetzung wird dann erfindungsgemäß thermogeformt, wobei sämtliche Verfahrensschritte einschließlich der Kühlung des Extrudates unter einer Inertgasatmosphäre durchgeführt werden. Im Vergleich dazu wird dieselbe Zusammensetzung nach demselben Verfahren aber ohne Inertgasatmosphäre hergestellt. Von der Ausgangszusammensetzung der erfindungsgemäß hergestellten Darreichungsform und der zum Vergleich hergestellten Darreichungsform wird die Färbung bestimmt. Die Bestimmung erfolgt mit Hilfe von "Munsell Book of Colour" von Munsell Colour Company Baltimore, Maryland, USA, Ausgabe 1966. Sofern die Färbung der erfindungsgemäß thermogeformten Darreichungsform eine Färbung mit der Identifikations-Nr. N 9,5/ höchstens jedoch eine Färbung mit der Identifikations-Nr. 5Y 9/1 aufweist, wird die Thermoverformung als eine "ohne Verfärbung" eingestuft. Sofern die Darreichungsform eine Färbung mit der Identifikations-Nr. 5Y 9/2 oder mehr aufweist bestimmt gemäß Munsell Book of Colour wird die Thermoverformung als eine "mit Verfärbung" eingestuft.

Überraschenderweise weisen die erfindungsgemäßen Darreichungsformen keine gemäß der vorstehenden Klassifizierung einzustufende Verfärbung auf, wenn das gesamte Herstellungsverfahren unter einer Inertgasatmosphäre, vorzugsweise unter einer Stickstoffatmosphäre mit Hilfe eines Extruders zur Thermoformgebung durchgeführt wird.

Diese erfindungsgemäße Variante zur Herstellung der erfindungsgemäßen Darreichungsformen ist gekennzeichnet dadurch, dass man
z) die Komponenten (A), ggf. (B), (C) und die gegebenenfalls vorhandene Komponente (D) mischt,
y) die resultierende Mischung im Extruder mindestens bis zum Erweichungspunkt der Komponente (C) erwärmt und unter Krafteinwirkung durch die Austrittsöffnung des Extruders extrudiert,
x) das noch plastische Extrudat vereinzelt und zur Darreichungsform formt oder
w) das abgekühlte und ggf. wieder erwärmte vereinzelte Extrudat zur Darreichungsform formt,
wobei die Verfahrensschritte y) und x) und ggf. die Verfahrensschritte z) und w) unter Inertgasatmosphäre, vorzugsweise Stickstoffatmosphäre, durchgeführt werden.

Das Mischen der Komponenten gemäß dem Verfahrensschritt z) kann ebenfalls bereits in dem Extruder erfolgen.

Die Mischung der Komponenten (A), ggf. (B), (C) und ggf. (D) kann auch in einem dem Fachmann bekannten Mischgerät erfolgen. Das Mischgerät kann beispielsweise ein Wälzmischer, Schüttelmischer, Schermischer oder Zwangsmischer sein. Vorzugsweise wird vor dem Abmischen mit den weiteren Komponenten die Komponente (C) und die gegebenenfalls vorhandene Komponente (D) erfindungsgemäß mit einem Antioxidanz versehen. Dies kann durch Vermischen der beiden Komponenten (C) und dem Antioxidanz erfolgen, vorzugsweise indem das Antioxidanz in einem leicht flüchtigen Lösungsmittel aufgelöst oder suspendiert und diese Lösung oder Suspension mit der Komponente (C) und der gegebenenfalls vorhandenen Komponente (D) homogen vermischt und das Lösungsmittel durch Trocknung, vorzugsweise unter Inertgasatmosphäre, entfernt wird.

Die im Extruder bis mindestens zum Erweichungspunkt der Komponente (C) erwärmte, vorzugsweise schmelzflüssige Mischung aus dem Extruder wird durch eine Düse mit mindestens einer Bohrung extrudiert.

Zur Durchführung des erfindungsgemäßen Verfahrens ist der Einsatz geeigneter Extruder erforderlich, bevorzugt Schneckenextruder (Walzenextruder), wobei Extruder, die mit zwei Schnecken (Walzen) ausgerüstet sind, besonders bevorzugt sind.

Die Extrusion wird vorzugsweise so durchgeführt, dass die Aufweitung des Extrusionsstrangs infolge der Extrusion vorzugsweise höchstens 50% beträgt, d.h. dass beispielsweise bei Verwendung einer Extrusionsdüse mit einem Durchmesser von 6 mm der extrudierte Strang einen Durchmesser von höchstens 9 mm aufweist. Bevorzugter beträgt die Strangaufweitung höchstens 40%, noch bevorzugter höchstens 35%, am bevorzugtesten höchstens 30% und insbesondere höchstens 25%. Es wurde überraschend festgestellt, dass bei zu starker mechanischer Beanspruchung des extrudierten Materials im Extruder eine erhebliche Strangaufweitung erfolgt, welche in unerwünschten Unregelmäßigkeiten der Eigenschaften, insbesondere der mechanischen Eigenschaften des extrudierten Strangs resultiert.

Vorzugsweise weist der Extruder mindestens zwei Temperaturzonen auf, wobei in der ersten Zone, die sich an eine Einzugs- und ggf. Mischzone anschließt, das Aufheizen der Mischung bis mindestens zum Erweichungspunkt der Komponente (C) stattfindet. Der Durchsatz der Mischung liegt vorzugsweise bei 2,0 kg bis 8,0 kg/Stunde.

Nach Erwärmung bis mindestens zum Erweichungspunkt der Komponente (C) wird die aufgeschmolzene Mischung mit Hilfe der Schnecken gefördert, weiter homogenisiert, komprimiert bzw. kompaktiert, so dass sie unmittelbar vor dem Austritt aus der Extruderdüse einen Mindestdruck von 5 bar, vorzugsweise mindestens 10 bar aufweist, und durch die Düse als Extrusionsstrang oder Extrusionsstränge, je nachdem wie viele Bohrungen die Düse aufweist, extrudiert. Die Düsengeometrie bzw. die Geometrie der Bohrungen ist frei wählbar. So kann die Düse bzw. die Bohrungen einen runden, oblongen oder ovalen Querschnitt aufweisen, wobei der runde Querschnitt vorzugsweise einen Durchmesser von 0,1 mm bis 15 mm, der oblonge Querschnitt vorzugsweise mit einer maximalen Längsanpassung von 21 mm und einer Querausdehnung von 10 mm vorliegt. Vorzugsweise hat die Düse bzw. die Bohrungen einen runden Querschnitt. Der Mantel des erfindungsgemäß zum Einsatz kommenden Extruders kann beheizt oder gekühlt werden. Die entsprechende Temperierung, d.h. Beheizung oder Kühlung, richtet sich danach, dass die zu extrudierende Mischung mindestens eine Durchschnittstemperatur (Produkttemperatur) entsprechend der Erweichungstemperatur der Komponente (C) aufweist und nicht über eine Temperatur steigt, bei welcher die zur verarbeitende physiologisch wirksame Substanz (A) Schaden nehmen kann. Vorzugsweise wird die Temperatur der zu extrudierenden Mischung unter 180°C, vorzugsweise unter 150°C, aber mindestens auf die Erweichungstemperatur der Komponente (C), eingestellt.

Nach der Extrusion der geschmolzenen Mischung und gegebenenfalls Kühlung des extrudierten Stranges bzw. der extrudierten Stränge erfolgt vorzugsweise eine Vereinzelung der Extrudate. Diese Vereinzelung kann vorzugsweise durch Zerschneiden der Extrudate mittels mitlaufender oder rotierender Messer, Wasserstrahlschneider, Drähten, Klingen oder mit Hilfe von Laserschneidem durchgeführt werden.

Für eine Zwischenlagerung bzw. Endlagerung des ggf. vereinzelten Extrudates bzw. der Endform der erfindungsgemäßen Darreichungsform ist keine Inertgasatmosphäre notwendig.

Das vereinzelte Extrudat kann mit üblichen Methoden pelletisiert oder zu Tabletten verpresst werden, um der Darreichungsform die Endform zu geben. Es ist aber auch möglich, das strangförmige Extrudat nicht zu vereinzeln und mit Hilfe von gegenläufigen Kalanderwalzen, die auf ihren Umlaufmantel gegenüberliegende Vertiefungen aufweisen, zur Endform, vorzugsweise zu einer Tablette, zu formen und diese mit Hilfe üblicher Methoden zu vereinzeln.

Sollte das gegebenenfalls vereinzelte Extrudat nicht sofort zur Endform geformt, sondern zur Lagerung abgekühlt werden, so ist nach der Lagerung für eine Inertgasatmosphäre, vorzugsweise für eine Stickstoffatmosphäre zu sorgen, die bei einem Erwärmen des gelagerten Extrudats bis zur Plastifizierung und endgültigen Formgebung zur Darreichungsform eingehalten werden muss.

Die Krafteinwirkung im Extruder auf die zumindest plastifizierte Mischung wird durch Steuerung der Umdrehungsgeschwindigkeit der Fördereinrichtung im Extruder und deren Geometrie und durch die Dimensionierung der Austrittsöffnung so eingestellt, dass sich im Extruder vorzugsweise vor der unmittelbaren Extrudierung der plastifizierten Mischung der dafür notwendige Druck aufbaut. Durch einfache Vorversuche können für die jeweilige Zusammensetzung die notwendigen Extrusionsparameter festgestellt werden, die zu einer Darreichungsform mit einer Bruchfestigkeit von mindestens 400 N, vorzugsweise mindestens 500 N führen.

Zur Extrusion eignet sich beispielsweise ein Doppelschneckenextruder der Fa. Leistritz (Nürnberg) vom Typ Micro 27 GL 40 D, Spindeldurchmesser 18 mm. Es können Schnecken mit exzentrischen Schneckenenden eingesetzt werden. Als Düse kann eine beheizbare Runddüse mit einem Durchmesser von 8 mm dienen. Das gesamte Verfahren sollte unter einer N₂-Atmosphäre durchgeführt werden. Die Extrusionparameter können beispielsweise auf folgende Werte eingesetellt werden: Schneckendrehzahl: 100 Upm; Durchsatz: 4 kg/h; Produkttemperatur: 125°C; und Manteltemperatur: 120°C.

### Verfahrensvariante 3:

Bei dieser Verfahrensvariante zur Herstellung der erfindungsgemäßen Darreichungsform erfolgt die Wärmezufuhr mit Hilfe von Ultraschall.

Dazu wird zunächst eine homogene Mischung aus wenigstens der Komponente (A) und der Komponente (C) (= Bindemittel) hergestellt. Dieser Mischung können noch weitere Hilfsstoffe, wie zum Beispiel Füllstoffe, Weichmacher, Gleitmittel oder Farbstoffe, zugemischt werden. Vorzugsweise wird als Weichmacher ein niedermolekulares Polyethylenglykol verwendet.

Das Mischen kann mit Hilfe von üblichen Mischern durchgeführt werden. Beispielsweise sind als Mischer Wälzmischer, die auch als Fall-, Trommel- oder Rotationsmischer bekannt sind, Containermischer, Fassmischer (Rhönradmischer oder Taumelmischer) oder Schüttelmischer, Schermischer, Zwangsmischer, Pflugscharmischer, Planeten-Mischkneter, Z-Kneter, Sigma-Kneter, Fluidmischer oder Intensivmischer geeignet.

Die Auswahl des geeigneten Mischers hängt unter anderem von der Rieselfähigkeit und Kohäsionskräfte des Mischgutes ab.

Die Mischung wird anschließend einer Formgebung unterworfen. Vorzugsweise während oder nach der Ultraschalleinwirkung erfolgt die Formgebung der Mischung, vorzugsweise durch Kompaktierung.

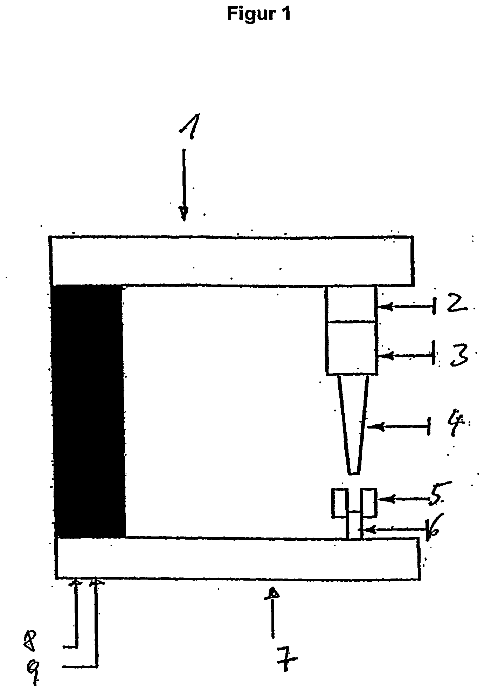

Bei der Ultraschalleinwirkung ist es besonders bevorzugt, dass ein direkter Kontakt zwischen der Mischung und der Sonotrode des Ultraschallgerätes vorhanden ist. Vorzugsweise wird gemäß dem erfindungsgemäßen Verfahren ein Ultraschallgerät eingesetzt, wie es in Figur 1 dargestellt ist.

In dieser Figur 1 bedeutet (1) die Presse, mit der die notwendige Kraft aufgebracht wird, (2) den Konverter, (3) den Booster, (4) die Sonotrode, (5) die Matrize zur Formgebung, (6) den Unterstempel, (7) die Grundplatte, (8) und (9) den Ultraschallgenerator und die Steuerung des Gerätes. Die verwendeten Bezugszeichen beziehen sich ausschließlich auf Figur 1.

Bei der Ultraschalleinwirkung sollte eine Frequenz von 1 kHz bis 2 MHz, vorzugsweise 15 bis 40 kHz, eingehalten werden. Die Dauer der Ultraschalleinwirkung sollte solange erfolgen, bis eine Erweichung des Polymers (C) erreicht wird. Vorzugsweise wird dies innerhalb von wenigen Sekunden, besonders bevorzugt innerhalb von 0,1 bis 5 Sekunden, vorzugsweise 0,5 bis 3 Sekunden, erreicht.

Durch die Ultraschalleinwirkung und die Krafteinwirkung erfolgt eine uniforme Energieübertragung, wodurch ein schnelles und homogenes Sintern der Mischung erreicht wird. Dadurch werden Darreichungsformen erhalten, die eine Bruchfestigkeit von mindestens 400 N, vorzugsweise mindestens 500 N haben und damit nicht pulverisierbar sind.

Bevor die Formgebung durchgeführt wird, kann nach dem Mischvorgang eine Granulierung der Mischung erfolgen, wonach die daraus resultierenden Granulate unter Ultraschalleinwirkung und Krafteinwirkung zu der Darreichungsform, wie Tabletten, geformt werden.

Die Granulierung kann in den dem Fachmann bekannten Maschinen und Apparaturen durchgeführt werden.

Sofern die Granulierung als Feuchtgranulierung durchgeführt wird, können als Granulierflüssigkeit Wasser oder wässrige Lösungen, wie z.B. Ethanol/Wasser oder Isopropanol/Wasser, eingesetzt werden.

Die Mischung oder die daraus hergestellten Granulate können auch zur weiteren Formgebung einer Schmelzextrusion unterworfen werden, wobei die Mischung unter Ultraschalleinwirkung und Krafteinwirkung zum Schmelzen gebracht wird und durch Düsen anschließend extrudiert wird. Die so gewonnenen Stränge oder der so gewonnene Strang kann mit Hilfe von bekannten Vorrichtungen auf die gewünschte Länge vereinzelt werden. Die so vereinzelten Formlinge können weiterhin gegebenenfalls unter Ultraschalleinwirkung und Krafteinwirkung zu der Endform verarbeitet werden.

Die Endformgebung zu der Darreichungsform erfolgt vorzugsweise unter Kraftanwendung in entsprechenden Formen.

Die vorstehend beschriebenen Formlinge können auch gemäß einem Kalandrierverfahren hergestellt werden, indem die Mischung bzw. die daraus hergestellten Granulate zunächst mittels Ultraschall- und Krafteinwirkung plastifiziert und durch eine entsprechende Düse extrudiert werden. Diese Extrudate werden anschließend zwischen zwei gegenläufig rotierenden Formwalzen zur endgültigen Form geformt, vorzugsweise unter Krafteinwirkung.

Wie bereits erwähnt, erfolgt die Formgebung zu der Endform der Darreichungsform durch Einsatz einer Mischung umfassend die Substanz (A) und das Polymer (C) mit einer Bruchfestigkeit von mindestens 400 N, vorzugsweise mindestens 500 N, vorzugsweise in Pulverform durch direkte Komprimierung unter Krafteinwirkung, wobei auf diese Mischung vor oder während der Krafteinwirkung Ultraschalleinwirkung vorgesehen ist. Die Kraft ist maximal die Kraft, die man üblicherweise zur Formgebung von Darreichungsformen, wie Tabletten, verwendet bzw. zum Verpressen von Granulaten zu der entsprechenden Endform.

Die erfindungsgemäß hergestellten Tabletten können auch Mehrschichttabletten sein.

Bei Mehrschichttabletten ist wenigstens die Schicht, welche die Substanz (A) enthält, einer Ultraschalleinwirkung und Krafteinwirkung zu unterziehen.

Die entsprechende notwendige Krafteinwirkung kann auch mit Hilfe von Extruderwalzen oder von Kalendarwalzen auf die Mischung aufgebracht werden. Vorzugsweise erfolgt die Formgebung der Darreichungsformen durch direktes Verpressen einer pulverförmigen Mischung aus den Komponenten der Darreichungsform oder entsprechenden daraus gebildeten Granulaten, wobei vorzugsweise während oder vor der Formgebung die Ultraschalleinwirkung erfolgt. Diese Einwirkung erfolgt solange, bis das Polymer (C) erweicht ist, was üblicherweise in weniger als 1 Sekunde bis maximal 5 Sekunden erreicht wird.

Als Presse ist beispielsweise eine Branson WPS, 94-003-A, Pneumatisch (Branson Ultraschall, Dietzenbach, Deutschland) mit planer Pressfläche geeignet, als Generator (2000 W) ein Branson PG-220A, 94-001-A analog (Branson Ultraschall) mit einem Sonotrodendurchmesser von 12 mm. Dabei kann eine Matrize mit einem Durchmesser von 12 mm, deren Boden ein Unterstempel mit einer planen Pressfläche von 12 mm Durchmesser bildet, verwendet werden. Geeignete Parameter zur Plastifizierung sind Frequenz: 20 kHz; Amplitude: 50%; Kraft: 250 N. Die Ultraschall- und Krafteinwirkung mit Hilfe der Sonotrode kann beispielsweise 0,5 Sekunden dauern, wobei Ultraschall- und Krafteinwirkung vorzugsweise gleichzeitig erfolgen.

### Verfahrensvariante 4:

Bei dieser Verfahrensvariante zur Herstellung der erfindungsgemäßen Darreichungsform werden die Komponenten (A), (C) und ggf. (D) und ggf. vorhandene Hilfsstoffe (B), wie Antioxidantien, Weichmacher und/oder retardierende Hilfsstoffe mit Hilfe eines Planetwalzen-Extruders zu der erfindungsgemäßen Darreichungsform verarbeitet.

Planetwalzen-Extruder sind bekannt und u.a. ausführlich im Handbuch der Kunststoff-Extrusionstechnik I (1989) "Grundlagen" im Kapitel 1.2 "Klassifizierung von Extrudern" Seiten 4 bis 6 beschrieben. Die entsprechende Beschreibung wird hiermit als Referenz eingeführt und gilt als Teil der Offenbarung.

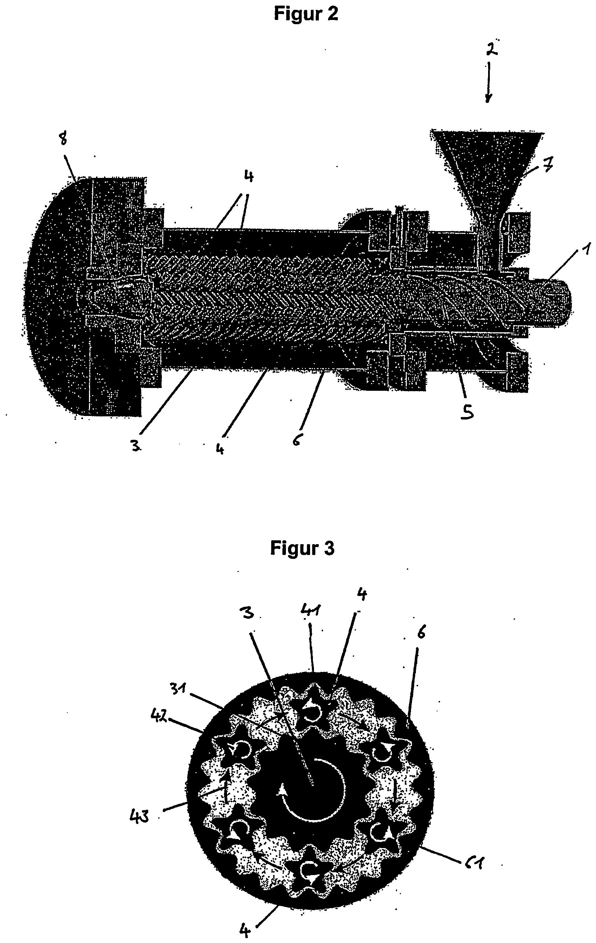

Im folgenden wird der Einsatz eines Planetwalzen-Extruders in dem erfindungsgemäßen Verfahren anhand der Figuren 2 und 3 erläutert. Diese Erläuterungen sind lediglich beispielhaft und schränken den allgemeinen Erfindungsgedanken nicht ein.
- Figur 2: zeigt den Schnitt durch einen Planetwalzen-Extruder und
- Figur 3: zeigt die Wirkungsweise des Planetwalzen-Extruders.

In Figur 2 ist ein Planetwalzen-Extruder dargestellt, der gemäß dem erfindungsgemäßen Verfahren eingesetzt werden kann. Dieser Extruder weist im wesentlichen eine Welle 1 auf, die bezogen auf die Transportrichtung der zu extrudierenden Mischung der vorstehend aufgeführten Komponenten zunächst als Einzugsschnecke 5 und im weiteren als Zentralspindel 3 des Planetwalzen-Extruders gestaltet ist. Um die Zentralspindel 3 sind vorzugsweise drei bis sieben Planetspindeln 4 angeordnet, die wiederum von einem Mantel in Form eines Gehäuses 6 umgeben sind.

Im Planetwalzen-Extruder wird unter Bezugnahme auf Figur 2 die Extrusion der im erfindungsgemäßen Verfahren zum Einsatz kommenden Zusammensetzung zur Herstellung einer pharmazeutischen Darreichungsform, vorzugsweise wie folgt durchgeführt. Wie durch Pfeil 2 dargestellt, werden die zu extrudierenden Komponenten durch die Dosiereinheit 7 in dem Bereich der Einzugsschnecke 5 dosiert und durch deren Drehung (Antrieb nicht dargestellt) in Richtung der Zentralspindel 3 befördert. Der Fachmann versteht, dass im Bereich der Einzugsschnecke ein Vermischen der Ausgangsstoffe (Komponenten) möglich ist. Es Ist aber auch möglich, die Komponenten der Darreichungsform vorzumischen und diese Mischung über die Dosiereinheit 7 in dem Bereich der Einzugsschnecke 5 zu dosieren. Im Einzugsbereich des Planetwalzen-Extruders wird die Mischung gefördert. Durch Erwärmung bis mindestens zum Erweichungspunkt der Komponente (C) wird die Mischung aufgeschmolzen und dort im Bereich der Zentralspindel, d. h. im Extrusionsbereich die aufgeschmolzene Mischung durch Zusammenwirkung der Zentralspindel 3 und der Planetspindeln 4 gefördert, weiter homogenisiert, komprimiert bzw. kompaktiert und durch die Düse 8 als Extrusionsstrang oder Extrusionsstränge, je nachdem wie viele Bohrungen die Düse aufweist, extrudiert. Die Düsengeometrie bzw. die Geometrie der Bohrungen ist frei wählbar. So kann die Düse bzw. die Bohrungen einen runden, oblongen oder ovalen Querschnitt aufweisen, wobei der runde Querschnitt vorzugsweise einen Durchmesser 0,1 mm bis 15 mm, der oblonge Querschnitt vorzugsweise mit einer maximalen Längsausdehnung von 21 mm und einer Querausdehnung von 10 mm vorliegt. Die Extrusionsdüse kann auch als Schlitzdüse gestaltet sein. Vorzugsweise hat die Düse bzw. die Bohrungen einen runden, ovalen oder oblongen Querschnitt. Sowohl der Mantel 6 des erfindungsgemäß zum Einsatz kommenden Planetwalzen-Extruders als auch die Zentralspindel können beheizt oder gekühlt werden. Die entsprechende Temperierung, d. h. Beheizung oder Kühlung, richtet sich danach, dass die zu extrudierende Mischung mindestens eine Durchschnittstemperatur entsprechend der Erweichungstemperatur der Komponente (C) aufweist und nicht über eine Temperatur steigt, bei der die zur verarbeitende Substanz (A) Schaden nehmen kann. Vorzugsweise wird die Temperatur der zu extrudierenden Mischung unter 180°C, vorzugsweise unter 150°C, aber mindestens auf die Erweichungstemperatur der Komponente (C), eingestellt. Die verwendeten Bezugszeichen beziehen sich ausschließlich auf Figuren 2 und 3.

Nach der Extrusion der geschmolzenen Mischung und gegebenenfalls Kühlung des extrudierten Stranges bzw. der extrudierten Stränge erfolgt eine nicht in Figur 2 dargestellte Vereinzelung der Extrudate. Diese Vereinzelung kann vorzugsweise durch Zerschneiden der Extrudate mittels mitlaufender oder rotierender Messer, Wasserstrahlschneider, Drähten, Klingen oder mit Hilfe von Laserschneidern durchgeführt werden.

Gegebenenfalls nach einem weiteren Abkühlen der vereinzelten Extrudate, die vorzugsweise in Scheiben vorliegen, erfolgt gegebenenfalls ein Umformen in die Endform der Darreichungsform, wobei wenn nötig, wieder Wärmeeinwirkung erfolgt.

Diese Formgebung beispielsweise zu Tabletten kann dadurch erfolgen, dass das plastische Extrudat mit Hilfe von zwei gegenläufig angetriebenen Walzen mit vorzugsweise zur Plastifizierung einander gegenüberliegenden Vertiefungen im Walzenmantel, deren Ausführung die Tablettenform bestimmt, unter Verpressung geformt wird.

Es ist aber auch möglich, aus den vereinzelten Extrudaten die Tabletten jeweils mit Hilfe einer gegebenenfalls beheizten Matrize und mindestens einem formgebenden Stempel zu formen. Dazu können vorzugsweise die nach der Vereinzelung des extrudierten Stranges erhaltenen zylinderförmigen Granulate verwendet werden. Außer zu Tabletten verpresst, können diese Granulate oder andere erhaltene multipartikuläre Formen, wie Pellets oder Spheriode, auch in Kapseln abgefüllt werden, um als erfindungsgemäß hergestellte Darreichungsform verwendet zu werden.

In einer weiteren bevorzugten Ausführungsform können die durch mehrere Bohrungen der Extrusionsdüse extrudierten Stränge gegebenenfalls nach deren Abkühlung durch Verflechtung oder Umschlingung entsprechend einer Seilherstellung zu einem gegenüber den einzelnen extrudierten Strängen dickeren Strang zusammengeführt werden. Dieser Strang kann gegebenenfalls durch Anlösen mit geeignetem Lösungsmittel oder durch Erwärmung bis zum Erweichungspunkt des Polymers (C) und gegebenenfalls Entfernen des Lösungsmittels entsprechend der vorstehend aufgeführten Vereinzelung und Verformung eines einzelnen Stranges weiter verarbeitet werden.

Figur 3 zeigt eine Querschnitt durch den Planetwalzen-Extruder. Um die sich drehende Zentralspindel 3 sind mindestens drei, in dem gezeigten Fall 6, Planetspindeln 4 angeordnet, deren Flanken 41 zu einem mit dem Flanken 31 der Zentralspindel 4 und zum anderen mit den Flanken 61 des Mantels 6 des Planetwalzen-Extruders zusammenwirken. Durch die Drehung der Zentralspindel 3 und das Abrollen der jeweiligen Flanken aufeinander drehen sich die Planetspindeln 4 jeweils wie mit Pfeil 42 um ihre eigene Achse und wie Pfeil 43 verdeutlicht, um die Zentralspindel 4 herum. Dadurch wird die erfindungsgemäß angestrebte Komprimierung bzw. Kompaktierung der erfindungsgemäß zum Einsatz kommenden Komponentenmischung der erfindungsgemäß hergestellten Darreichungsformen bewirkt. Die verwendeten Bezugszeichen beziehen sich ausschließlich auf Figuren 2 und 3.

Sofern notwendig, kann der zum Einsatz kommende Planetwalzen-Extruder nicht nur einen Extrusionsbereich aufweisen, sondern mindestens noch einen weiteren, um gegebenenfalls auch die zu extrudierende Mischung entgasen zu können.

Das erfindungsgemäße Verfahren kann diskontinuierlich oder kontinuierlich, vorzugsweise kontinuierlich, durchgeführt werden.

Als Extruder eignet sich beispielsweise ein Planetwalzen-Extruder mit vier Planetspindeln vom Typ BCG 10 der Fa. LBB Bohle (Ennigerloh, Deutschland) mit einer Extrusionsdüse mit einem Durchmesser von 8 mm. Eine gravimetrische Dosierung von 3,0 kg pro Stunde ist geeignet. Die Extrusion kann beispielsweise mit einer Drehzahl von 28,6 Upm bei einer Produkttemperatur von ca. 88 °C durchgeführt werden.

### Verfahrensvariante 5:

Zur Durchführung dieser Variante zur Herstellung der erfindungsgemäßen Darreichungsform werden wenigstens die Komponenten (A), (C) und ggf. (D) und ggf. vorhandene Hilfsstoffe (B), wie Antioxidantien, Weichmacher und/oder retardierende Hilfsstoffe unter Zugabe eines Lösungsmittels für die Komponente (C), d.h. für das oder die Polymere (C), zu der Darreichungsform verarbeitet.

Dazu werden die Komponenten (A), ggf. (B), (C) und die ggf. vorhandene Komponente (D) gemischt und die resultierende Formulierungsmischung nach Zugabe des Lösungsmittels und ggf. nach einer Granulierung zu der Darreichungsform geformt.

Die Mischung der Komponenten (A), ggf. (B), (C) und ggf. (D) erfolgt in einem dem Fachmann bekannten Mischgerät. Das Mischgerät kann beispielsweise ein Wälzmischer, Schüttelmischer, Schermischer oder Zwangsmischer sein.

Die Zugabe des Lösungsmittels für das Polymere (C) erfolgt zumindest in solchen Mengen, das die Formulierungsmischung gleichmäßig befeuchtet wird.

Als Lösungsmittel für das Polymere (C) eignen sich vorzugsweise wässrige Lösungsmittel, wie Wasser, Mischungen von Wasser und aliphatischen Alkoholen, vorzugsweise Alkoholen mit C1 bis C6, Estern, Ethern, Kohlenwasserstoffen, besonders bevorzugt destilliertes Wasser, kurzkettige Alkohole, wie Methanol, Ethanol, Isopropanol, Butanol oder wässrige Alkohollösungen.

Die Zugabe des Lösungsmittels erfolgt vorzugsweise unter Rühren. Anschließend wird die gleichmäßig befeuchtete Masse getrocknet. Die Trocknung erfolgt vorzugsweise unter Wärmeeinwirkung bei Temperaturen, bei denen eine Verfärbung der Masse ausgeschlossen werden kann. Durch einfache Vorversuchte ist diese Temperatur feststellbar.

Vor oder nach der Trocknung kann die Masse auf Teilmassen aufgeteilt werden, die vorzugsweise jeweils der Masse einer Einheit der Darreichungsform entsprechen. Die entsprechend getrockneten Massen werden dann zu der Darreichungsform geformt.

Vorzugsweise geschieht dies unter Einsatz von Tablettenpressen.

Es ist auch möglich, die Befeuchtung der Formulierungsmischung so durchzuführen, dass vor der Zugabe des Lösungsmittels die Formulierungsmischung, vorzugsweise in Formen auf Teilmassen verteilt, in einem flüssigen Dispergierungsmittel unter Rühren zu dispergieren und dann das Lösungsmittel zuzugeben. Die Komponente (C) ist in dem Dispergiermittel nicht löslich, das mit dem Lösungsmittel mischbar sein muss.

Als Dispergiermittel eignen sich vorzugsweise hydrophile Lösungsmittel, wie aliphatische Alkohole, Ketone, Ester. Kurzkettige Alkohole werden bevorzugt eingesetzt.

Alternativ kann die Befeuchtung der Formulierungsmischung auch so erfolgen, dass das Lösungsmittel als Schaum in die Formulierungsmischung eingearbeitet wird. Vorzugsweise wird ein solcher Schaum des Lösungsmittels mit Hilfe hochtouriger Mixer, vorzugsweise unter Zugabe üblicher Schaumstabillsatoren, hergestellt. Beispielsweise eignen sich als Stabilisatoren hydrophile Polymere wie z.B. Hydroxypropylmethylcellulose.

Vorzugsweise wird auch der Schaum unter Rühren in die Formulierungsmischung eingearbeitet, wodurch vorzugsweise eine granulierte Masse erhalten wird.

Die granulierte Masse wird vor oder nach ihrer Aufteilung in Teilmassen, die vorzugsweise der Masse einer Einheit der Darreichungsform entspricht, getrocknet und anschließend zur Darreichungsform geformt.

Die Trocknung und Formung kann vorzugsweise wie vorstehend angegeben erfolgen. Das erfindungsgemäße Verfahren kann auch so durchgeführt werden, dass zu der Formulierungsmischung soviel Lösungsmittel zugegeben wird, dass eine formbare Paste entsteht.

Eine solche Paste kann vor oder nach ihrer Trocknung, die wie vorstehend aufgeführt, durchgeführt werden kann, in Teilmassen aufgeteilt werden und die getrockneten Massen gegebenenfalls nach einer weiteren Verteilung jeweils auf eine Masse entsprechend der Masse einer Einheit der Darreichungsform zur Darreichungsform geformt oder umgeformt werden.

Dabei ist es möglich, die Teilmassen in Form von Strängen auszubilden, die mit Hilfe eines Siebes oder eines Strangformers erzeugt werden können. Die getrockneten Stränge werden vorzugsweise vereinzelt und zur Darreichungsform geformt. Diese Formung erfolgt vorzugsweise mit Hilfe einer Tablettenpresse, unter Einsatz von Formwalzen oder mit Walzen ausgerüsteten Formbändern.

Es ist auch möglich, die Paste zu einem flächenförmigen Gebilde zu verarbeiten und aus dem getrockneten Gebilde die Darreichungsform zu stanzen.

Vorteilhafterweise wird die Paste mit Hilfe eines Extruders verarbeitet, wobei je nach Gestaltung der Extrusion diese Stränge oder flächenförmigen Gebilde erzeugt werden, die durch Abschlagen oder Schneiden bzw. Stanzen vereinzelt werden. Die vereinzelten Teilmassen können wie vorstehend ausgeführt zu der Darreichungsform geformt, verformt oder ausgestanzt werden. Entsprechende Vorrichtungen sind dem Fachmann bekannt.

Dabei kann das erfindungsgemäße Verfahren kontinuierlich oder diskontinuierlich durchgeführt werden.

Es ist auch möglich, zu der Formulierungsmischung soviel Lösungsmittel zuzugeben, dass zumindest die Polymerkomponente (C) gelöst wird. Eine solche Lösung oder Dispersion/Suspension wird vorzugsweise zu einem flächenförmigen Gebilde verarbeitet, wobei bevorzugt ein Extruder mit einer Flachdüse zum Einsatz kommt oder die Lösung auf eine flächenförmige, ebene Unterlage ausgegossen wird.

Nach Trocknung, wie vorstehend angegeben, können aus den flächenförmigen Gebilden die Darreichungsformen durch Stanzen oder Kalandrieren erhalten werden. Es ist auch möglich, die Lösung, wie vorstehend angegeben, zu Strängen zu verarbeiten und diese, bevorzugt nach ihrer Trocknung, zu vereinzeln und zur Darreichungsform zu formen.

Alternativ kann die Lösung auch in solchen Teilmengen aufgeteilt werden, dass sie nach dem Trocknen jeweils der Masse einer Einheit der Darreichungsform entspricht, wobei vorzugsweise dafür bereits Formen entsprechend der Form einer Einheit der Darreichungsform eingesetzt werden.

Sofern die Lösung in beliebige Teilmengen aufgeteilt wird, können die Teilmengen nach dem Trocknen gegebenenfalls wieder vereinigt und zu der Darreichungsform geformt werden, wie z. B. in eine Kapsel abgefüllt oder zu einer Tablette verpresst werden.

Vorzugsweise werden die mit Lösungsmittel versetzten Formulierungsmischungen bei Temperaturen von 20°C bis 40°C verarbeitet, wobei außer bei der Trocknung zur Entfernung des Lösungsmittels und des ggf. vorhandenen Dispergierungsmittels keine höheren Temperaturen angewendet werden. Die Temperatur zum Trocknen muss unterhalb der Zersetzungstemperatur der Komponenten gewählt werden. Gegebenenfalls kann nach der Formgebung zur Darreichungsform nochmals eine Trocknung entsprechend der vorstehend beschriebenen Trocknung erfolgen.

Es sind auch Kombinationen einzelner Verfahrensschritte der vorstehenden Verfahrensvarianten möglich, um die erfindungsgemäße Darreichungsform herzustellen.

Die vorstehend beschriebenen Verfahrensvarianten 2 und 4 umfassen die Extrusion einer Zusammensetzung, welche die Komponenten (A), (C), ggf. (B) und ggf. (D) umfasst. Als Extruder kommen dabei bevorzugt Doppelschneckenextruder oder Planetwalzenextruder zum Einsatz, wobei Doppelschneckenextruder besonders bevorzugt sind.

Es wurde überraschend gefunden, dass mit Hilfe von Planetwalzen- und Doppelschneckenextrudern Extrudate erhältlich sind, welche eine vorteilhafte Morphologie aufweisen. So wurde gefunden, dass unter geeigneten Bedingungen das Extrudat von einer Mantelfläche umgeben ist, welche als "Extrusionshaut" bezeichnet werden kann. Dabei handelt es sich um ein manschettenförmiges, tubuläres Gebilde, welches das Extrudat entlang seiner longitudinalen Extrusionsachse derart umgibt, dass die äußere Oberfläche dieses manschettenförmigen Gebildes die geschlossene Mantelfläche des Extrudat bildet. Lediglich die Stirnseiten des Extrudats werden üblicherweise nicht von der Extrusionshaut bedeckt.

Die Extrusionshaut unterscheidet sich hinsichtlich ihrer Morphologie vom Kern des Extrudats, welchen sie wie eine Manschette umgibt und mit dem sie bevorzugt nahtlos verbunden ist. Üblicherweise ist die Extrusionshaut im Querschnitt des Extrudats mit bloßem Auge zu erkennen, ggf. mit Hilfe eines Mikroskops, da sich infolge der unterschiedlichen Morphologie des den Kern bzw. die Extrusionshaut bildenden Materials auch deren optische Eigenschaften unterscheiden. Es scheint, dass infolge des Extrusionsprozesses das die Extrusionshaut bildende Material anderen mechanischen bzw. thermischen Einflüssen ausgesetzt wird als das den Kern bildende Material, wodurch eine heterogene, bei kreisförmigem Düsenquerschnitt radialsymmetrische, Morphologie des Extrusionsstrangs erzeugt wird. Dabei unterscheiden sich das Material, welches die Extrusionshaut bildet, und das Material, welches den Kern bildet, im wesentlichen hinsichtlich ihrer Morphologie, vorzugsweise jedoch nicht hinsichtlich ihrer Zusammensetzung, insbesondere nicht hinsichtlich des relativen Anteils der Komponenten (A), (C), ggf. (B) und ggf. (D).

Die Extrusionshaut bedeckt üblicherweise die gesamte Mantelfläche des Extrudats im Sinne einer einteiligen, röhrenförmigen Manschette, unabhängig davon, welche Düsengeometrie gewählt wird. So kann das Extrudat kreisförmige, elliptische oder auch andere Querschnittsflächen haben.

Die Extrusionshaut weist vorzugsweise eine einheitliche Schichtdicke auf. Bevorzugt liegt die Schichtdicke der Extrusionshaut im Bereich von 0,1 bis 4,0 mm, bevorzugter 0,15 bis 3,5 mm, noch bevorzugter 0,2 bis 3,0 mm, am bevorzugtesten 0,2 bis 2,5 mm und insbesondere 0,2 bis 2,0 mm. In einer bevorzugten Ausführungsform macht die Schichtdicke der Extrusionshaut in der Summe über beide gegenüberliegenden Seiten0,5 bis 50%, bevorzugter 1,0 bis 40%, noch bevorzugter 1,5 bis 35%, am bevorzugtesten 2,0 bis 30% und insbesondere 2,5 bis 25% des Durchmessers des Extrudats aus.

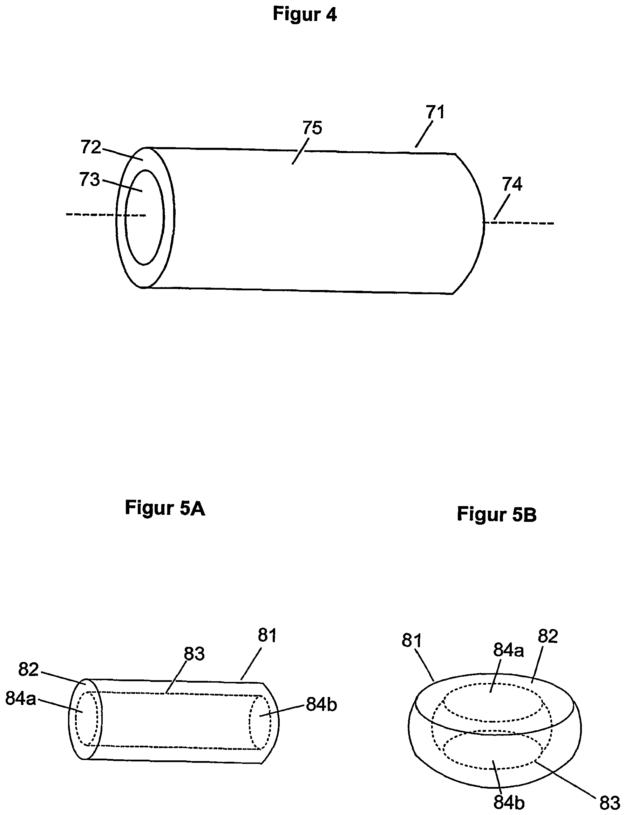

Figur 4 zeigt eine schematische Darstellung eines Extrudats (71) mit einer manschettenförmigen Extrusionshaut (72), welche den Kern (73) entlang der longitudinalen Extrusionsachse (74) vollständig umgibt. Die äußere Oberfläche der Extrusionshaut (72) bildet die Mantelfläche (75) des Extrudats (71).

Es wurde überraschend gefunden, dass Extrudate, welche eine derartige Extrusionshaut aufweisen, vorteilhafte mechanische Eigenschaften aufweisen. Sie sind besonders gut als Zwischenprodukte bei der Herstellung der erfindungsgemäßen Darreichungsformen geeignet, da sie vorteilhaft weiterberarbeitet werden können, insbesondere durch Vereinzeln und/oder Formen.

Werden die erfindungsgemäßen Darreichungsformen mit Hilfe eines Extrusionsverfahrens hergestellt, bei dem als Zwischenprodukt das vorstehend beschriebene, durch eine Extrusionshaut gekennzeichnete Extrudat erhalten wird, so zeichnen sich die daraus hergestellten Darreichungsformen vorzugsweise ebenfalls durch eine besondere Morphologie aus.

In einer bevorzugten Ausführungsform lassen sich diejenigen Bereiche, welche im extrudierten Zwischenprodukt die Extrusionshaut gebildet haben, im Querschnitt der Darreichungsform mit bloßem Auge erkennen, ggf. mit Hilfe eines Mikroskops. Dies hängt damit zusammen, dass üblicherweise durch die Weiterverarbeitung, insbesondere durch Vereinzeln und/oder Formen des Extrudats, die unterschiedliche Materialbeschaffenheit und damit auch die unterschiedlichen optischen Eigenschaften von Kern und Extrusionshaut erhalten bleiben. Nachfolgend wird derjenige Bereich der Darreichungsform, welcher im Zuge der Weiterverarbeitung des Extrudats (Zwischenprodukt) zur Darreichungsform aus der Extrusionshaut hervorgegangen ist, als "manschettenförmiger Bereich" bezeichnet.

Bevorzugt umfasst die erfindungsgemäße Darreichungsform einen manschettenförmigen Bereich und einen darin befindlichen Kern. Dabei ist der manschettenförmige Bereich vorzugsweise nahtlos mit dem Kern verbunden. Vorzugsweise enthalten sowohl der manschettenförmige Bereich als auch der Kern die im wesentlichen gleiche chemische Zusammensetzung, d.h. im wesentlichen den gleichen relativen Anteil der Komponenten (A), (C), ggf. (B) und ggf. (D). Das den manschettenförmigen Bereich bildende Material weist dabei eine Morphologie auf, welche sich von der Morphologie des den Kern bildenden Materials unterscheidet. Üblicherweise kommt diese unterschiedliche Morphologie auch in unterschiedlichen optischen Eigenschaften zum Ausdruck, so dass der manschettenförmige Bereich und der Kern im Querschnitt der Darreichungsform meist bereits mit bloßem Auge erkannt werden können.

Wurde die erfindungsgemäße Darreichungsform beschichtet, beispielsweise mit einem Filmüberzug, so ist der manschettenförmige Bereich zwischen dem Filmüberzug und dem Kern angeordnet.

Da das die Extrusionshaut enthaltene Extrudat (Zwischenprodukt) auf verschiedene Weise zur erfindungsgemäßen Darreichungsform weiterverarbeitet werden kann, kann auch der manschettenförmige Bereich unterschiedliche Anordnungen und Ausdehnungen innerhalb der erfindungsgemäßen Darreichungsform einnehmen. Allen Anordnungen ist jedoch gemeinsam, dass der manschettenförmige Bereich teilweise die Oberfläche des Kerns bedeckt, üblicherweise jedoch nicht dessen gesamte Oberfläche. Bevorzugt werden zwei gegenüberliegende Oberflächenseiten des Kerns nicht, oder zumindest nicht vollständig vom manschettenförmigen Bereich bedeckt. Mit anderen Worten weist der manschettenförmige Bereich vorzugsweise zwei gegenüberliegende Öffnungen/Aussparungen auf.

Der manschettenförmige Bereich kann eine einheitliche Schichtdicke aufweisen. Es ist jedoch auch möglich, dass bei der Herstellung infolge der Formung (z.B. Press-Fromung) des Extrudats unterschiedliche Bereiche der Extrusionshaut verschieden stark gestaucht oder gedehnt werden, so dass die Schichtdicke des manschettenförmigen Bereichs innerhalb der Darreichungsform variieren kann.

Bevorzugt weist der manschettenförmige Bereich eine Schichtdicke im Bereich von 0,1 bis 4,0 mm, bevorzugter 0,15 bis 3,5 mm, noch bevorzugter 0,2 bis 3,0 mm, am bevorzugtesten 0,2 bis 2,5 mm und insbesondere 0,2 bis 2,0 mm auf.

Figuren 5A und 5B zeigen zwei schematisierte, bevorzugte Anordnungen des manschettenförmigen Bereichs innerhalb der erfindungsgemäßen Darreichungsform. Die Darreichungsformen (81) weisen dabei einen manschettenförmigen Bereich (82) auf, welcher den Kern (83) teilweise umschließt. Die beiden gegenüberliegenden Oberflächenseiten (84a) und (84b) des Kerns (83) werden jedoch nicht vom manschettenförmigen Bereich (82) bedeckt.

Das Verfahren zur Herstellung der erfindungsgemäßen Darreichungsform wird bevorzugt kontinuierlich durchgeführt. Vorzugsweise beinhaltet das Verfahren die Extrusion einer homogenen Mischung der Komponenten (A), (C), ggf. (B) und ggf. (D). Dabei ist es besonders vorteilhaft, wenn das erhaltene Zwischenprodukt, beispielsweise der durch Extrusion erhaltene Strang, einheitliche Eigenschaften aufweist. Insbesondere wünschenswert sind eine einheitliche Dichte, einheitliche Wirsktoffverteilung, einheitliche mechanische Eigenschaften, einheitliche Porosität, einheitliche Oberflächenbeschaffenheit, etc. Nur unter diesen Voraussetzungen kann auch die Einheitlichkeit der pharmakologischen Eigenschaften, wie z.B. die Konstanz des Freisetzungsverhaltens, gewährleistet und die Menge an Ausschuss gering gehalten werden.

Vorzugsweise lässt sich das erfindungsgemäße Verfahren derat durchführen, dass die Menge an Ausschuss weniger als 25%, bevorzugter weniger als 20%, am bevorzugtesten weniger als 15% und insbesondere weniger als 10% beträgt, wobei die Ausschusskriterien die Standards der FDA im Hinblick auf die Intervariabilität des Gehalts der Komponente (A), ihr Freisetzungsprofil und/oder die Dichte der Darreichungsform im Vergleich zweier Darreichungsformen sind, welche vorzugsweise derselben Charge entnommen sind.

Es wurde überraschend gefunden, dass die vorstehend genannten Eigenschaften mit Hilfe von Doppelschneckenextrudern und Planetwalzenextrudern erhältlich sind, wobei Doppelschneckenextruder besonders bevorzugt sind.

Das erfindungsgemäße Verfahren beinhaltet vorzugsweise die Extrusion einer Mischung der Komponenten (A), (C), ggf. (B) und ggf. (D), vorzugsweise mit Hilfe eines Planetwalzenextruders oder eines Doppelschneckenextruders. Nach der Extrusion wird das Extrudat vorzugsweise vereinzelt, geformt und ggf. beschichtet, um die endgültige Darreichungsform zu erhalten.

In einer bevorzugten Ausführungsform des erfindungsgemäßen Verfahrens wird das Formen im plastifizierten Zustand der Mischung der Komponenten (A), (C), ggf. (B) und ggf. (D) durchgeführt. Es wurde überraschend gefunden, dass bei Extrusion bestimmter Polymere (C), insbesondere von Polyethylenoxiden hohen Molekulargewichts, Zwischenprodukte erhalten werden, welche einen gewissen Memory-Effekt, d.h. ein gewisses Rückstellverhalten zeigen: werden die vereinzelten Extrudate bei Umgebungstemperatur z.B. durch Press-Formung geformt, so werden Darreichungsformen erhalten, welche dazu neigen, durch Lagerung unter Stressbedingungen ihre ursprüngliche äußere Form einzunehmen, d.h. zu ihrer Form zurückzukehren, welche sie vor der Formung hatten.

Die Form der Darreichungsformen bei Lagerung unter Stressbedingungen, z.B. bei 40°C/75% rel. Feuchtigkeit, kann auch aus anderen Gründen instabil sein.

Der Memory-Effekt beeinträchtigt signifikant die Lagerstabilität der Darreichungsform, da im Zuge der Rückstellung der äußeren Form zahlreiche Eigenschaften der Darreichungsform verändert werden. Dasselbe gilt für jegliche Veränderungen der äußeren Form aufgrund anderer Ursachen.

Es wurde gefunden, dass beispielsweise in Abhängigkeit von den Extrusionsbedingungen eine signifikante Strangaufweitung erfolgt, welche mit einer Volumenzunahme des Extrudats einhergeht, d.h. mit einer Verringerung seiner Dichte. Diese Aufweitung kann durch anschließendes Pressformen des vereinzelten Extrudats bei einem ausreichenden Druck kompensiert werden, da unter diesen Bedingungen die Aufweitung des Materials rückgängig gemacht werden kann.

Wurde jedoch das Pressformen bei Umgebungstemperatur durchgeführt, so führt der Memory-Effekt bei Lagerung des komprimierten Extrudats dazu, dass dieses anschwillt und expandiert, wodurch das Volumen der Darreichungsform signifikant zunimmt.

Es wurde überraschend gefunden, dass ein solcher Memory-Effekt unterdrückt werden kann, wenn das Formen des vereinzelten Extrudats bei erhöhter Temperatur durchgeführt wird, d.h. im plastifizierten Zustand der Mischung der Komponenten (A), (C), ggf. (B) und ggf. (D). Vorzugsweise erfolgt das Formen bei einem Druck von wenigstens 1 kN, bevorzugter im Bereich von 2 kN bis 50 kN, z.B. mit Hilfe einer Tablettenpresse. Bevorzugt erfolgt das Formen bei einer Temperatur, welche etwa 40°C, bevorzugter etwa 30°C und insbesondere etwa 25°C unterhalb des Schmelzbereichs der Mischung der Komponenten (A), (C), ggf. (B) und ggf. (D) liegt. Der Schmelzbereich einer gegebenen Mischung kann mit Hilfe herkömmlicher Methoden bestimmt werden, vorzugsweise durch DSC (z.B. mit einem DSC Modell 2920 (TA Instruments, New Castle), ultrareinem Stickstoff als Spülgas mit einer Flussrate von 150 ml/min; einem ungefähren Probengewicht von 10-20 g, versiegelt in nicht-hermetischen Aluminiumpfannen; Temperaturgradient 10°C/min).

In einer bevorzugten Ausführungsform verändert sich die äußere Form der Darreichungsformen im wesentlichen nicht, wenn eine Lagerung für mindestens 12 h, bevorzugt für mindestens 24 h, bei 40°C und 75% rel. Feuchtigkeit erfolgt, vorzugsweise in offenen Behältern.

In einer bevorzugten Ausführungsform nimmt das Volumen der Darreichungsform bei Lagerung für mindestens 12 h, bevorzugt mindestens 24 h, bei einer Temperatur von 20°C unterhalb des Schmelzbereichs der Mischung der Komponenten (A), (C), ggf. (B) und ggf. (D), ggf. bei 40°C und 75% rel. Feuchtigkeit, um nicht mehr als 20% oder 17,5%, bevorzugter um nicht mehr als 15% oder 12,5%, noch bevorzugter um nicht mehr als 10% oder 7,5%, am bevorzugtesten um nicht mehr als 6,0%, 5,0% oder 4,0% und insbesondere um nicht mehr als 3,0%, 2,0% oder 1,0%, zu.

Die erfindungsgemäße Darreichungsform weist eine kontrollierte Freisetzung des Wirkstoffes auf. Sie eignet sich dabei vorzugsweise für eine zweimal tägliche Verabreichung an Patienten.

Die erfindungsgemäße Darreichungsform weist einen oder mehrere Substanzen (A) in einer darüber hinaus retardierten Form auf, wobei die Retardierung durch Einbetten der Substanz in eine retardierende Matrix erzielt wird. Die Substanzfreisetzung muss aber so gesteuert sein, dass durch die Zugabe von retardierenden Materialien keine Beeinträchtigung der notwendigen Härte erfolgt.

Die kontrollierte Freisetzung aus der erfindungsgemäßen Darreichungsform wird vorzugsweise durch Einbettung der Substanz in eine Matrix erzielt. Die als Matrixmaterialien dienenden Hilfsstoffe kontrollieren die Freisetzung. Matrixmaterialien können beispielweise hydrophile, gelbildende Materialien sein, woraus die Freisetzung hauptsächlich durch Diffusion erfolgt, oder hydrophobe Materialien, woraus die Freisetzung hauptsächlich durch Diffusion aus den Poren in der Matrix erfolgt.

Als Matrixmaterialien können physiologisch verträgliche, hydrophile Materialien verwendet werden, welche dem Fachmann bekannt sind. Vorzugsweise werden als hydrophile Matrixmaterialien Polymere, besonders bevorzugt Celluloseether, Celluloseester und/oder Acrylharze verwendet. Ganz besonders bevorzugt werden als Matrixmaterialien Ethylcellulose, Hydroxypropylmethylcellulose, Hydroxypropylcellulose, Hydroxymethyl cellulose, Poly(meth)acrylsäure und/oder deren Derivate, wie deren Salze, Amide oder Ester eingesetzt.

Ebenfalls bevorzugt sind Matrixmaterialien aus hydrophoben Materialien, wie hydrophoben Polymeren, Wachsen, Fetten, langkettigen Fettsäuren, Fettalkoholen oder entsprechenden Estern oder Ethern oder deren Gemische. Besonders bevorzugt werden als hydrophobe Materialien Mono- oder Diglyceride von C12-C30-Fettsäuren und/oder C12-C30-Fettalkohole und/oder Wachse oder deren Gernische eingesetzt.

Es ist auch möglich, Mischungen der vorstehend genannten hydrophilen und hydrophoben Materialien als Matrixmaterialien einzusetzen.

Das Matrixmaterial umfasst die Komponenten (C) und ggf. vorhandene Komponente (D), die zur Erzielung der erfindungsgemäß notwendigen Bruchfestigkeit von mindestens 400 N dienen.

Sofern die erfindungsgemäße Darreichungsform zur oralen Applikation vorgesehen ist, kann sie bevorzugt auch einen magensaftresistenten Überzug aufweisen, der sich in Abhängigkeit vom pH-Wert der Freisetzungsumgebung auflöst.
Durch diesen Überzug kann erreicht werden, daß die erfindungsgemäße Darreichungsform den Magentrakt unaufgelöst passiert und der Wirkstoff erst im Darmtrakt zur Freisetzung gelangt. Vorzugsweise löst sich der magensaftresistente Überzug bei einem pH-Wert zwischen 5 und 7,5 auf.

Entsprechende Materialien und Verfahren zur Retardierung von Wirkstoffen sowie zum Aufbringen magensaftresistenter Überzüge sind dem Fachmann beispielsweise aus "Coated Pharmaceutical Dosage Forms.- Fundamentals, Manufacturing Techniques, Biopharmaceutical Aspects, Test Methods and Raw Materials" von Kurt H. Bauer, K. Lehmann, Hermann P. Osterwald, Rothgang, Gerhart, 1. Auflage, 1998, Medpharm Scientific Publishers bekannt. Die entsprechende Literaturbeschreibung wird hiermit als Referenz eingeführt und gilt als Teil der Offenbarung.

Ein Gegenstand der Erfindung betrifft die Verwendung einer vorstehend beschriebenen, physiologisch wirksamen Substanz (A) und/oder eines vorstehend beschriebenen synthetischen oder natürlichen Polymers (C) zur Herstellung der erfindungsgemäßen Darreichungsform zur Vorbeugung und/oder Behandlung einer Erkrankung unter Verhinderung einer Überdosierung der physiologisch wirksamen Substanz (A), insbesondere infolge einer Zerkleinerung der Darreichungsform durch mechanische Einwirkung.

Die Erfindung betrifft auch die Verwendung einer vorstehend beschriebenen, physiologisch wirksamen Substanz (A) und/oder eines vorstehend beschriebenen synthetische oder natürlichen Polymers (C) zur Herstellung der erfindungsgemäßen Darreichungsform zur Verhinderung einer unbeabsichtigten Störung, insbesondere Aufhebung, der retardierten Freisetzung der physiologisch wirksamen Substanz (A) infolge einer Zerkleinerung der Darreichungsform durch mechanische Einwirkung.

Ferner betrifft die Erfindung die Verwendung einer erfindungsgemäßen Darreichungsform zur Herstellung eines Medikaments zur Vorbeugung und/oder Behandlung einer Erkrankung unter Verhinderung einer Überdosierung der physiologisch wirksamen Substanz (A), insbesondere infolge einer Zerkleinerung des Medikamentes durch mechanische Einwirkung.

Schließlich betrifft die Erfindung die Verwendung der erfindungsgemäßen Darreichungsform zur Herstellung eines Medikaments zur Vorbeugung und/oder Behandlung einer Erkrankung unter Verhinderung einer unbeabsichtigten Störung, insbesondere Aufhebung, der retardierten Freisetzung der physiologisch wirksamen Substanz (A) infolge einer Zerkleinerung des Medikaments durch mechanische Einwirkung.

Dabei ist die mechanische Einwirkung vorzugsweise ausgewählt aus der Gruppe bestehend aus Kauen, Mörsern, Hämmern und Anwenden von Apparaturen zur Pulverisierung herkömmlicher Darreichungsformen.

Bei den erfindungsgemäß erhaltenen Darreichungsformen wird die Bruchfestigkeit nach der aufgeführten Meßmethode bestimmt, wobei von Tabletten abweichende Darreichungsformen ebenso geprüft werden.

Zur Ermittlung der Bruchfestigkeit der erfindungsgemäßen Darreichungsform werden Darreichungsformen, bevorzugt Tabletten, mit einem Durchmesser von 10 mm und einer Höhe von 5 mm hergestellt.

Mit diesen Darreichungsformen, vorzugsweise Tabletten, wird gemäß der Methode zur Bestimmung der Bruchfestigkeit von Tabletten, veröffentlicht im Europäischen Arzneibuch 1997, Seite 143, 144, Methode Nr. 2.9.8. unter Einsatz der nachstehend aufgeführten Apparatur die Bruchfestigkeit der Darreichungsform bestimmt. Als Apparatur für die Messung wird eine Zwick Materialprüfmaschine "Zwick Z 2.5", Materialprüfmaschine Fmax 2.5 kN mit einem Traversenweg von max. 1150 mm, der durch einen Aufbau mit Hilfe einer Säule und einer Spindel einzustellen ist, einen freien Arbeitsraum nach hinten von 100 mm und einer zwischen 0,1 bis 800 mm/min. einstellbaren Prüfgeschwindigkeit und einer Software: testControl eingesetzt. Es wird ein Druckstempel mit schraubbaren Einsätzen und einem Zylinder (Durchmesser 10 mm), ein Kraftaufnehmer, Fmax. 1 kN, Durchmesser 8 mm, Klasse 0.5 ab 10 N, Klasse 1 ab 2 N nach ISO 7500-1, mit Hersteller-Prüfzertifikat M nach DIN 55350-18 (Zwick-Bruttokraft Fmax 1,45 kN) zur Messung eingesetzt (alles Apparaturen der Firma Zwick GmbH & Co. KG, Ulm, Deutschland) mit der Bestell-Nr. BTC-FR 2.5 TH. D09 für die Prüfmaschine, der Bestell-Nr. BTC-LC 0050N. P01 für den Kraftaufnehmer, der Bestell-Nr. BO 70000 S06 für die Zentriervorrichtung.

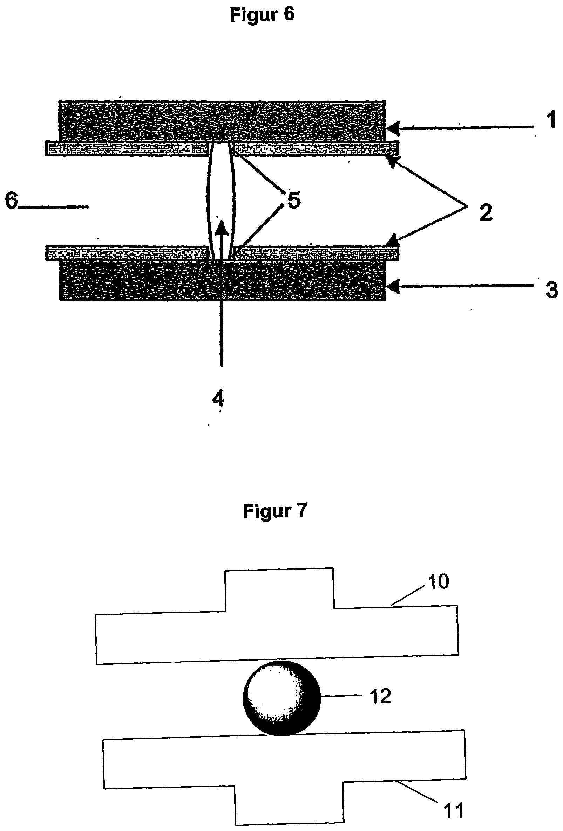

Figur 6 zeigt die Messung der Bruchfestigkeit einer Tablette, insbesondere die dafür eingesetzte Justierungsvorrichtung (6) der Tablette (4) vor und während der Messung. Dazu wird die Tablette (4) zwischen der oberen Druckplatte (1) und der unteren Druckplatte (3) der nicht dargestellten Vorrichtung zur Kraftaufbringung mit Hilfe von zwei 2-teiligen Einspannvorrichtungen, die jeweils mit der oberen bzw. unteren Druckplatte nach Einstellung des zur Aufnahme und zur Zentrierung der zu messenden Tablette notwendigen Abstands (5) fest verbunden (nicht dargestellt) werden. Zur Einstellung des Abstands (5) können die 2-teiligen Einspannvorrichtungen jeweils auf der Druckplatte, auf der sie gelagert sind, horizontal nach außen oder innen bewegt werden. Die verwendeten Bezugszeichen beziehen sich ausschließlich auf Figur 6.

Liegt die Darreichungsform multipartikulär vor, so kann die Bruchfestigkeit der einzelnen Partikel alternativ auch mit Hilfe zweier Druckplatten bestimmt werden, wie sie beispielsweise in Figur 7 abgebildet sind.

Figur 7 zeigt eine obere Druckplatte (10) und eine unter Druckplatte (11), zwischen welche die Probe (12), beispielsweise ein Pellet, eingebracht wird. Mit Hilfe der beiden Druckplatten erfolgt eine Krafteinwirkung auf die Probe. Die Auswertung des Messergebnisses erfolgt analog zu der vorstehend im Zusammenhang mit Figur 6 beschriebenen Methode.

Als bruchfest bei einer bestimmten Krafteinwirkung werden auch die Tabletten eingestuft, bei denen kein Bruch feststellbar, aber ggf. eine plastische Verformung der Tablette durch die Krafteinwirkung erfolgt ist.

Im folgenden wird die Erfindung anhand von Beispielen erläutert. Diese Erläuterungen sind lediglich beispielhaft und schränken den allgemeinen Erfindungsgedanken nicht ein.

In einer ersten Reihe von Beispielen wurden Diltiazem Hydrochlorid, Verapamil Hydrochlorid und Carbamazepin als Wirkstoffe (Substanz (A)) verwendet.

### Beispiel 1:

| Komponenten | Pro Tablette | Gesamtansatz |
|---|---|---|
| Diltiazem HCl | 90,0 mg | 720 mg |
| Polyethylenoxid, NF, MG 7 000 000 (Polyox WSR 303, Dow Chemicals) | 154,2 mg | 1233,6 mg |
| Gesamtgewicht | 244,2 mg | 1,9536 g |

Alle Komponenten wurden in einem Freifallmischer gemischt. Ein Tablettierwerkzeug mit Oberstempel, Unterstempel und Matrize für Tabletten mit 10 mm Durchmesser und einem Wölbungsradius von 8 mm wurde in einem Heizschrank auf 80°C erhitzt. Mittels des erhitzten Werkzeugs wurde die Pulvermischung verpresst, wobei der Pressdruck für mindestens 15 s aufrechterhalten wurde durch Einspannen des Tablettierwerkzeugs in einen Schraubstock.

Die Bruchfestigkeit der Tabletten wurde gemäß der angegebenen Methode mit der angegebenen Apparatur bestimmt. Bei einer Krafteinwirkung von 500 N trat kein Bruch der Tabletten auf. Die Tablette konnte mit einem Hammer nicht zerkleinert werden. Dies war auch mit Hilfe von Mörser und Pistill nicht möglich.

Die in-vitro-Freisetzung des Wirkstoffs aus der Zubereitung wurde in der Blattrührerapparatur nach Pharm. Eur. Bestimmt (Paddle mit Sinker). Die Temperatur des Freisetzungsmediums betrug 37°C und die Umdrehungsgeschwindigkeit des Rührers 50 min⁻¹. Zu Beginn der Untersuchung wurde jede Tablette in jeweils 900 ml künstlichen Magensaft pH 1,2 gegeben. Nach 30 Minuten wurde durch Zugabe von Lauge der pH-Wert auf 2,3 erhöht, nach weiteren 90 Minuten auf pH 6,5 und nach nochmals 60 weiteren Minuten auf pH 7,2. Die jeweils zu einem Zeitpunkt im Lösungsmedium befindliche freigesetzte Menge des Wirkstoffs wurde spektralphotometrisch bei 236 nm in 2 mm Küvetten bestimmt.

| Zeit | Freigesetzte Menge |
|---|---|
| 30 min | 12 % |
| 240 min | 43 % |
| 480 min | 63 % |
| 600 min | 71 % |
| 720 min | 77 % |

### Beispiel 2:

Analog zu Beispiel 1 wurden Oblong-Tabletten mit einem Durchmesser von 9 mm und einer Längsausdehnung von 20 mm mit folgender Zusammensetzung hergestellt:

| Komponenten | Pro Tablette | Gesamtansatz |
|---|---|---|
| Verapamil HCl | 240,0 mg | 1920 mg |
| Polyethylenoxid, NF, MG 7 000 000 (Polyox WSR 303, Dow Chemicals) | 411,4 mg | 3291,2 mg |
| Gesamtgewicht | 651,4 mg | 4,2112 g |

Die Bruchfestigkeit der Tabletten wurde gemäß der angegebenen Methode mit Hilfe der angegebenen Apparatur bestimmt. Bei einer Krafteinwirkung von 500 N trat kein Bruch der Tabletten auf.

Die in-vitro-Freisetzung des Wirkstoffs wurde analog zu Beispiel 1 bestimmt (UV-Detektion bei 279 nm) und betrug:

| Zeit | Freigesetzte Menge |
|---|---|
| 30 min | 6 % |
| 240 min | 20 % |
| 480 min | 30 % |
| 600 min | 35 % |
| 720 min | 39 % |

### Beispiel 3:

Analog zu Beispiel 1 wurden runde Tabletten mit einem Durchmesser von 20 mm folgender Zusammensetzung hergestellt:

| Komponenten | Pro Tablette | Gesamtansatz |
|---|---|---|
| Carbamazepin | 600 mg | 4800 mg |
| Polyethylenoxid, NF, MG 7 000 000 (Polyox WSR 303, Dow Chemicals) | 1028,5 mg | 8228,0 mg |
| Gesamtgewicht | 1628,5 mg | 13,028 g |

Die Bruchfestigkeit der Tabletten wurde gemäß der angegebenen Methode mit Hilfe der angegebenen Apparatur bestimmt. Bei einer Krafteinwirkung von 500 N trat kein Bruch der Tabletten auf.

Die in-vitro-Freisetzung des Wirkstoffs wurde analog zu Beispiel 1 bestimmt (UV-Detektion bei 285 nm) und betrug:

| Zeit | Freigesetzte Menge |
|---|---|
| 30 min | 1 % |
| 240 min | 5 % |
| 480 min | 9 % |
| 600 min | 11 % |
| 720 min | 13 % |

In einer weiteren Reihe von Beispielen wurde Nifedipin als Wirkstoff (Substanz (A)) verwendet.

### Beispiel 4:

Es wurden Tabletten folgender Zusammensetzung hergestellt:

| Rohstoff | Pro Tablette | Pro Ansatz | Anteilig |
|---|---|---|---|
| Nifedipin | 20 mg | 2 g | 10% |
| Polyethylenoxid 900 000 (Polyox WSR 1105 Dow Chemicals) | 180 mg | 18 g | 90% |

Nifedipin und Polyethylenoxid wurden im Freifallmischer gemischt. Die Mischung wurde auf einer Ekzentertablettenpresse (Modell EK 0 Fa. Korsch) zu Tabletten mit einem Gewicht von 200 mg gepresst. Es wurden runde Tabletten mit einem Durchmesser von 8 mm und einem Wölbungsradius von 8 mm hergestellt. Das Tablettierwerkeug bestehend aus Matrize, Ober- und Unterstempel und mit einem Durchmesser 10 mm und einem Wölbungsradius 8 mm wurden in einem Heizschrank auf 100°C erhitzt. Mittels heißem Werkzeug wurden die zuvor hergestellten Tabletten erneut verpresst, wobei der Pressdruck für mindestens 15s aufrechterhalten wurde.

Die Bruchfestigkeit der Tabletten wurde nach der Methode mit der angegebenen Apparatur bestimmt. Bei der Krafteinwirkung von 500 N trat kein Bruch auf. Die Tablette konnte mit einem Hammer nicht zerkleinert werden, dies war auch nicht mit Hilfe von Mörser und Pistill möglich.

### Beispiel 5:

Analog zu Beispiel 4 wurden Tabletten folgender Zusammensetzung hergestellt:

| Rohstoff | Pro Tablette | Pro Ansatz | Anteilig |
|---|---|---|---|
| Nifedipin | 20 mg | 2 g | 10% |
| Polyethylenoxid 600 000 (Polyox WSR 205 Dow Chemicals) | 180 mg | 18 g | 90% |

Die Bruchfestigkeit der Tabletten wurde nach der Methode mit der angegebenen Apparatur bestimmt. Bei der Krafteinwirkung von 500 N trat kein Bruch auf. Die Tablette konnte mit einem Hammer nicht zerkleinert werden, dies war auch nicht mit Hilfe von Mörser und Pistill möglich.

### Beispiel 6:

Analog zu Beispiel 4 wurden Tabletten folgender Zusammensetzung hergestellt:

| Rohstoff | Pro Tablette | Pro Ansatz | Anteilig |
|---|---|---|---|
| Nifedipin | 20 mg | 2 g | 10% |
| Polyethylenoxid 5 000 000 (Polyox WSR Coagulant Dow Chemicals) | 180 mg | 18 g | 90% |

Die Bruchfestigkeit der Tabletten wurde nach der Methode mit der angegebenen Apparatur bestimmt. Bei der Krafteinwirkung von 500 N trat kein Bruch auf. Die Tablette konnte mit einem Hammer nicht zerkleinert werden, dies war auch nicht mit Hilfe von Mörser und Pistill möglich.

### Beispiel 7:

Analog zu Beispiel 4 wurden Tabletten folgender Zusammensetzung hergestellt:

| Rohstoff | Pro Tablette | Pro Ansatz | Anteilig |
|---|---|---|---|
| Nifedipin | 20 mg | 2 g | 10% |
| Polyethylenoxid 100 000 (Polyox WSR N 10 Dow Chemicals) | 20 mg | 2 g | 10% |
| Polyethylenoxid 5 000 000 (Polyox WSR Coagulant Dow Chemicals) | 160 mg | 160 g | 80% |

Die Bruchfestigkeit der Tabletten wurde nach der Methode mit der angegebenen Apparatur bestimmt. Bei der Krafteinwirkung von 500 N trat kein Bruch auf. Die Tablette konnte mit einem Hammer nicht zerkleinert werden, dies war auch nicht mit Hilfe von Mörser und Pistill möglich.

In einer weiteren Serie wurden Tabletten mit Tramadol HCl als physiologisch wirksame Substanz (A) hergestellt:

### Beispiel 8:

| Komponenten | Pro Tablette | Gesamtansatz |
|---|---|---|
| Tramadolhydrochlorid | 100 mg | 100 g |
| Polyethylenoxid, NF, MFI (190°C bei 21,6 kg/10 min) <0,5 g. MG 7 000 000 (Polyox WSR 303, Dow Chemicals) | 200 mg | 200 g |
| Gesamtgewicht | 300 mg | 300 g |

Tramadolhydrochlorid und Polyethylenoxidpulver wurden in einem Freifallmischer gemischt. Ein Tablettierwerkzeug mit Oberstempel, Unterstempel und Matrize für Tabletten mit 10 mm Durchmesser und einem Wölbungsradius von 8 mm wurde in einem Heizschrank auf 80°C erhitzt. Mittels des erhitzten Werkzeugs wurden jeweils 300 mg der Pulvermischung verpreßt, wobei der Preßdruck für mindestens 15 s aufrechterhalten wurde durch Einspannen des Tablettierwerkzeugs in einen Schraubstock.

Die Bruchfestigkeit der Tabletten wurde gemäß der angegebenen Methode mit der angegebenen Apparatur bestimmt. Bei einer Krafteinwirkung von 500 N trat kein Bruch der Tabletten auf.

Die Tablette konnte mit einem Hammer nicht zerkleinert werden. Dies war auch mit Hilfe von Mörser und Pistill nicht möglich.

Die in-vitro-Freisetzung des Wirkstoffs aus der Zubereitung wurde in der Blattrührerapparatur nach Pharm. Eur. bestimmt. Die Temperatur des Freisetzungsmediums betrug 37°C und die Umdrehungsgeschwindigkeit des Rührers 75 min ⁻¹. Zu Beginn der Untersuchung wurde jede Tablette in jeweils 600 ml künstlichen Magensaft pH 1,2 gegeben. Nach 30 Minuten wurde durch Zugabe von Lauge der pH-Wert auf 2,3 erhöht, nach weiteren 90 Minuten auf pH 6,5 und nach nochmals 60 weiteren Minuten auf pH 7,2. Die jeweils zu einem Zeitpunkt im Lösungsmedium befindliche freigesetzte Menge des Wirkstoffs wurde spektralphotometrisch bestimmt.

| Zeit | Freigesetzte Menge |
|---|---|
| 30 min | 15 % |
| 240 min | 52 % |
| 480 min | 80 % |
| 720 min | 99 % |

### Beispiel 9:

Die Pulvermischung aus Beispiel 8 wurde in Portionen zu 300 mg auf 80°C erhitzt und in die Matrize des Tablettierwerkzeugs eingefüllt. Anschließend erfolgte die Verpressung. Die Tablette weist dieselben Eigenschaften wie die Tablette in Beispiel 8 auf.

### Beispiel 10:

| Rohstoff | Pro Tablette | Gesamtansatz |
|---|---|---|
| Tramadolhydrochlorid | 50 mg | 100 g |
| Polyethylenoxid, NF, MG 7 000 000 (Polyox WSR 303, Dow Chemicals) | 100 mg | 200 g |
| Gesamtgewicht | 150 mg | 300 g |

Tramadolhydrochlorid und die vorstehend angegebenen Komponenten wurden in einem Freifallmischer gemischt. Ein Tablettierwerkzeug mit Oberstempel, Unterstempel und Matrize für Tabletten mit 7 mm Durchmesser wurde in einem Heizschrank auf 80°C erhitzt. Mittels des erhitzten Werkzeugs wurden jeweils 150 mg der Pulvermischung verpreßt, wobei der Preßdruck für mindestens 15 s durch Einspannen des Tablettierwerkzeugs in einen Schraubstock aufrechterhalten wurde.

Die Bruchfestigkeit der Tabletten wurde gemäß der angegebenen Methode mit Hilfe der angegebenen Apparatur bestimmt. Bei einer Krafteinwirkung von 500 N trat kein Bruch der Tabletten auf.

Die in-vitro-Freisetzung des Wirkstoffs wurde wie in Beispiel 8 bestimmt und betrug:

| Zeit | Freigesetzte Menge |
|---|---|
| 30 min | 15 % |
| 240 min | 62 % |
| 480 min | 88 % |
| 720 min | 99 % |

### Beispiel 11:

| Rohstoff | Pro Tablette | Gesamtansatz |
|---|---|---|
| Tramadolhydrochlorid | 100 mg | 100 g |
| Polyethylenoxid, NF, MG 7 000 000 (Polyox WSR 303, Dow Chemicals) | 180 mg | 180 g |
| Xanthan, NF | 20 mg | 20 g |
| Gesamtgewicht | 300 mg | 300 g |

Tramadolhydrochlorid, Xanthan und Polyethylenoxid wurden in einem Freifallmischer gemischt. Ein Tablettierwerkzeug mit Oberstempel, Unterstempel und Matrize für Tabletten mit 10 mm Durchmesser und einem Wölbungsradius von 8 mm wurde in einem Heizschrank auf 80°C erhitzt. Mittels des erhitzten Werkzeugs wurden jeweils 300 mg der Pulvermischung verpreßt, wobei der Preßdruck für mindestens 15 s durch Einspannen des Tablettierwerkzeugs ein einen Schraubstock aufrechterhalten wurde.

Die Bruchfestigkeit der Tabletten wurde gemäß der angegebenen Methode mit Hilfe der angegebenen Apparatur gemessen. Bei einer Krafteinwirkung von 500 N trat kein Bruch der Tabletten auf. Die Tabletten wurden etwas plastisch verformt.

Die in-vitro-Freisetzung des Wirkstoffs aus der Zubereitung wurde wie in Beispiel 8 bestimmt und betrug:

| Zeit | Freigesetzte Menge |
|---|---|
| 30 min | 14 % |
| 240 min | 54 % |
| 480 min | 81 % |
| 720 min | 99 % |

### Beispiel 12:

| Rohstoff | Pro Tablette | Gesamtansatz |
|---|---|---|
| Tramadolhydrochlorid | 50 mg | 100 g |
| Polyethylenoxid, NF, MG 7 000 000 (Polyox WSR 303, Dow Chemicals) | 90 mg | 180 g |
| Xanthan, NF | 10 mq | 20 g |
| Gesamtgewicht | 300 mg | 300 g |

Tramadolhydrochlorid, Xanthan und Polyethylenoxid wurden in einem Freifallmischer gemischt. Ein Tablettierwerkzeug mit Oberstempel, Unterstempel und Matrize für Oblongtabletten mit 10 mm Länge und 5 mm Breite wurde in einem Heizschrank auf 90°C erhitzt. Mittels des erhitzten Werkzeugs wurden jeweils 150 mg der Pulvermischung verpreßt, wobei der Preßdruck für mindestens 15 s durch Einspannen des Tablettierwerkzeugs in einen Schraubstock aufrechterhalten.

Die Bruchfestigkeit der Tabletten wurde gemäß der angegebenen Methode mit Hilfe der angegebenen Apparatur gemessen. Bei einer Krafteinwirkung von 500 N trat kein Bruch der Tabletten auf. Die Tabletten wurden etwas plastisch verformt.

Die in-vitro-Freisetzung des Wirkstoffs aus der Zubereitung wurde wie in Beispiel 8 bestimmt und betrug:

| Zeit | Freigesetzte Menge |
|---|---|
| 30 min | 22 % |
| 120 min | 50 % |
| 240 min | 80 % |
| 360 min | 90 % |
| 480 min | 99 % |

### Beispiel 13:

Wie in Beispiel 8 beschrieben, wurde eine Tablette mit folgender Zusammensetzung hergestellt:

| Komponenten | ProTablette | Pro Ansatz |
|---|---|---|
| Oxycodon Hydrochlorid | 20,0 mg | 0,240 g |
| Xanthan NF | 20,0 mg, | 0,240 g |
| Polyethylenoxid, NF, MFI (190°C bei 21,6 kg/10 min < 0,5 g MG7 000 000 (Polyox WSR 303 Dow Chemicals) | 110,0 mg | 1,320 g |
| Gesamtgewicht | 150,0 mg, | 1,800 g |

Die Freisetzung des Wirkstoffs wurde wie folgt bestimmt:
Die In-vitro-Freisetzung des Wirkstoffs aus der Tablette wurde in der Blattrührerapparatur nach Pharm. Eur. bestimmt. Die Temperatur des Freisetzungsmediums betrug 37°C und die Umdrehungsgeschwindigkeit 75 U pro Minute. Als Freisetzungsmedium diente der in der USP beschriebene Phosphatpuffer pH 6,8. Die zum jeweiligen Prüfzeitpunkt im Lösungsmittel befindliche Menge des Wirkstoffs wurde spektralphotometrisch bestimmt.

| Zeit | Mittelwert |
|---|---|
| 0 min | 0 % |
| 30 min | 17 % |
| 240 min | 61 % |
| 480 min | 90 % |
| 720 min | 101,1 % |

Die Bruchfestigkeit der Tabletten wurde gemäß der angegebenen Methode mit Hilfe der angegebenen Apparatur gemessen. Bei einer Krafteinwirkung von 500 N trat kein Bruch der Tabletten auf.

### Beispiel 14:

Es wurden Tabletten folgender Zusammensetzung hergestellt:

| Rohstoff | Pro Tablette | Pro Ansatz | Anteilig |
|---|---|---|---|
| Tramadol HCl | 100 mg | 10 g | 20% |
| Polyethylenoxid 7 000 000 (Polyox WSR 303, Dow Chemicals) | 375 mg | 37,5 g | 75% |
| Carnaubawachs | 25 mg | 2,5 g | 5,0% |

Tramadol Hydrochlorid, Polyethylenoxid und Carnaubawachs wurden im Freifallmischer gemischt. Die Mischung wurde auf einer Ekzentertablettenpresse (Modell EK 0 Fa. Korsch) zu Tabletten gepresst, die Masse der Tabletten beträgt 500 mg. Es wurden runde Tabletten mit einem Durchmesser von 10 mm und einem Wölbungsradius von 8 mm hergestellt. Das Tablettierwerkeug bestehend aus Matrize, Ober- und Unterstempel und mit einem Durchmesser 10 mm und einem Wölbungsradius 8 mm wurden in einem Heizschrank auf 130°C erhitzt. Mittels heißem Werkzeug wurden die zuvor hergestellten Tabletten erneut verpresst, wobei der Pressdruck für mindestens 15s aufrechterhalten wurde.

Die Bruchfestigkeit der Tabletten wurde nach der Methode mit der angegebenen Apparatur bestimmt. Bei der Krafteinwirkung von 500 N trat kein Bruch auf. Die Tablette konnte mit einem Hammer nicht zerkleinert werden, dies war auch nicht mit Hilfe von Mörser und Pistill möglich.

### Beispiel 15:

Analog zu Beispiel 14 wurden Tabletten folgender Zusammensetzung hergestellt:

| Rohstoff | Pro Tablette | Pro Ansatz | Anteilig |
|---|---|---|---|
| Tramadol HCl | 100 mg | 10 g | 20% |
| Polyethylenoxid 5 000 000 (Polyox WSR Coaqulant Dow Chemicals) | 375 mg | 37,5 g | 75% |
| Carnaubawachs | 25 mg | 2,5 g | 5,0% |

Die Bruchfestigkeit der Tabletten wurde nach der Methode mit der angegebenen Apparatur bestimmt. Bei der Krafteinwirkung von 500 N trat kein Bruch auf. Die Tablette konnte mit einem Hammer nicht zerkleinert werden, dies war auch nicht mit Hilfe von Mörser und Pistill möglich.

### Beispiel 16:

Es wurden Tabletten folgender Zusammensetzung hergestellt:

| Rohstoff | Pro Tablette | Pro Ansatz | Anteilig |
|---|---|---|---|
| Tramadol HCl | 100,0 mg | 1490 g | 29,8% |
| Polyethylenoxid 7 000 000 (Polyox WSR 303, Dow Chemicals) | 151,0 mg | 2250 g | 45,0% |
| Hypromellose (Metholose 90 SH 100 000 cP von ShinEtsu) | 33,6 mg | 500 g | 10,0% |
| Eudragit E Granulat (Fa. Röhm) | 16,8 mg | 250 g | 5,0% |
| PEG 6000 | 33,6 mg | 500 g | 10,0% |
| Alfa Tocopherol | 0,1 mg | 5 g | 0,1% |
| Aerosil (hochdisperses Siliciumdioxid) | 0,1 mg | 5 g | 0,1% |

50 g des Polyethylenoxids wurden mit 5 g alfa-Tocopherol und Aerosil in einem Mörser zu einer homogenen Mischung verarbeitet. Diese wurde mit den weiteren Komponenten in einem Freifallmischer für 15 Minuten gemischt. Anschließend wurden sie mit dem Planetwalzenextruder vom Typ BCG 10 der Fa. LBB Bohle (Ennigerloh) extrudiert. Es wurden 4 Planetspindeln eingesetzt. Der Durchmesser der Düse betrug 8 mm. Die Pulverdosierung erfolgte gravimetrisch, 10 kg pro Stunde. Für die Extrusion wurden folgende Herstellparameter gewählt: Drehzahl: 50 UpM, Manteltemperatur: 100 °C, die Temperatur der Zentralspindel 100°C; Temperatur der Düsenheizung 120°C. Nach der Herstellung kühlten die Extrudate auf Raumtemperatur ab. Anschließend wurden sie in Scheiben geschnitten, die das Gewicht der Tablette hatten. Die Umformung der Tabletten erfolgte mit einer Ekzenterpresse der Firma Korsch vom Typ EKO. Als Tablettierwerkzeug dienten runde Stempel (Durchmesser 10 mm) mit einem Wölbungsradius von 8 mm.

Die Bruchfestigkeit der Tabletten wurde nach der Methode mit der angegebenen Apparatur bestimmt. Bei der Krafteinwirkung von 500 N trat kein Bruch auf. Die Tablette konnte mit einem Hammer nicht zerkleinert werden, dies war auch nicht mit Hilfe von Mörser und Pistill möglich.

Die in vitro Freisetzung des Wirkstoffs aus der Zubereitung wurde in der Blattrührerapparatur mit Sinker nach Pharm Eur. bestimmt. Die Temperatur des Freisetzungsmediums betrug 37°C und die Umdrehungsgeschwindigkeit des Rührers 75min⁻¹. Als Freisetzungsmedium wurden 600 ml Darmsaft pH 6,8 verwendet. Die jeweils zu einem Zeitpunkt im Lösungsmedium freigesetzte Menge an Wirkstoff wurde spektralphotometrisch bestimmt.

| Zeit | Freigesetzte Wirkstoffmenge |
|---|---|
| 30 min | 17 % |
| 240 min | 65 % |
| 480 min | 93 % |
| 720 min | 99 % |

### Beispiel 17:

Analog zu Beispiel 16 wurden Tabletten folgender Zusammensetzung hergestellt:

| Rohstoff | Pro Tablette | Pro Ansatz | Anteilig |
|---|---|---|---|
| Tramadol HCl | 100,0 mg | 1490 g | 29,8% |
| Polyethylenoxid 7 000 000 Polyox WSR 303, Dow Chemicals) | 151,0 mg | 2250 g | 45,0% |
| Hypromellose (Metholose 90 SH 100 000 cP von ShinEtsu) | 33,6 mg | 500 g | 10,0% |
| Stamylan 1965 (SABIC® LDPE 1965T Polyethylen mit niedriger Dichte) | 16,8 mg | 250 g | 5,0% |
| PEG 6000 | 33,6 mg | 500 g | 10,0% |
| Alfa Tocopherol | 0,1 mg | 5 g | 0,1% |
| Aerosil (hochdisperses Siliciumdioxid) | 0,1 mg | 5 g | 0,1% |

Die Bruchfestigkeit der Tabletten wurde nach der Methode mit der angegebenen Apparatur bestimmt. Bei der Krafteinwirkung von 500 N trat kein Bruch auf. Die Tablette konnte mit einem Hammer nicht zerkleinert werden, dies war auch nicht mit Hilfe von Mörser und Pistill möglich.

Die in vitro Freisetzung des Wirkstoffs aus der Zubereitung wurde in der Blattrührerapparatur mit Sinker nach Pharm Eur. bestimmt. Die Temperatur des Freisetzungsmediums betrug 37°C und die Umdrehungsgeschwindigkeit des Rührers 75min⁻¹. Als Freisetzungsmedium wurden 600 ml Darmsaft pH 6,8 verwendet. Die jeweils zu einem Zeitpunkt im Lösungsmedium freigesetzte Menge an Wirkstoff wurde spektralphotometrisch bestimmt.

| Zeit | Freigesetzte Wirkstoffmenge |
|---|---|
| 30 min | 17 % |
| 240 min | 62 % |
| 480 min | 85 % |
| 720 min | 94 % |

## Patentansprüche

1. Darreichungsform umfassend
- eine physiologisch wirksame Substanz (A),
- ggf. einen oder mehrere physiologisch verträgliche Hilfsstoffe (B);
- ein synthetisches Polymer (C); und
- ggf. ein natürliches, halbsynthetisches oder synthetisches Wachs (D);
wobei die Substanz (A) in einer Retardmatrix vorliegt; wobei die Retardmatrix das Polymer (C) und/oder das gegebenenfalls vorhandene Wachs (D) als Retardmatrixmaterial umfasst;
wobei das Polymer (C) ein Polyalkylenoxid ist ausgewählt aus der Gruppe bestehend aus Polymethylenoxid, Polyethylenoxid, Polypropylenoxid, deren Copolymerisaten, deren Blockcopolymerisaten und deren Mischungen, wobei Polymer (C) ein viskositätsmittleres Molekulargewicht von mindestens 0,5 10⁶ g/mol aufweist; und wobei die Darreichungsform
- eine Bruchfestigkeit von mindestens 400 N aufweist, und
- unter physiologischen Bedingungen nach 5 Stunden höchstens 99% der Substanz (A) freisetzt;
mit der Maßgabe, dass die Darreichungsform
(1) keine Tablette mit einer Bruchfestigkeit von mindestens 500 N ist, welche ein Gesamtgewicht von 300,0 mg, einen Durchmesser von 10 mm und einen Wölbungsradius von 8 mm aufweist; durch Verpressen eines auf 80°C erwärmten Gemisches bestehend aus 100 mg Tramadolhydrochlorid und 200 mg Polyethylenoxid mit einem Molekulargewicht von 7 000 000 pro Tablette mittels eines Tablettierwerkzeugs mit Oberstempel, Unterstempel und Matrize unter Aufrechterhaltung des Pressdrucks für mindestens 15 Sekunden erhältlich ist; bei einer *in-vitro*-Freisetzungsbestimmung gemäß *Ph. Eur.* in einer Blattrührerapparatur mit dem Freisetzungsmedium bei einer Umdrehungsgeschwindigkeit von 75 U. pro Minute, nach 30 min. 15%, nach 240 min. 52%, nach 480 min. 80% und nach 720 min. 99% der Substanz (A) freisetzt; und wobei zu Beginn der Untersuchung die Tablette in 600 ml künstlichen Magensaft mit einem pH-Wert von 1,2 gegeben wird, welcher durch Zugabe von Lauge nach 30 min. auf 2,3, nach weiteren 90 min. auf 6,5 und nach nochmals 60 weiteren min. auf 7,2 erhöht wird;
(2) keine Tablette mit einer Bruchfestigkeit von mindestens 500 N ist, welche ein Gesamtgewicht von 300,0 mg, einen Durchmesser von 10 mm und einen Wölbungsradius von 8 mm aufweist; durch Verpressen eines auf 80°C erwärmten Gemisches bestehend aus 100 mg Tramadolhydrochlorid und 200 mg Polyethylenoxid mit einem Molekulargewicht von 7 000 000 pro Tablette mittels eines Tablettierwerkzeugs mit Oberstempel, Unterstempel und Matrize unter Aufrechterhaltung des Pressdrucks für mindestens 15 Sekunden erhältlich ist, wobei das Gemisch in die Matrize des Tablettierwerkzeugs eingefüllt wird; bei einer *in-vitro*-Freisetzungsbestimmung gemäß *Ph. Eur.* in einer Blattrührerapparatur mit dem Freisetzungsmedium bei einer Umdrehungsgeschwindigkeit von 75 U. pro Minute, nach 30 min. 15%, nach 240 min. 52%, nach 480 min. 80% und nach 720 min. 99% der Substanz (A) freisetzt; und wobei zu Beginn der Untersuchung die Tablette in 600 ml künstlichen Magensaft mit einem pH-Wert von 1,2 gegeben wird, welcher durch Zugabe von Lauge nach 30 min. auf 2,3, nach weiteren 90 min. auf 6,5 und nach nochmals 60 weiteren min. auf 7,2 erhöht wird;
(3) keine Tablette mit einer Bruchfestigkeit von mindestens 500 N ist, welche ein Gesamtgewicht von 150,0 mg und einen Durchmesser von 7 mm aufweist; durch Verpressen eines auf 80°C erwärmten Gemisches bestehend aus 50 mg Tramadolhydrochlorid und 100 mg Polyethylenoxid mit einem Molekulargewicht von 7 000 000 pro Tablette mittels eines Tablettierwerkzeugs mit Oberstempel, Unterstempel und Matrize unter Aufrechterhaltung des Pressdrucks für mindestens 15 Sekunden erhältlich ist; bei einer *in-vitro*-Freisetzungsbestimmung gemäß *Ph. Eur.* in einer Blattrührerapparatur mit dem Freisetzungsmedium bei einer Umdrehungsgeschwindigkeit von 75 U. pro Minute, nach 30 min. 15%, nach 240 min. 62%, nach 480 min. 88% und nach 720 min. 99% der Substanz (A) freisetzt; und wobei zu Beginn der Untersuchung die Tablette in 600 ml künstlichen Magensaft mit einem pH-Wert von 1,2 gegeben wird, welcher durch Zugabe von Lauge nach 30 min. auf 2,3, nach weiteren 90 min. auf 6,5 und nach nochmals 60 weiteren min. auf 7,2 erhöht wird;
(4) keine Tablette mit einer Bruchfestigkeit von mindestens 500 N ist, welche ein Gesamtgewicht von 300,0 mg, einen Durchmesser von 10 mm und einen Wölbungsradius von 8 mm aufweist; durch Verpressen eines auf 80°C erwärmten Gemisches bestehend aus 100 mg Tramadolhydrochlorid, 20 mg Xanthan und 180 mg Polyethylenoxid mit einem Molekulargewicht von 7 000 000 pro Tablette mittels eines Tablettierwerkzeugs mit Oberstempel, Unterstempel und Matrize unter Aufrechterhaltung des Pressdrucks für mindestens 15 Sekunden erhältlich ist; bei einer *in-vitro*-Freisetzungsbestimmung gemäß *Ph. Eur.* in einer Blattrührerapparatur mit dem Freisetzungsmedium bei einer Umdrehungsgeschwindigkeit von 75 U. pro Minute, nach 30 min. 14%, nach 240 min. 54%, nach 480 min. 81% und nach 720 min. 99% der Substanz (A) freisetzt; und wobei zu Beginn der Untersuchung die Tablette in 600 ml künstlichen Magensaft mit einem pH-Wert von 1,2 gegeben wird, welcher durch Zugabe von Lauge nach 30 min. auf 2,3, nach weiteren 90 min. auf 6,5 und nach nochmals 60 weiteren min. auf 7,2 erhöht wird;
(5) keine Oblongtablette mit einer Bruchfestigkeit von mindestens 500 N ist, welche ein Gesamtgewicht von 150,0 mg, eine Länge von 10 mm und eine Breite von 5 mm aufweist; durch Verpressen eines auf 90°C erwärmten Gemisches bestehend aus 50 mg Tramadolhydrochlorid, 10 mg Xanthan und 90 mg Polyethylenoxid mit einem Molekulargewicht von 7 000 000 pro Tablette mittels eines Tablettierwerkzeugs mit Oberstempel, Unterstempel und Matrize unter Aufrechterhaltung des Pressdrucks für mindestens 15 Sekunden erhältlich ist; bei einer *in-vitro*-Freisetzungsbestimmung gemäß *Ph. Eur.* in einer Blattrührerapparatur mit dem Freisetzungsmedium bei einer Umdrehungsgeschwindigkeit von 75 U. pro Minute, nach 30 min. 22%, nach 120 min. 50%, nach 240 min. 80%, nach 360 min. 90% und nach 480 min. 99% der Substanz (A) freisetzt; und wobei zu Beginn der Untersuchung die Tablette in 600 ml künstlichen Magensaft mit einem pH-Wert von 1,2 gegeben wird, welcher durch Zugabe von Lauge nach 30 min. auf 2,3, nach weiteren 90 min. auf 6,5 und nach nochmals 60 weiteren min. auf 7,2 erhöht wird;
(6) keine Tablette mit einer Bruchfestigkeit von mindestens 500 N ist, welche ein Gesamtgewicht von 150,0 mg, einen Durchmesser von 10 mm und einen Wölbungsradius von 8 mm aufweist; durch Verpressen eines auf 80°C erwärmten Gemisches bestehend aus 20 mg Oxycodon Hydrochlorid, 20,0 mg Xanthan und 110 mg Polyethylenoxid mit einem Molekulargewicht von 7 000 000 pro Tablette mittels eines Tablettierwerkzeugs mit Oberstempel, Unterstempel und Matrize unter Aufrechterhaltung des Pressdrucks für mindestens 15 Sekunden erhältlich ist; und bei einer *in-vitro*-Freisetzungsbestimmung gemäß *Ph. Eur.* in einer Blattrührerapparatur mit dem in der USP beschriebenen Phosphat-gepuffertem Freisetzungsmedium mit pH 6,8 bei 37 °C und einer Umdrehungsgeschwindigkeit von 75 U. pro Minute, nach 30 min. 17%, nach 240 min. 61%, nach 480 min. 90% und nach 720 min. 101,1% der Substanz (A) freisetzt;
(7) keine Tablette mit einer Bruchfestigkeit von mindestens 500 N ist, welche ein Gesamtgewicht von 341,2 mg, einen Durchmesser von 10 mm und einen Wölbungsradius von 8 mm aufweist; durch Lösen von 0,2 mg Butylhydroxytoluol in 96%-igem Ethanol, Trocknung der erhaltenen 7,7%-igen ethanolischen Lösung bei 40°C für 12 h in Gegenwart von 221 mg Polyethylenoxid mit einem Molekulargewicht von 7 000 000, und durch anschließendes Verpressen zusammen mit einem auf 80°C erwärmten Gemisch bestehend aus 100 mg Tramadolhydrochlorid und 20 mg Hydroxypropylmethylcellulose pro Tablette mittels eines Tablettierwerkzeugs mit Oberstempel, Unterstempel und Matrize unter Aufrechterhaltung des Pressdrucks für mindestens 15 Sekunden erhältlich ist; bei einer *in-vitro*-Freisetzungsbestimmung gemäß *Ph. Eur.* in einer Blattrührerapparatur mit dem Freisetzungsmedium bei einer Umdrehungsgeschwindigkeit von 75 U. pro Minute, nach 30 min. 17%, nach 240 min. 59%, nach 480 min. 86% und nach 720 min. 98% der Substanz (A) freisetzt; und wobei zu Beginn der Untersuchung die Tablette in 600 ml künstlichen Magensaft mit einem pH-Wert von 1,2 gegeben wird, welcher durch Zugabe von Lauge nach 30 min. auf 2,3, nach weiteren 90 min. auf 6,5 und nach nochmals 60 weiteren min. auf 7,2 erhöht wird;
(8) keine Tablette mit einer Bruchfestigkeit von mindestens 500 N ist, welche ein Gesamtgewicht von 341,0 mg, einen Durchmesser von 10 mm und einen Wölbungsradius von 8 mm aufweist; durch Verpressen eines auf 80°C erwärmten Gemisches bestehend aus 100 mg Tramadolhydrochlorid, 221 mg Polyethylenoxid mit einem Molekulargewicht von 7 000 000 und 20 mg Hydroxypropylmethylcellulose pro Tablette mittels eines Tablettierwerkzeugs mit Oberstempel, Unterstempel und Matrize unter Aufrechterhaltung des Pressdrucks für mindestens 15 Sekunden erhältlich ist; bei einer *in-vitro*-Freisetzungsbestimmung gemäß *Ph. Eur.* in einer Blattrührerapparatur mit dem Freisetzungsmedium bei einer Umdrehungsgeschwindigkeit von 75 U. pro Minute, nach 30 min. 16%, nach 240 min. 57%, nach 480 min. 84% und nach 720 min. 96% der Substanz (A) freisetzt; und wobei zu Beginn der Untersuchung die Tablette in 600 ml künstlichen Magensaft mit einem pH-Wert von 1,2 gegeben wird, welcher durch Zugabe von Lauge nach 30 min. auf 2,3, nach weiteren 90 min. auf 6,5 und nach nochmals 60 weiteren min. auf 7,2 erhöht wird;
(9) keine biplane Tablette mit einer Bruchfestigkeit von mindestens 500 N ist, welche ein Gesamtgewicht von 335,0 mg aufweist; durch Lösen von 0,2 mg Butylhydroxytoluol in 96%-igem Ethanol, Mischen der erhaltenen 7,7%-igen ethanolischen Lösung mit 150 mg Polyethylenoxid mit einem Molekulargewicht von 7 000 000 in einem Schnellmischer für 30 min., anschließendes Zugeben von 17,8 mg Polyethylenoxid mit einem Molekulargewicht von 7 000 000 und Rühren für 30 min., und anschließendes Trocknen bei 40°C für 12h; Zugabe von 100 mg Tramadolhydrochlorid, 33,5 mg Hydroxypropylmethylcellulose und 33,5 mg Polyethylenglycol; und Extrusion der Mischung in einem Doppelschneckenextruder unter Stickstoffatmosphäre bei einer Schneckendrehzahl von 100 U. pro min., einem Durchsatz von 4 kg/h, einer Produkttemperatur von 125°C und einer Manteltemperatur von 120°C erhältlich ist;
(10) keine Tablette mit einer Bruchfestigkeit von mindestens 500 N ist, welche ein Gesamtgewicht von 586,0 mg und einen Durchmesser von 12 mm aufweist; durch Verpressen eines Gemisches bestehend aus 205 mg Tramadolhydrochlorid und 381 mg Polyethylenoxid mit einem Molekulargewicht von 7 000 000 pro Tablette mittels gleichzeitiger Einwirkung einer Kraft von 250 N und einem Ultraschall mit einer Frequenz von 20 Hz und einer Amplitudenstärke von 50%, für 0,5 Sekunden, erhältlich ist; bei einer *in-vitro*-Freisetzungsbestimmung gemäß *Ph. Eur.* in einer Blattrührerapparatur mit Sinker bei einer Umdrehungsgeschwindigkeit von 75 U. pro Minute, nach 30 min. 13%, nach 240 min. 51%, nach 480 min. 76% und nach 720 min. 100% der Substanz (A) freisetzt; und wobei das Freisetzungsmedium 600 ml Darmsaft mit einer Temperatur von 37°C und einem pH-Wert von 6,8 ist; und
(11) keine Tablette mit einer Bruchfestigkeit von mindestens 500 N ist, welche ein Gesamtgewicht von 300,0 mg und einen Durchmesser von 10 mm und einen Wölbungsradius von 8 mm aufweist; durch Verpressen eines Gemisches bestehend aus 100 mg Tramadolhydrochlorid und 200 mg Polyethylenoxid mit einem Molekulargewicht von 7 000 000 pro Tablette mittels Einwirkung
- einer Kraft von 970 N und einem Ultraschall mit einer Frequenz von 20 Hz und einer Amplitudenstärke von 75%, gleichzeitig für 0,15 Sekunden, anschließend
- einer Kraft von 970 N und einem Ultraschall mit einer Frequenz von 20 Hz und einer Amplitudenstärke von 32,5%, gleichzeitig für 0,55 Sekunden, und anschließend
- einer Kraft von 970 N für 2,3 Sekunden,
erhältlich ist; bei einer *in-vitro*-Freisetzungsbestimmung gemäß *Ph. Eur.* in einer Blattrührerapparatur mit Sinker bei einer Umdrehungsgeschwindigkeit von 75 U. pro Minute, nach 30 min. 17,1%, nach 240 min. 60,6%, nach 480 min. 84% und nach 720 min. 94,2% der Substanz (A) freisetzt; und wobei das Freisetzungsmedium 600 ml Darmsaft mit einer Temperatur von 37°C und einem pH-Wert von 6,8 ist.

2. Darreichungsform nach Anspruch 1, **dadurch gekennzeichnet, dass** sie keine psychotrop wirkende Substanz enthält.

3. Darreichungsform nach Anspruch 1 oder 2, **dadurch gekennzeichnet, dass** sie eine Bruchfestigkeit von mindestens 500 N aufweist.

4. Darreichungsform nach einem der vorstehenden Ansprüche, **dadurch gekennzeichnet, dass** sie in Form einer Tablette vorliegt.

5. Darreichungsform nach einem der vorstehenden Ansprüche, **dadurch gekennzeichnet, dass** sie weder Tramadol Hydrochlorid noch Oxycodon Hydrochlorid enthält.

6. Darreichungsform nach einem der vorstehenden Ansprüche, **dadurch gekennzeichnet, dass** die Substanz (A) ein Arzneistoff ist, ausgewählt aus der Gruppe bestehend aus Mitteln zur Behandlung und Vorbeugung von Erkrankungen des alimentären Systems und Stoffwechsels; Mitteln zur Behandlung und Vorbeugung von Erkrankungen des Bluts und der blutbildenden Organe; Mitteln zur Behandlung und Vorbeugung von Erkrankungen des kardiovaskulären Systems; Dermatika; Mitteln zur Behandlung und Vorbeugung von Erkrankungen des Urogenitalsystems und Sexualhormonen; systemischen Hormonpräparaten ausschließlich Sexualhormonen und Insulinen; Antiinfektiva zur systemischen Anwendung; antineoplastischen und immunmodulierenden Mitteln; Mitteln zur Behandlung und Vorbeugung von Erkrankungen der Muskeln und des Skelettsystems; Mitteln zur Behandlung und Vorbeugung von Erkrankungen des Nervensystems; antiparasitären Mitteln, Insektiziden und Repellenzien; Mitteln zur Behandlung und Vorbeugung von Erkrankungen des Respirationstrakts; Mitteln zur Behandlung und Vorbeugung von Erkrankungen der Sinnesorgane; Allgemeinen Diätetika und Radiotherapeutika.

7. Verfahren zur Herstellung einer Darreichungsform nach einem der Ansprüche 1 bis 6, umfassend die folgenden Schritte:
(a) Mischen der Komponente (A), ggf. (B), (C), ggf. (D);
(b) ggf. Vorformen der aus Schritt (a) erhaltenen Mischung, vorzugsweise unter Wärme- und/oder Krafteinwirkung auf die aus (a) erhaltene Mischung, wobei die zugeführte Wärmemenge bevorzugt nicht dazu ausreicht, die Komponente (C) bis zu ihrem Erweichungspunkt zu erwärmen;
(c) Härten der Mischung unter Wärme- und Krafteinwirkung, wobei die Wärmezufuhr während und/oder vor der Krafteinwirkung erfolgen kann und die zugeführte Wärmemenge dazu ausreicht, die Komponente (C) wenigstens bis zu ihrem Erweichungspunkt zu erwärmen;
(d) ggf. Vereinzeln der gehärteten Mischung;
(e) ggf. Formen der Darreichungsform; und
(f) ggf. Beschichten mit einem Filmüberzug.

8. Verfahren nach Anspruch 7, **dadurch gekennzeichnet, dass** in Schritt (c) ein Doppelschneckenextruder oder ein Planetwalzenextruder eingesetzt wird.

## Claims

1. Pharmaceutical form comprising
- a physiologically effective substance (A),
- optionally one or more physiologically tolerable excipients (B);
- a synthetic polymer (C); and
- optionally a natural, semisynthetic or synthetic wax (D);
wherein the substance (A) is present in a modified-release matrix; wherein the modified-release matrix comprises the polymer (C) and/or the optionally present wax (D) as modified-release matrix material;
wherein the polymer (C) is a polyalkylene oxide selected from the group consisting of polymethylene oxide, polyethylene oxide, polypropylene oxide, the copolymers thereof, the block copolymers thereof and the mixtures thereof, wherein polymer (C) has a viscosity-average molecular weight of at least 0.5 10⁶ g/mol; and wherein the pharmaceutical form
- has a breaking strength of at least 400 N, and
- releases, under physiological conditions, at most 99% of the substance (A) after 5 hours;
with the proviso that the pharmaceutical form
(1) is not a tablet having a breaking strength of at least 500 N, which tablet has a total weight of 300.0 mg, a diameter of 10 mm and a radius of curvature of 8 mm; is obtainable by compressing a mixture heated to 80°C and consisting of 100 mg of tramadol hydrochloride and 200 mg of polyethylene oxide having a molecular weight of 7 000 000 per tablet by means of a tabletting tool with upper punch, lower punch and die with maintenance of the applied pressure for at least 15 seconds; releases 15%, 52%, 80% and 99% of the substance (A) after 30 min, 240 min, 480 min and 720 min, respectively, in an *in vitro* determination of release as per *Ph. Eur.* in a paddle-stirrer apparatus containing the release medium at a rotational speed of 75 rpm; and wherein at the start of the test the tablet is added to 600 ml of artificial gastric juice of pH 1.2, which pH is increased by addition of alkaline solution to 2.3 after 30 min, to 6.5 after a further 90 min and to 7.2 after again 60 further min;
(2) is not a tablet having a breaking strength of at least 500 N, which tablet has a total weight of 300.0 mg, a diameter of 10 mm and a radius of curvature of 8 mm; is obtainable by compressing a mixture heated to 80°C and consisting of 100 mg of tramadol hydrochloride and 200 mg of polyethylene oxide having a molecular weight of 7 000 000 per tablet by means of a tabletting tool with upper punch, lower punch and die with maintenance of the applied pressure for at least 15 seconds, wherein the mixture is filled into the die of the tabletting tool; releases 15%, 52%, 80% and 99% of the substance (A) after 30 min, 240 min, 480 min and 720 min, respectively, in an *in vitro* determination of release as per *Ph. Eur.* in a paddle-stirrer apparatus containing the release medium at a rotational speed of 75 rpm; and wherein at the start of the test the tablet is added to 600 ml of artificial gastric juice of pH 1.2, which pH is increased by addition of alkaline solution to 2.3 after 30 min, to 6.5 after a further 90 min and to 7.2 after again 60 further min;
(3) is not a tablet having a breaking strength of at least 500 N, which tablet has a total weight of 150.0 mg and a diameter of 7 mm; is obtainable by compressing a mixture heated to 80°C and consisting of 50 mg of tramadol hydrochloride and 100 mg of polyethylene oxide having a molecular weight of 7 000 000 per tablet by means of a tabletting tool with upper punch, lower punch and die with maintenance of the applied pressure for at least 15 seconds; releases 15%, 62%, 88% and 99% of the substance (A) after 30 min, 240 min, 480 min and 720 min, respectively, in an *in vitro* determination of release as per *Ph. Eur.* in a paddle-stirrer apparatus containing the release medium at a rotational speed of 75 rpm; and wherein at the start of the test the tablet is added to 600 ml of artificial gastric juice of pH 1.2, which pH is increased by addition of alkaline solution to 2.3 after 30 min, to 6.5 after a further 90 min and to 7.2 after again 60 further min;
(4) is not a tablet having a breaking strength of at least 500 N, which tablet has a total weight of 300.0 mg, a diameter of 10 mm and a radius of curvature of 8 mm; is obtainable by compressing a mixture heated to 80°C and consisting of 100 mg of tramadol hydrochloride, 20 mg of xanthan gum and 180 mg of polyethylene oxide having a molecular weight of 7 000 000 per tablet by means of a tabletting tool with upper punch, lower punch and die with maintenance of the applied pressure for at least 15 seconds; releases 14%, 54%, 81% and 99% of the substance (A) after 30 min, 240 min, 480 min and 720 min, respectively, in an *in vitro* determination of release as per *Ph. Eur.* in a paddle-stirrer apparatus containing the release medium at a rotational speed of 75 rpm; and wherein at the start of the test the tablet is added to 600 ml of artificial gastric juice of pH 1.2, which pH is increased by addition of alkaline solution to 2.3 after 30 min, to 6.5 after a further 90 min and to 7.2 after again 60 further min;
(5) is not an oblong tablet having a breaking strength of at least 500 N, which tablet has a total weight of 150.0 mg, a length of 10 mm and a width of 5 mm; is obtainable by compressing a mixture heated to 90°C and consisting of 50 mg of tramadol hydrochloride, 10 mg of xanthan gum and 90 mg of polyethylene oxide having a molecular weight of 7 000 000 per tablet by means of a tabletting tool with upper punch, lower punch and die with maintenance of the applied pressure for at least 15 seconds; releases 22%, 50%, 80%, 90% and 99% of the substance (A) after 30 min, 120 min, 240 min, 360 min and 480 min, respectively, in an *in vitro* determination of release as per *Ph. Eur.* in a paddle-stirrer apparatus containing the release medium at a rotational speed of 75 rpm; and wherein at the start of the test the tablet is added to 600 ml of artificial gastric juice of pH 1.2, which pH is increased by addition of alkaline solution to 2.3 after 30 min, to 6.5 after a further 90 min and to 7.2 after again 60 further min;
(6) is not a tablet having a breaking strength of at least 500 N, which tablet has a total weight of 150.0 mg, a diameter of 10 mm and a radius of curvature of 8 mm; is obtainable by compressing a mixture heated to 80°C and consisting of 20 mg of oxycodone hydrochloride, 20.0 mg of xanthan gum and 110 mg of polyethylene oxide having a molecular weight of 7 000 000 per tablet by means of a tabletting tool with upper punch, lower punch and die with maintenance of the applied pressure for at least 15 seconds; and releases 17%, 61%, 90% and 101.1% of the substance (A) after 30 min, 240 min, 480 min and 720 min, respectively, in an *in vitro* determination of release as per *Ph. Eur.* in a paddle-stirrer apparatus containing the phosphate-buffered release medium of pH 6.8 at 37°C, as described in the USP, and a rotational speed of 75 rpm;
(7) is not a tablet having a breaking strength of at least 500 N, which tablet has a total weight of 341.2 mg, a diameter of 10 mm and a radius of curvature of 8 mm; is obtainable by dissolving 0.2 mg of butylhydroxytoluene in 96% ethanol, drying the 7.7% ethanolic solution obtained at 40°C for 12 h in the presence of 221 mg of polyethylene oxide having a molecular weight of 7 000 000 and by subsequently compressing them together with a mixture heated to 80°C and consisting of 100 mg of tramadol hydrochloride and 20 mg of hydroxypropylmethylcellulose per tablet by means of a tabletting tool with upper punch, lower punch and die with maintenance of the applied pressure for at least 15 seconds; releases 17%, 59%, 86% and 98% of the substance (A) after 30 min, 240 min, 480 min and 720 min, respectively, in an *in vitro* determination of release as per *Ph. Eur.* in a paddle-stirrer apparatus containing the release medium at a rotational speed of 75 rpm; and wherein at the start of the test the tablet is added to 600 ml of artificial gastric juice of pH 1.2, which pH is increased by addition of alkaline solution to 2.3 after 30 min, to 6.5 after a further 90 min and to 7.2 after again 60 further min;
(8) is not a tablet having a breaking strength of at least 500 N, which tablet has a total weight of 341.0 mg, a diameter of 10 mm and a radius of curvature of 8 mm; is obtainable by compressing a mixture heated to 80°C and consisting of 100 mg of tramadol hydrochloride, 221 mg of polyethylene oxide having a molecular weight of 7 000 000 and 20 mg of hydroxypropylmethylcellulose per tablet by means of a tabletting tool with upper punch, lower punch and die with maintenance of the applied pressure for at least 15 seconds; releases 16%, 57%, 84% and 96% of the substance (A) after 30 min, 240 min, 480 min and 720 min, respectively, in an *in vitro* determination of release as per *Ph. Eur.* in a paddle-stirrer apparatus containing the release medium at a rotational speed of 75 rpm; and wherein at the start of the test the tablet is added to 600 ml of artificial gastric juice of pH 1.2, which pH is increased by addition of alkaline solution to 2.3 after 30 min, to 6.5 after a further 90 min and to 7.2 after again 60 further min;
(9) is not a biplanar tablet having a breaking strength of at least 500 N, which tablet has a total weight of 335.0 mg; is obtainable by dissolving 0.2 mg of butylhydroxytoluene in 96% ethanol, mixing the 7.7% ethanolic solution obtained with 150 mg of polyethylene oxide having a molecular weight of 7 000 000 in a high-speed mixer for 30 min, subsequently adding 17.8 mg of polyethylene oxide having a molecular weight of 7 000 000 and stirring for 30 min, and subsequently drying at 40°C for 12 h; adding 100 mg of tramadol hydrochloride, 33.5 mg of hydroxypropylmethylcellulose and 33.5 mg of polyethylene glycol; and extruding the mixture in a twin-screw extruder under a nitrogen atmosphere at a screw speed of 100 rpm, a throughput of 4 kg/h, a product temperature of 125°C and a jacket temperature of 120°C;
(10) is not a tablet having a breaking strength of at least 500 N, which tablet has a total weight of 586.0 mg and a diameter of 12 mm; is obtainable by compressing a mixture consisting of 205 mg of tramadol hydrochloride and 381 mg of polyethylene oxide having a molecular weight of 7 000 000 per tablet by means of simultaneous action of a force of 250 N and an ultrasound treatment with a frequency of 20 Hz and an amplitude strength of 50% for 0.5 seconds; releases 13%, 51%, 76% and 100% of the substance (A) after 30 min, 240 min, 480 min and 720 min, respectively, in an *in vitro* determination of release as per *Ph. Eur.* in a paddle-stirrer apparatus with sinker at a rotational speed of 75 rpm; and wherein the release medium is 600 ml of gastric juice having a temperature of 37°C and a pH of 6.8; and
(11) is not a tablet having a breaking strength of at least 500 N, which tablet has a total weight of 300.0 mg and a diameter of 10 mm and a radius of curvature of 8 mm; is obtainable by compressing a mixture consisting of 100 mg of tramadol hydrochloride and 200 mg of polyethylene oxide having a molecular weight of 7 000 000 per tablet by means of action of
- a force of 970 N and an ultrasound treatment with a frequency of 20 Hz and an amplitude strength of 75%, simultaneously for 0.15 seconds, then
- a force of 970 N and an ultrasound treatment with a frequency of 20 Hz and an amplitude strength of 32.5%, simultaneously for 0.55 seconds, and then
- a force of 970 N for 2.3 seconds;
releases 17.1%, 60.6%, 84% and 94.2% of the substance (A) after 30 min, 240 min, 480 min and 720 min, respectively, in an *in vitro* determination of release as per *Ph. Eur.* in a paddle-stirrer apparatus with sinker at a rotational speed of 75 rpm; and wherein the release medium is 600 ml of gastric juice having a temperature of 37°C and a pH of 6.8.

2. Pharmaceutical form according to Claim 1, **characterized in that** it does not contain a psychotropically acting substance.

3. Pharmaceutical form according to Claim 1 or 2, **characterized in that** it has a breaking strength of at least 500 N.

4. Pharmaceutical form according to any of the preceding claims, **characterized in that** it is in the form of a tablet.

5. Pharmaceutical form according to any of the preceding claims, **characterized in that** it contains neither tramadol hydrochloride nor oxycodone hydrochloride.

6. Pharmaceutical form according to any of the preceding claims, **characterized in that** the substance (A) is a drug substance selected from the group consisting of agents for the treatment and prevention of disorders of the alimentary system and metabolism; agents for the treatment and prevention of disorders of the blood and the haematopoietic organs; agents for the treatment and prevention of disorders of the cardiovascular system; dermatics; agents for the treatment and prevention of disorders of the urogenital system and sex hormones; systemic hormone preparations excluding sex hormones and insulins; antiinfectives for systemic application; antineoplastic and immunomodulatory agents; agents for the treatment and prevention of disorders of the muscles and the skeletal system; agents for the treatment and prevention of disorders of the nervous system; antiparasitic agents, insecticides and repellents; agents for the treatment and prevention of disorders of the respiratory tract; agents for the treatment and prevention of disorders of the sensory organs; general dietetic products and therapeutic radiopharmaceuticals.

7. Process for producing a pharmaceutical form according to any of Claims 1 to 6, comprising the following steps:
(a) mixing the components (A), optionally (B), (C), optionally (D);
(b) optionally preshaping the mixture obtained from step (a), preferably under the action of heat and/or force on the mixture obtained from (a), wherein the supplied quantity of heat is preferably insufficient to heat the component (C) up to its softening point;
(c) hardening the mixture under the action of heat and force, wherein the supply of heat can take place during and/or before the action of force and the supplied quantity of heat is sufficient to heat the component (C) at least up to its softening point;
(d) optionally singularizing the hardened mixture;
(e) optionally shaping the pharmaceutical form; and
(f) optionally coating it with a film coating.

8. Process according to Claim 7, **characterized in that**, in step (c), a twin-screw extruder or a planetary roller extruder is used.

## Revendications

1. Forme pharmaceutique, comprenant :
- une substance physiologiquement active (A),
- éventuellement un ou plusieurs adjuvants physiologiquement compatibles (B) ;
- un polymère synthétique (C) ; et
- éventuellement une cire naturelle, semi-synthétique ou synthétique (D) ;
la substance (A) se présentant dans une matrice de retard ; la matrice de retard comprenant le polymère (C) et/ou la cire (D) éventuellement présente en tant que matériau de matrice de retard ;
le polymère (C) étant un polyoxyde d'alkylène choisi dans le groupe constitué par le polyoxyde de méthylène, le polyoxyde d'éthylène, le polyoxyde de propylène, leurs copolymères, leurs copolymères séquencés et leurs mélanges, le polymère (C) présentant un poids moléculaire moyen en viscosité d'au moins 0,5 10⁶ g/mol ; et
la forme pharmaceutique
- présentant une résistance à la rupture d'au moins 400 N, et
- libérant au plus 99 % de la substance (A) en conditions physiologiques après 5 heures ;
à condition que la forme pharmaceutique
(1) ne soit pas un comprimé ayant une résistance à la rupture d'au moins 500 N, qui présente un poids total de 300,0 mg, un diamètre de 10 mm et un rayon de courbure de 8 mm ; pouvant être obtenu par compression d'un mélange porté à 80 °C constitué par 100 mg de chlorhydrate de tramadol et 200 mg de polyoxyde d'éthylène ayant un poids moléculaire de 7 000 000 par comprimé au moyen d'un outil de pastillage avec poinçon supérieur, poinçon inférieur et matrice avec maintien de la pression de compression pendant au moins 15 secondes ; libère lors d'une détermination de la libération *in vitro* selon *Ph. Eur.* dans un appareil à agitateur à pales avec le milieu de libération à une vitesse de rotation de 75 tours par minute après 30 minutes 15 %, après 240 minutes 52 %, après 480 minutes 80 % et après 720 minutes 99 % de la substance (A) ; le comprimé étant introduit au début de l'essai dans 600 ml de milieu gastrique artificiel d'un pH de 1,2, qui est augmenté par ajout d'une base après 30 minutes à 2,3, après 90 minutes supplémentaires à 6,5 et après encore 60 minutes supplémentaires à 7,2 ;
(2) ne soit pas un comprimé ayant une résistance à la rupture d'au moins 500 N, qui présente un poids total de 300,0 mg, un diamètre de 10 mm et un rayon de courbure de 8 mm ; pouvant être obtenu par compression d'un mélange porté à 80 °C constitué par 100 mg de chlorhydrate de tramadol et 200 mg de polyoxyde d'éthylène ayant un poids moléculaire de 7 000 000 par comprimé au moyen d'un outil de pastillage avec poinçon supérieur, poinçon inférieur et matrice avec maintien de la pression de compression pendant au moins 15 secondes, la matrice de l'outil de pastillage étant remplie avec le mélange ; libère lors d'une détermination de la libération *in vitro* selon *Ph. Eur.* dans un appareil à agitateur à pales avec le milieu de libération à une vitesse de rotation de 75 tours par minute après 30 minutes 15 %, après 240 minutes 52 %, après 480 minutes 80 % et après 720 minutes 99 % de la substance (A) ; le comprimé étant introduit au début de l'essai dans 600 ml de milieu gastrique artificiel d'un pH de 1,2, qui est augmenté par ajout d'une base après 30 minutes à 2,3, après 90 minutes supplémentaires à 6,5 et après encore 60 minutes supplémentaires à 7,2 ;
(3) ne soit pas un comprimé ayant une résistance à la rupture d'au moins 500 N, qui présente un poids total de 150,0 mg et un diamètre de 7 mm ; pouvant être obtenu par compression d'un mélange porté à 80 °C constitué par 50 mg de chlorhydrate de tramadol et 100 mg de polyoxyde d'éthylène ayant un poids moléculaire de 7 000 000 par comprimé au moyen d'un outil de pastillage avec poinçon supérieur, poinçon inférieur et matrice avec maintien de la pression de compression pendant au moins 15 secondes ; libère lors d'une détermination de la libération *in vitro* selon *Ph*. *Eur.* dans un appareil à agitateur à pales avec le milieu de libération à une vitesse de rotation de 75 tours par minute après 30 minutes 15 %, après 240 minutes 62 %, après 480 minutes 88 % et après 720 minutes 99 % de la substance (A) ; le comprimé étant introduit au début de l'essai dans 600 ml de milieu gastrique artificiel d'un pH de 1,2, qui est augmenté par ajout d'une base après 30 minutes à 2,3, après 90 minutes supplémentaires à 6,5 et après encore 60 minutes supplémentaires à 7,2 ;
(4) ne soit pas un comprimé ayant une résistance à la rupture d'au moins 500 N, qui présente un poids total de 300,0 mg, un diamètre de 10 mm et un rayon de courbure de 8 mm ; pouvant être obtenu par compression d'un mélange porté à 80 °C constitué par 100 mg de chlorhydrate de tramadol, 20 mg de xanthane et 180 mg de polyoxyde d'éthylène ayant un poids moléculaire de 7 000 000 par comprimé au moyen d'un outil de pastillage avec poinçon supérieur, poinçon inférieur et matrice avec maintien de la pression de compression pendant au moins 15 secondes ; libère lors d'une détermination de la libération *in vitro* selon *Ph. Eur.* dans un appareil à agitateur à pales avec le milieu de libération à une vitesse de rotation de 75 tours par minute après 30 minutes 14 %, après 240 minutes 54 %, après 480 minutes 81 % et après 720 minutes 99 % de la substance (A) ; le comprimé étant introduit au début de l'essai dans 600 ml de milieu gastrique artificiel d'un pH de 1,2, qui est augmenté par ajout d'une base après 30 minutes à 2,3, après 90 minutes supplémentaires à 6,5 et après encore 60 minutes supplémentaires à 7,2 ;
(5) ne soit pas un comprimé oblong ayant une résistance à la rupture d'au moins 500 N, qui présente un poids total de 150,0 mg, une longueur de 10 mm et une largeur de 5 mm ; pouvant être obtenu par compression d'un mélange porté à 90 °C constitué par 50 mg de chlorhydrate de tramadol, 10 mg de xanthane et 90 mg de polyoxyde d'éthylène ayant un poids moléculaire de 7 000 000 par comprimé au moyen d'un outil de pastillage avec poinçon supérieur, poinçon inférieur et matrice avec maintien de la pression de compression pendant au moins 15 secondes ; libère lors d'une détermination de la libération *in vitro* selon *Ph. Eur.* dans un appareil à agitateur à pales avec le milieu de libération à une vitesse de rotation de 75 tours par minute après 30 minutes 22 %, après 120 minutes 50 %, après 240 minutes 80 %, après 360 minutes 90 % et après 480 minutes 99 % de la substance (A) ; le comprimé étant introduit au début de l'essai dans 600 ml de milieu gastrique artificiel d'un pH de 1,2, qui est augmenté par ajout d'une base après 30 minutes à 2,3, après 90 minutes supplémentaires à 6,5 et après encore 60 minutes supplémentaires à 7,2 ;
(6) ne soit pas un comprimé ayant une résistance à la rupture d'au moins 500 N, qui présente un poids total de 150,0 mg, un diamètre de 10 mm et un rayon de courbure de 8 mm ; pouvant être obtenu par compression d'un mélange porté à 80 °C constitué par 20 mg de chlorhydrate d'oxycodon, 20,0 mg de xanthane et 110 mg de polyoxyde d'éthylène ayant un poids moléculaire de 7 000 000 par comprimé au moyen d'un outil de pastillage avec poinçon supérieur, poinçon inférieur et matrice avec maintien de la pression de compression pendant au moins 15 secondes ; et libère lors d'une détermination de la libération *in vitro* selon *Ph. Eur.* dans un appareil à agitateur à pales avec le milieu de libération tamponné par du phosphate décrit dans l'USP à pH 6,8 à 37 °C et une vitesse de rotation de 75 tours par minute après 30 minutes 17 %, après 240 minutes 61 %, après 480 minutes 90 % et après 720 minutes 101,1 % de la substance (A) ;
(7) ne soit pas un comprimé ayant une résistance à la rupture d'au moins 500 N, qui présente un poids total de 341,2 mg, un diamètre de 10 mm et un rayon de courbure de 8 mm ; pouvant être obtenu par dissolution de 0,2 mg de butylhydroxytoluène dans de l'éthanol à 96 %, séchage de la solution éthanolique à 7,7 % obtenue à 40 °C pendant 12 h en présence de 221 mg de polyoxyde d'éthylène ayant un poids moléculaire de 7 000 000, et par compression ultérieure conjointement avec un mélange porté à 80 °C constitué par 100 mg de chlorhydrate de tramadol et 20 mg d'hydroxypropylméthylcellulose par comprimé au moyen d'un outil de pastillage avec poinçon supérieur, poinçon inférieur et matrice avec maintien de la pression de compression pendant au moins 15 secondes ; libère lors d'une détermination de la libération *in vitro* selon *Ph. Eur.* dans un appareil à agitateur à pales avec le milieu de libération à une vitesse de rotation de 75 tours par minute après 30 minutes 17 %, après 240 minutes 59 %, après 480 minutes 86 % et après 720 minutes 98 % de la substance (A) ; le comprimé étant introduit au début de l'essai dans 600 ml de milieu gastrique artificiel d'un pH de 1,2, qui est augmenté par ajout d'une base après 30 minutes à 2,3, après 90 minutes supplémentaires à 6,5 et après encore 60 minutes supplémentaires à 7,2 ;
(8) ne soit pas un comprimé ayant une résistance à la rupture d'au moins 500 N, qui présente un poids total de 341,0 mg, un diamètre de 10 mm et un rayon de courbure de 8 mm ; pouvant être obtenu par compression d'un mélange porté à 80 °C constitué par 100 mg de chlorhydrate de tramadol, 221 mg de polyoxyde d'éthylène ayant un poids moléculaire de 7 000 000 et 20 mg d'hydroxypropylméthylcellulose par comprimé au moyen d'un outil de pastillage avec poinçon supérieur, poinçon inférieur et matrice avec maintien de la pression de compression pendant au moins 15 secondes ; libère lors d'une détermination de la libération *in vitro* selon *Ph. Eur.* dans un appareil à agitateur à pales avec le milieu de libération à une vitesse de rotation de 75 tours par minute après 30 minutes 16 %, après 240 minutes 57 %, après 480 minutes 84 % et après 720 minutes 96 % de la substance (A) ; le comprimé étant introduit au début de l'essai dans 600 ml de milieu gastrique artificiel d'un pH de 1,2, qui est augmenté par ajout d'une base après 30 minutes à 2,3, après 90 minutes supplémentaires à 6,5 et après encore 60 minutes supplémentaires à 7,2 ;
(9) ne soit pas un comprimé biplanaire ayant une résistance à la rupture d'au moins 500 N, qui présente un poids total de 335,0 mg ; pouvant être obtenu par dissolution de 0,2 mg de butylhydroxytoluène dans de l'éthanol à 96 %, mélange de la solution éthanolique à 7,7 % obtenue avec 150 mg de polyoxyde d'éthylène ayant un poids moléculaire de 7 000 000 dans un mélangeur rapide pendant 30 minutes, puis ajout de 17,8 mg de polyoxyde d'éthylène ayant un poids moléculaire de 7 000 000 et agitation pendant 30 minutes, puis séchage à 40 °C pendant 12 h ; ajout de 100 mg de chlorhydrate de tramadol, 33,5 mg d'hydroxypropylméthylcellulose et 33,5 mg de polyéthylène glycol ; et extrusion du mélange dans une extrudeuse bivis dans une atmosphère d'azote à une vitesse de rotation des vis de 100 tours par minute, un débit de 4 kg/h, une température du produit de 125 °C et une température de l'enveloppe de 120 °C ;
(10) ne soit pas un comprimé ayant une résistance à la rupture d'au moins 500 N, qui présente un poids total de 586,0 mg et un diamètre de 12 mm ; pouvant être obtenu par compression d'un mélange constitué par 205 mg de chlorhydrate de tramadol et 381 mg de polyoxyde d'éthylène ayant un poids moléculaire de 7 000 000 par comprimé sous l'effet simultané d'une force de 250 N et d'ultrasons d'une fréquence de 20 Hz et d'une amplitude de 50 %, pendant 0,5 seconde ; libère lors d'une détermination de la libération *in vitro* selon *Ph. Eur.* dans un appareil à agitateur à pales avec lest à une vitesse de rotation de 75 tours par minute après 30 minutes 13 %, après 240 minutes 51 %, après 480 minutes 76 % et après 720 minutes 100 % de la substance (A) ; le milieu de libération étant 600 ml de suc intestinal à une température de 37 °C et un pH de 6,8 ; et
(11) ne soit pas un comprimé ayant une résistance à la rupture d'au moins 500 N, qui présente un poids total de 300,0 mg et un diamètre de 10 mm et un rayon de courbure de 8 mm ; pouvant être obtenu par compression d'un mélange constitué par 100 mg de chlorhydrate de tramadol et 200 mg de polyoxyde d'éthylène ayant un poids moléculaire de 7 000 000 par comprimé sous l'effet
- d'une force de 970 N et d'ultrasons d'une fréquence de 20 Hz et d'une amplitude de 75 %, simultanément pendant 0,15 seconde ; puis
- d'une force de 970 N et d'ultrasons d'une fréquence de 20 Hz et d'une amplitude de 32,5 %, simultanément pendant 0,55 seconde ; puis
- d'une force de 970 N pendant 2,3 secondes ;
libère lors d'une détermination de la libération *in vitro* selon *Ph. Eur.* dans un appareil à agitateur à pales avec lest à une vitesse de rotation de 75 tours par minute après 30 minutes 17,1 %, après 240 minutes 60,6 %, après 480 minutes 84 % et après 720 minutes 94,2 % de la substance (A) ; le milieu de libération étant 600 ml de suc intestinal à une température de 37 °C et un pH de 6,8.

2. Forme pharmaceutique selon la revendication 1, **caractérisée en ce qu'**elle ne contient pas de substance à effet psychotrope.

3. Forme pharmaceutique selon la revendication 1 ou 2, **caractérisée en ce qu'**elle présente une résistance à la rupture d'au moins 500 N.

4. Forme pharmaceutique selon l'une quelconque des revendications précédentes, **caractérisée en ce qu'**elle se présente sous la forme d'un comprimé.

5. Forme pharmaceutique selon l'une quelconque des revendications précédentes, **caractérisée en ce qu'**elle ne contient ni du chlorhydrate de tramadol, ni du chlorhydrate d'oxycodon.

6. Forme pharmaceutique selon l'une quelconque des revendications précédentes, **caractérisée en ce que** la substance (A) est un médicament choisi dans le groupe constitué par les agents pour le traitement et la prévention de maladies du système alimentaire et du métabolisme ; les agents pour le traitement et la prévention de maladies du sang et des organes formant le sang ; les agents pour le traitement et la prévention de maladies du système cardiovasculaire ; les topiques cutanés ; les agents pour le traitement et la prévention de maladies du système urogénital et des hormones sexuelles ; les préparations hormonales systémiques à l'exclusion des hormones sexuelles et des insulines ; les anti-infectieux pour utilisation systémique ; les agents antinéoplasiques et immunomodulateurs ; les agents pour le traitement et la prévention de maladies des muscles et du système squelettique ; les agents pour le traitement et la prévention de maladies du système nerveux ; les agents antiparasitaires, les insecticides et les répulsifs ; les agents pour le traitement et la prévention de maladies des voies respiratoires ; les agents pour le traitement et la prévention de maladies des organes sensoriels ; les agents diététiques généraux et les agents radiothérapeutiques.

7. Procédé de fabrication d'une forme pharmaceutique selon l'une quelconque des revendications 1 à 6, comprenant les étapes suivantes :
(a) le mélange des composants (A), éventuellement (B), (C), éventuellement (D) ;
(b) éventuellement le pré-façonnage du mélange obtenu à l'étape (a), de préférence sous l'effet de chaleur et/ou d'une force sur le mélange obtenu en (a), la quantité de chaleur introduite n'étant de préférence pas suffisante pour porter le composant (C) jusqu'à son point de ramollissement ;
(c) le durcissement du mélange sous l'effet de chaleur et d'une force, l'apport de chaleur pouvant avoir lieu pendant et/ou avant l'effet de la force et la quantité de chaleur introduite étant suffisante pour porter le composant (C) jusqu'à son point de ramollissement ;
(d) éventuellement l'individualisation du mélange durci ;
(e) éventuellement le façonnage de la forme pharmaceutique ; et
(f) éventuellement le revêtement avec un film d'enrobage.

8. Procédé selon la revendication 7, **caractérisé en ce qu'**à l'étape (c), une extrudeuse bivis ou une extrudeuse à cylindres planétaires est utilisée.
